# EUROPEAN PATENT SPECIFICATION

(11) **EP 1 115 877 B1**
(45) Date of publication and mention of the grant of the patent: **14.01.2009**
(21) Application number: 99949142.6
(22) Date of filing: 22.09.1999
(51) Int. Cl.: C12N 15/63, C12N 15/86, C12N 7/01, C12N 5/10, C07K 14/47, A61K 48/00

(54) **POLYNUCLEOTIDE CONSTRUCTS AND USES THEREOF**
POLYNUCLEOTIDKONSTRUKTE UND IHRE VERWENDUNGEN
PRODUITS DE RECOMBINAISON POLYNUCLEOTIDIQUES ET LEURS UTILISATIONS

(30) Priority: 23.09.1998 WO PCT/GB98/02885; 28.01.1999 GB 9901906; 16.02.1999 GB 9903538
(43) Date of publication of application: 18.07.2001
(73) Proprietor: Oxford Biomedica (UK) Limited, Oxford 0X4 4GA (GB)
(72) Inventor: BINLEY, Katie Mary, Oxford OX3 7XF (GB); NAYLOR, Stuart, Amersham, Bucks HP6 6AN (GB)
(74) Representative: Harding, Charles Thomas
(86) International application number: PCT/GB1999/003181
(87) International publication number: WO 2000/017371

(56) References cited:
- WO-A-95/21927
- WO-A-96/20276
- WO-A-98/15294
- WO-A-98/19695
- WO-A-98/31701
- WO-A-99/15684
- K. BOAST ET AL.: "Characterization of physiologically regulated vectors for the treatment of ischemic disease" HUMAN GENE THERAPY, vol. 10, no. 13, 1 September 1999 (1999-09-01), pages 2197-2208, XP000876772 MARY ANN LIEBERT, INC. PUBLISHERS, NEW YORK, US
- G U DACHS ET AL: "Targeting gene expression to hypoxic tumor cells" NATURE MEDICINE,US,NATURE PUBLISHING, CO, vol. 3, no. 5, 1 May 1997 (1997-05-01), pages 515-520, XP002096343 ISSN: 1078-8956
- K.M. LONERGAN ET AL.: "Regulation of hypoxia-inducible mRNAs by the von Hippel-Lindau tumor suppressor protein requires binding to complexes containing elongins B/C and Cul2" MOL. CELL. BIOL., vol. 18, no. 2, February 1998 (1998-02), pages 732-741, XP002130567 ASM WASHINGTON, DC,US cited in the application
- K. BINLEY ET AL.: "An adenoviral vector regulated by hypoxia for the treatment of ischaemic disease and cancer" GENE THERAPY, vol. 6, no. 10, October 1996 (1996-10), pages 1721-1727, XP000876756 MACMILLAN PRESS, UK
- MADAN A ET AL: "A 24-BASE-PAIR SEQUENCE 3' TO THE HUMAN ERYTHROPOIETIN GENE CONTAINS A HYPOXIA-RESPONSIVE TRANCRIPTIONAL ENHANCER" PROCEEDINGS OF THE NATIONAL ACADEMY OF SCIENCES OF USA,US,NATIONAL ACADEMY OF SCIENCE. WASHINGTON, vol. 90, no. 9, 1 May 1993 (1993-05-01), pages 3928-3932, XP000561802 ISSN: 0027-8424

## Description

### Field of the Invention

Polynucleotide constructs comprising hypoxia response elements operably linked to promoter sequences for use in targeting expression of therapeutic genes to cells such as tumour cells which are under conditions of hypoxia.

### Background to the Invention

Gene therapy, the treatment or prevention of disease by gene transfer, is one of a number of approaches being used to treat a range of malignancies that occur in humans. In particular, an increased understanding of the molecular genetics of cancer has led to the development of various novel therapeutic strategies for cancer. Gene-based therapies currently being tested in clinical trials involve largely the *ex vivo* or *in vivo* use of viral or liposomal vectors to deliver genes to tumours for: (i) tumour suppressor gene replacement or oncogene inactivation, (ii) the expression of cytokines/vaccines known to activate or enhance anti-tumour immune mechanisms, (iii) enhanced drug sensitivity (e.g. pro-drug delivery or activation) (iv) drug resistance for bone marrow protection from high dose chemotherapy, and (v) inhibitors of tumour angiogenesis.

Targeting the expression of such therapeutic genes specifically to solid tumours has, until recently, been problematic. In some instances, recombinant viral vectors bearing therapeutic genes have been targeted to specific cell types by the insertion of ligands/antibodies into the viral capsid. This approach requires the expression of tumour-specific antigens, as well as tissue-specific antigens, by the malignant cell. Thus any therapy is restricted to use in particular patient groups and tumour types showing expression of the selected tumour antigen. Alternatively, naked or viral-incorporated DNA can be injected directly in to the tumour (or at least into the local blood supply for the tumour) to maximise specificity of delivery and expression at the tumour site. Although, this approach has met with limited success for superficial tumours (e.g. breast and melanoma lesions), it relies on the accurate localisation of the tumour and does not ensure DNA uptake by the entire tumour mass or treat secondary local or distant metastatic deposits.

Recently, an alternative approach for targeting therapeutic gene expression to tumours has been developed (Dachs *et al*., 1997). This utilises the abnormal physiology that exists in almost all solid tumours, regardless of their origin or location. Specifically, it takes advantage of the tumour-specific condition of severe ischaemia, and its effects on specific enhancer regions of certain genes, to control the expression of heterologous genes (Dachs *et al*., 1997; UK Patent Application No. 9701975.6).

Aggressive tumours generally have insufficient blood supply, partly because tumour cells grow faster than the endothetial celis that make up the blood vessels, and partly because the newly formed vascular supply is disorganised. This results in areas of ischaemia and nutrient deprivation, including regions with both reduced oxygen tension (hypoxia) and glucose levels. Oxygen electrode measurements of tumours have shown significant proportions of readings below 2.5 mmHg (normal tissues ranges from 24 to 66 mmHg). Moreover, hypoxic cells are markedly less sensitive to radiotherapy - and chemotherapy, which is why, in part, increased levels of tumour hypoxia correlate with reduced survival in many forms of cancer.

Hypoxia is a powerful regulator of gene expression in a wide range of different cell types (Wang and Semenza, 1993a). Studies of the genes involved in vascularization and carbohydrate metabolism have identified a number of genes which are inducted under hypoxic conditions. For example, the gene encoding erythropoietin is known to be regulated by hypoxia.

Erythropoietin (EPO) is a hormone which regulates erythropoiesis and hence blood oxygen content. Cis-activating DNA sequences that function as tissue-specific hypoxia-inducible enhancers of human erythropoietin expression have been identified (Semenza *et al*., 1991). These enhancers contain regulatory elements termed hypoxia response elements (HREs) which are the binding sites for hypoxia-inducible transcription factors such as hypoxia inducible factor-1 (HIF-1). A DNA enhancer sequence located 3' to the mouse erythropoietin gene has been shown to confer oxygen-regulated expression on a variety of heterologous promoters (Pugh *et al*., 1991).

A second example of a hypoxia-associated regulatory sequence is an enhancer element which lies 5' to the mouse phosphoglycerate kinase (PGK) gene promoter. The sequence of the enhancer has been published (McBurney *et al*., 1991) and its hypoxia-inducible properties disclosed in WO-A-95/21927. The HRE within this PGK enhancer has been defined as an 18 bp sequence.

WO 98/15294 discloses regulatory elements in vectors comprising three repeats of the HRE derived from the enolase A gene. These repeats are placed upstream of the SV40 viral promotor.

Dachs *et al*. (1997) used a multimeric form of the mouse PGK-1 gene (Firth *et* a/., 1994) to control expression of both marker and therapeutic genes by human fibrosarcoma cells in response to hypoxia *in vitro* and within solid tumours *in vivo* (Dachs *et al*., 1997).

Thus, since ischaemia is a general feature of solid tumours regardless of their cellular origin or patient population, it is possible to exploit tumour hypoxia to obtain selective expression of genes in tumours (Dachs *et al*., 1997).

Ischaemic damage may also occur in may other tissues when the blood supply to the tissue is reduced or cut off. Stroke, deep vein thrombosis, pulmonary embolus and renal failure are examples of conditions that can cause such damage. The cell death of cardiac tissue, called myocardial infarction, is due in large part to tissue damage caused by ischemia and/or ischemia followed by reperfusion. Recurrent ischaemia and reperfusion typically results in oxidative damage to cells from reactive oxygen species. The extent and type of damage depends on the severity and nature of the hypoxic stress. For example, the stress may cause tissue necrosis. Alternatively, the stress may initiate apoptosis (programmed cell death) to eliminate the damaged cells.

Ideally, a hypoxia-inducible expression construct for use in tumour therapy should be capable of directing high level expression under hypoxic conditions whilst only directing low basal levels of transcription under normoxic conditions. This combination allows maximum delivery of therapeutic product to tumour cells without affecting normal surrounding oxygenated tissue. However, although studies carried out to date have demonstrated that it is possible to obtain hypoxia-activated transcription in tumour cells, the levels of transcription obtained and the hypoxia induction ratios (expression under hypoxia/expression under normoxia) have been disappointing. For example, although WO-A-95/21927 tests constructs having three copies of the mouse PGK HRE linked to the PGK-1 promoter, the 9-27 promoter or the thymidine kinase gene promoter, the maximum induction ratio obtained under hypoxic and normoxic conditions was about eighteen-fold.

### Summary of the Invention

By contrast, we have now surprisingly found that a novel regulatory construct comprising three repeats of the mouse phosphoglycerate kinase (PGK) gene HRE (optionally with the middle repeat comprising a deletion in the HIF-1 consensus binding site) operably linked to a strong viral promoter, the SV40 or MLV promoter, is capable of driving very high levels of transcription under conditions of hypoxia whilst providing only low basal levels of transcription under normal oxygen conditions. The induction ratio of hypoxic/normoxic conditions was at least 100-fold. Thus a polynucleotide construct of the present invention may be advantageously used to target specifically cells within a tumour mass which are under conditions of hypoxia, without affecting normal surrounding tissue.

In addition, we now describe, for the first time, lentiviral vectors that are responsive to hypoxia and to agents that mimic hypoxia. This regulation can be harnessed *in vitro* to enhance production of the vector and it can be used *in vivo* to regulate gene expression in response to a physiological signal. Such vectors have utility in a wide range of diseases where ischaemia, including hypoxia, is a feature, for example, cardiovascular disease, peripheral arterial disease, cancer and arthritis.

The present invention provides a polynucleotide comprising at least three repeats of a phosphoglycerate kinase (PGK) hypoxia response element (HRE) operably linked to an SV40 promoter or a murine leukaemia virus (MLV) promoter. Preferably, the HRE repeats are direct repeats. More preferably, the repeats are in their naturally occurring orientation.

A preferred functional HRE of the present invention consists of a nucleotide sequence as shown in SEQ ID. No. 1 or SEQ ID. No. 2. A preferred HRE/promoter construct of the invention comprises a nucleotide sequence as shown in SEQ ID. No.3, SEQ ID. No. 4, SEQ ID. No. 5 or SEQ ID. No. 9.

In a preferred embodiment, the polynucleotide of the invention comprises at least three repeats of the HRE operably linked to the promoter and upstream (5' to) the promoter and at least three repeats of the HRE operably linked to the promoter and downstream (3' to) the promoter.

The present invention also provides a polynucleotide of the invention operably linked to a nucleic acid of interest (NOI), to produce in effect an expression cassette, such that the polynucleotide directs expression of the NOI in a host cell. Thus the present invention further provides a polynucleotide of the invention operably linked to an NOI for use in expressing the NOI in a host cell, for example a mammalian cell, more preferably a human cell.

The NOI preferably encodes a polypeptide of therapeutic use, such as a polypeptide which is cytotoxic or a polypeptide capable of converting a precursor prodrug into a cytotoxic compound. The NOI may also preferably be selected from polynucleotide sequences encoding proteins involved in the regulation of cell division, enzymes involved in cellular metabolic pathways, transcription factors and heat shock proteins.

We have also shown that inclusion of a nucleic acid sequence encoding a HIF-1α polypeptide improves the induction under hypoxic conditions. Accordingly, in a highly preferred embodiment, the NOI encodes a HIF-1α polypeptide.

The present invention also provides a nucleic acid vector comprising a polynucleotide of the invention. In a preferred embodiment, the present invention provides a viral vector comprising a polynucleotide of the invention. Preferably the viral vector is a retroviral, such as a lentiviral, an adenoviral or a herpes simplex viral vector, more preferably a lentiviral vector.

Although NOI sequences inserted into retroviral vectors may be placed under the control of the 5' LTR sequence (modified or otherwise) or an internal promoter, we have now shown that there is a particular advantage to configuring such vectors as single transcription unit vectors whereby the HRE/promoter construct of the invention is placed within the 3'LTR such that the resultant duplication of the 3'LTR also leads to duplication of the regulatory sequence. We have now shown that this arrangement enhances the activated response to hypoxia in a synergistic manner.

This technique will be equally applicable to other regulatory sequences and consequently, a retroviral vector of the invention comprising a heterologous regulatory sequence within the U3 region of the viral genome is also provided. Accordingly, there is described a method for producing a retroviral vector which comprises inserting a regulatory sequence into an LTR region of a retroviral vector such that the regulatory sequence is duplicated in the proviral genome. Preferably, the region is the U3 region. Preferably, the retroviral vector also comprises an NOI which is operably linked to the regulatory sequence. More preferably the NOI is not in a region of the retroviral vector which results in duplication of the NOI in the proviral genome.

In a particular aspect adenoviral vectors that contain the components that are required to make a retroviral or a lentiviral vector are used to transduce, for example, CD34⁺ stem cells. The cell that has been transduced with the adenoviral vector therefore secretes the retroviral/lentiviral vector. The adenoviral vector in the cells therefore acts as an *in situ* retroviral factory as described previously (WO-A-97/27310).

The present invention further provides a host cell comprising a polynucleotide of the invention. Host cells of the invention may be produced *in vitro*, *in vivo* or *ex vivo*. In a particularly preferred embodiment, the host cell may be a differentiated cell, for example a macrophage or an endothelial cell, more preferably a terminally differentiated cell. Alternatively, the host cell may be an undifferentiated cell, for example a haematopoietic stem cell (HSC).

Preferably, the differentiated cell is derived from the HSC. This aspect is advantageous as it provides a means for providing for selective expression in or by or from, for example, a macrophage that has been differentiated from the HSC.

There is also described a host cell, which cell may be a differentiated cell or an undifferentiated cell which is capable of being differentiated to a differentiated cell, which host cell comprises a polynucleotide of the invention comprising an HRE/promoter operably linked to an NOI wherein the polynucleotide of the invention has been introduced into the cell by viral transduction, preferably by adenoviral transduction and/or lentiviral transduction.

Polynucleotides of the invention operably linked to an NOI comprising a therapeutic gene may advantageously be used to deliver therapeutic products to mammalian cells. In particular, mammalian cells which are under hypoxic conditions will preferentially express the NOI under the control of the HRE/promoter construct whereas surrounding cells under conditions of normoxia will express the NOI at much lower levels.

Accordingly, the present invention provides a polynucleotide, a nucleic acid vector, viral vector or host cell of the invention for use in the treatment of a human or animal patient suffering from a disease in which hypoxia is a cause or a symptom or is otherwise present.

The present invention also provides a pharmaceutical composition comprising a polynucleotide, a vector, a viral vector or host cell of the invention together with a pharmaceutically acceptable carrier or diluent.

In a still further aspect, the present invention provides a method of producing a viral strain comprising introducing a polynucleotide of the invention into the genome of a virus. Preferably, the method comprises introducing the polynucleotide into the genome of the virus by homologous recombination between said genome and a vector of the invention.

Ischaemia can be an insufficient supply of blood to a specific organ or tissue. A consequence of decreased blood supply is an inadequate supply of oxygen to the organ or tissue (hypoxia). Prolonged hypoxia may result in injury to the affected organ or tissue.

A preferred aspect of the present invention relates to uses of any of the aforementioned products in the treatment or prevention of a human or animal patient suffering from a disease in which hypoxia is a cause or a symptom or is otherwise prevent, particularly, but not exclusively, a condition such as cancer (in particular solid tumours).

### Detailed Description of the Invention

### A. Polynucleotides

Polynucleotides of the invention may comprise DNA or RNA. They may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present invention, it is to be understood that the polynucleotides described herein may be modified by any method available in the art. Such modifications may be carried out, for example, to enhance the *in vivo* activity or life span of polynucleotides of the invention.

### 1. Promoter/enhancer sequences

An HRE for use in the present invention is a minimal nucleotide sequence that acts under conditions of hypoxia on a promoter sequence to which it is operably linked to increase the rate of transcription of a nucleotide sequence of interest (NOI) under the control of the promoter. Preferably, the HRE and promoter which comprise the polynucleotide of the present invention are selected such that expression of an NOI gene operably linked to these elements is minimal in the presence of a healthy supply of oxygen and is upregulated under hypoxic or anoxic conditions. More preferably the ratio of the rate of transcription under normoxic conditions (for example 21% oxygen) to the rate of transcription under hypoxic conditions (for example less than 1% oxygen or 0.1% oxygen) is at least 20:1, more preferably at least 50:1 or 100:1.

The polynucleotide of the present invention comprises at least three HREs. Where more than one HRE is used, they are typically spaced apart by at least 4 to 6, more preferably at least 6 nucleotides. In a especially preferred embodiment, at least two of the HREs are spaced apart by at least 20 nucleotides, more preferably at least 30 or 35 nucleotides. Spacing is typically measured from the 3' end of the hypoxia inducible factor (for example HIF-1) binding site within one HRE to the 5' end of the hypoxia inducible factor binding site of the next HRE. The HIF-1 binding site has the consensus C/A.G.G/C.ACGT.G/C/A (or its complement). In one embodiment, the spacer comprises the sequence 5' GTCGTGCAGGCA 3' (SEQ I.D. No. 10), or its complement, more preferably the sequence 5'TCTAGTGTCGTGCAGGCATCTAGT 3' (SEQ I.D. No. 11), or its complement.

In a preferred embodiment, the spacer lacks an hypoxia inducible factor (such as HIF-1) binding site. For example, it may contain a mutant hypoxia inducible factor binding site obtained by mutation of all or part of a consensus site such that its ability to bind an hypoxia inducible factor has been substantially reduced or abolished (see below).

Multiple HRE repeats may be in the same or different orientation. In a preferred embodiment the HREs are in the same orientation, in particular in the same orientation as found in the naturally occurring regulatory control sequence from which they originate, where appropriate. HREs may be upstream of the promoter (5') or downstream of the promoter (3') or both.

A variety of HREs are known in the art. For example HREs have been found in association with a number of genes including the erythropoietin (EPO) gene (Madan *et al*., 1993; Semenza and Wang, 1992). Other HREs have been isolated from regulatory regions of both the muscle glycolytic enzyme pyruvate kinase (PKM) gene (Takenaka *et al*., 1989), the human muscle-specific β-enolase gene (ENO3; Peshavaria and Day, 1991) and the endothelin-1 (ET-1) gene (Inoue *et al*., 1989). A list of HREs is given in Table 1 below. The HRE according to the present invention is the phosphoglycerate kinase (PGK) HRE (WO95/21927), such as the mouse PGK HRE P24 sequence CGCGTCGTGCAGGACGTGACAAAT (SEQ ID. No. 1) or its trancated P18 version GTCGTGCAGGACGTGACA (SEQ ID. No. 2). Another HRE is the D2 HRE of Example 21 - GTCGTGCAGTACGTGACA (SEQ ID. No. 12).

A particular example of a hypoxia regulated enhancer is a binding element for transcription factor HIF-1 (Dachs *et al*., 1997; Wang and Semenza, 1993a; Firth *et al*., 1994 and/or a binding element for endothelial PAS domain protein (EPAS). In a particularly preferred embodiment, an HRE for use in the present invention is capable of binding both HIF-1 and E-PAS. However, an HRE for use in the present invention may be capable of preferentially binding E-PAS as opposed to HIF-1. Thus, an HRE for use in the present invention may bind E-PAS at least 10 times, preferably at least 20, 30, 40 or 50 times more strongly than HIF-1. This can be tested by, for example, *in vitro* binding assays such as electrophoretic band shift assays or by competition assays using nucleotides comprising HRE sites immobilised on a solid substrate such as sepharose beads.

**Table 1 - HRE'S**

| | |
|---|---|
| hEPO | GGGCCCTACGTGCTGTCTCACACAGC |
| mEPO | GGGCCCTACGTGCTGCCTCGCATGGC |
| mPGK | CGCGTCGTGCAGGACGTGACAAAT |
| mLDH | CCAGCGGACGTGCGGGAACCCACGTGTAGG |
| Glucose trpt | TCCACAGGCGTGCCGTCTGACACGCA |
| hVEGF | CCACAGTGCATACGTGGGCTCCAACAGGTCCTCTT |
| rVEGF | ACAGTGCATACGTGGGCTTCCACA |
| hNOS | ACTACGTGCTGCCTAGG |
| hAldolase | CCCCTCGGACGTGACTCGGACCACAT |
| hEnolase | ACGCTGAGTGCGTGCGGGACTCGGAGTACGTGACGGA |
| mHeme Oxygenase | CGGACGCTGGCGTGGCACGTCCTCTC |

Where an EPAS-binding HRE is used, it may be desirable to enhance hypoxia specificity/inducibility by introducing into the host cell an NOI encoding the EPAS. Alternatively, the host cell used may naturally express the ERAS (for example epithelial cells, including tumour cells, and macrophage cells).

By contrast, where a HIF-1 binding HRE is used, it may be desirable to enhance hypoxia specificity/inducibility by introducing into the host cell an NOI encoding HIF-1, such as HIF-1α under the control of the HRE by way of an autoregulatory system.

It is also possible to modify a known HRE, or design a novel HRE based on the HRE consensus sequence, to obtain an HRE suitable for use in the present invention provided that it is able to confer hypoxia-inducible transcription, typically when combined with a promoter sequence, on an operably linked NOI. Modifications include nucleotide substitutions, deletions and insertions. In a particularly preferred embodiment, at least three HRE repeats are used and one HRE element, for example the middle repeat, is mutated to render its HIF-1 site non-functional, for example by deletion. In this way, at least two functional HRE elements remain separated by a spacer derived from a functional HRE element but which lacks an hypoxia-inducible factor binding site. An example of such a construct is Obhre11 which comprises three direct repeats of a mouse PGK HRE element, the middle one of which comprises a deletion in its HIF-1 binding site.

A further example of modification of an HRE to improve its hypoxic response is provided in Example 21.

An HRE may also contain additional sequences, for example those with which it is naturally associated as part of an enhancer, or other sequences.

The level of expression of an NOI or NOIs under the control of a particular polynucleotide of the invention may be modulated by manipulating the enhancer/promoter region. For example, different domains within a promoter region may possess different gene regulatory activities. The roles of these different regions are typically assessed using vector constructs having different variants of the promoter with specific regions deleted (that is, deletion analysis). This approach may be-used to identify, for example, the smallest region capable of conferring tissue specificity or the smallest region conferring hypoxia inducibility.

Promoters used in the polynucleotides of the invention are the strong viral promoters SV40, or murine leukaemia virus (MLV). Thus preferred HRE/promoter constructs include the constructs shown in SEQ ID. Nos 3, 4 and 5.

However, in another preferred embodiment, particularly where a viral promoter and a HIF-1 autoregulated construct is used, it may be desirable to reduce basal transcription by using a promoter that lacks one or more of the transcriptional regulatory sequences normally associated with the TATA box or initiator sequence of the promoter. For example the promoter may tack a CAAT box motif, as in the case of the MLV promoter described in Example 20, and/or an Sp1 consensus binding site, such as is normally found within the SV40 promoter. It may also be possible to use a minimal promoter consisting essentially of a TATA box-or initiator linked to an HRE.

In addition to the HRE sequences and promoter, the polynucleotide of the invention may comprise additional regulatory control sequences. For example, additional levels of transcriptional control may be used to ensure that expression directed by the polynucleotide of the invention is confined to certain cell types or under certain conditions. Thus additional enhancers may be operably linked to the polynucleotide of the invention, either downstream, upstream or both.

The additional regulatory sequence may be a sequence found in eukaryotic genes. For example, it may be a sequence derived from the genome of a ceil in which expression of the NOI is to occur, preferably a tumour. Subject to the over-riding control of the HRE enhancer/promoter construct of the invention, the additional regulatory sequence may function to confer ubiquitous expression or alternatively tissue-specific expression. It is particularly preferred that additional regulatory sequences are used that are preferentially active in one or more specific cell types - such as any one or more of macrophages, endothelial cells or combinations thereof. Further examples include respriatory aîrway epithelial cells, hepatocytes, muscle cells, cardiac myocytes, synoviocytes, primary mammary epithelial cells and post-mitotically terminally differentiated non-replicating cells such as macrophages.

Examples of sequences which are cell specific include a macrophage-specific promoter or enhancer such as a CSF-1 promoter-enhancer (see Example 8), or elements from a mannose receptor gene promoter-enhancer. Alternatively, elements which are preferentially active in neutrophils, or a lymphocyte-specific enhancer such as an IL-2 gene enhancer, may be used. A particularly preferred tissue specific regulatory sequence is an HIV-derived sequence that confers macrophage-specific expression (such as the promoter sequences described in Example 8 - XiaMac (SEQ ID. No. 7).

Another example is an endothelium specific promoter, which may be used to restrict the expression of an NOI to vascular endothelium. In particular the correct choice of regulatory sequences can restrict expression to the neo-vasculature that is specific to tumours. Jaggar *et al*. (1997) have described the use of the E-selectin and KDR promoters to express therapeutic genes from retroviral vectors specifically in endothelial cells. Hypoxia-regulated endothelium specific promoters may be particularly useful for the delivery of anti-angiogenic factors to tumour vasculature.

The term "tissue specific" means a regulatory control Sequence which is not necessarily restricted in activity to a single tissue type but which nevertheless shows selectivity in that it may be active in one group of tissues and less active or silent in another group.

A number of tissue specific enhancers, described above, may be particularly advantageous in practising the present invention. In most instances, these enhancers may be isolated as convenient restriction digestion fragments suitable for cloning in a selected vector. Alternatively, enhancers fragments may be isolated using the polymerase chain reaction. Cloning of the amplified fragments may be facilitated by incorporating restriction sites at the 5' end of the primers. Enhancer fragments may also be synthesised using, for example, solid-phase technology.

A particular example of a regulated sequence that may be added to the HRE/promoter of the invention to restrict expression to macrophages under defined conditions is the IRF system (Taniguchi *et al*., 1995; Kuhl *et al*., 1987). In this situation an interferon response element (IRE) is used that can bind the transcription factors IRF1 and IRF2. IRF 2 is constitutively bound to this IRE in macrophages and lacks the ability to activate transcription. Interferon activates IRF1 expression that can then compete for binding with IRF2. The ability of IRF1 to activate transcription thereby reverses IRF2 repression. IRF1 also induces IRF2 gene expression thereby limiting activation of transcription by 'auto shut off. Inclusion of a tetramer of the IRE can block SV40 promoter function in the absence of IRF 1 activation. Inclusion of this tetrameric sequence downstream of the HRE 5' to the ATATAA (i.e. the TATA box like element in the SV40 promoter) confers repression in the absence of activation by interferon.

Interferon gamma is naturally present in inflammatory responses including the response to tumours. Alternatively interferon gamma can be provided exogenously as a protein or as a gene and delivered as a gene therapy. In a particular aspect of the invention an autocrine regulatory circuit can provide interferon gamma. In this case a simple HRE promoter such as OBHRE is linked to interferon gamma coding sequence. A second gene, for example a pro-drug activating enzyme or any from the above list is linked to the XiaMac promoter that contains an IRF-1 responsive sequence. (SEQ ID. No. 8 - see Examples) The promoter is inactive in all cells including macrophages. Upon exposure to hypoxia in the pathological condition the interferon gamma is expressed. The expression of the therapeutic gene is then activated by the macrophage specific factors and the hypoxia responsive factors. This two phase strategy can be applied to any repressor protein.

Additional regulatory sequences may also comprise elements that respond to specific stimuli, for example elements that bind steroid hormone receptors.

It may also be desirable to include regulatory elements that are inducible, for example such that expression can be regulated by administration of exogenous substances. In this way, levels of expression of the NOI can be regulated during the life-time of the cell. Inducible means that the levels of expression obtained using the promoter can be regulated. For example, in a preferred embodiment a polynucleotide of the invention comprises regulatory sequences responsive to the tet repressor/VP16 transcriptional activator fusion protein previously reported (Gossen and Bujard, 1992; Gossen *et al*, 1995). A second polynucleotide would then typically comprise a strong promoter (e.g. the CMV IE promoter) driving the expression of the tet repressor/VP16 fusion protein. Thus in this example expression of the first NOI would depend on the presence or absence of tetracycline.

A desirable characteristic of the polynucleotides of the present invention is that they possess a relatively low transcriptional activity in the absence of activated hypoxia-regulated enhancer elements, even in the target tissue. For example, "silencer" elements may be used which suppress the activity of a selected promoter in the absence of hypoxia. Another strategy is to use a HIF-1 autoregulated construct as described above and in the Examples.

### 2. Nucleic acids of Interest (NOI)

The polynucleotide of the invention is typically operably linked to an NOI, usually a heterologous gene. The term "heterologous gene" encompasses any gene. The heterologous gene may be any allelic variant of a wild-type gene, or it may be a mutant gene. The term "gene" is intended to cover nucleic acid sequences which are capable of being at least transcribed. Thus, sequences encoding mRNA, tRNA and rRNA are included within this definition. The sequences may be in the sense or antisense orientation with respect to the promoter. Antisense constructs can be used to inhibit the expression of a gene in a cell according to well-known techniques. Nucleic acids may be, for example, ribonucleic acid (RNA) or deoxyribonucleic acid (DNA) or analogues thereof. Sequences encoding mRNA will optionally include some or all of 5' and/or 3' transcribed but untranslated flanking sequences naturally, or otherwise, associated with the translated coding sequence. It may optionally further include the associated transcriptional control sequences normally associated with the transcribed sequences, for example transcriptional stop signals, polyadenylation sites and downstream enhancer elements. Nucleic acids may comprise cDNA or genomic DNA (which may contain introns). However, it is generally preferred to use cDNA because it is expressed more efficiently since intron removal is not required.

The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequence.

In accordance with the present invention, suitable NOI sequences include those that are of therapeutic and/or diagnostic application such as, but are not limited to: sequences encoding cytokines, chemokines, hormones, antibodies, engineered immunoglobulin-like molecules, a single chain antibody, fusion proteins, enzymes, immune co-stimulatory molecules, immunomodulatory molecules, anti-sense RNA, a transdominant negative mutant of a target protein, a toxin, a conditional toxin, an antigen, a tumour suppressor protein and growth factors, membrane proteins, vasoactive proteins and peptides, anti-viral proteins and ribozymes, and derivatives therof (such as with an associated reporter group).

Suitable NOIs for use in the present invention in the treatment or prophylaxis of cancer include NOIs encoding proteins which: destroy the target cell (for example a ribosomal toxin), act as: tumour suppressors (such as wild-type p53); activators of anti-tumour immune mechanisms (such as cytokines, co-stimulatory molecules and immunoglobulins); inhibitors of angiogenesis; or which provide enhanced drug sensitivity (such as pro-drug activation enzymes); indirectly stimulate destruction of target cell by natural effector cells (for example, strong antigen to stimulate the immune system or convert a precursor substance to a toxic substance which destroys the target cell (for example a prodrug activating enzyme). Encoded proteins could also destroy bystander tumour cells (for example with secreted antitumour antibody-ribosomal toxin fusion protein), indirectly stimulated destruction of bystander tumour cells (for example cytokines to stimulate the immune system or procoagulant proteins causing local vascular occlusion) or convert a precursor substance to a toxic substance which destroys bystander tumour cells (eg an enzyme which activates a prodrug to a diffusible drug).

NOI(s) may be used which encode antisense transcripts or ribozymes which interfere with expression of cellular genes for tumour persistence (for example against aberrant *myc* transcripts in Burkitts lymphoma or against *bcr-abl* transcripts in chronic myeloid leukemia. The use of combinations of such NOIs is also envisaged.

Instead of, or as well as, being selectively expressed in target tissues, the NOI or NOIs may encode a pro-drug activation enzyme or enzymes which have no significant effect or no deleterious effect until the individual is treated with one or more pro-drugs upon which the enzyme or enzymes act. In the presence of the active NOI, treatment of an individual with the appropriate pro-drug leads to enhanced reduction in tumour growth or survival.

A pro-drug activating enzyme may be delivered to a tumour site for the treatment of a cancer. In each case, a suitable pro-drug is used in the treatment of the patient in combination with the appropriate pro-drug activating enzyme. An appropriate pro-drug is administered in conjunction with the vector. Examples of pro-drugs include: etoposide phosphate (with alkaline phosphatase); 5-fluorocytosine (with cytosine deaminase); doxorubicin-N-p-hydroxyphenoxyacetamide (with penicillin-V-amidase); para-N-bis(2-chloroethyl) aminobenzoyl glutamate (with carboxypeptidase G2); cephalosporin nitrogen mustard carbamates (with β-lactamase); SR4233 (with P450 Reducase); ganciclovir (with HSV thymidine kinase); mustard pro-drugs with nitroreductase and cyclophosphamide (with P450).

Examples of suitable pro-drug activation enzymes for use in the invention include a thymidine phosphorylase which activates the 5-fluoro-uracil pro-drugs capcetabine and furtulon; thymidine kinase from herpes simplex virus which activates ganciclovir; a cytochrome P450 which activates a pro-drug such as cyclophosphamide to a DNA damaging agent; and cytosine deaminase which activates 5-fluorocytosine. Preferably, an enzyme of human origin is used

Suitable NOIs for use in the treatment or prevention of ischaemic heart disease include NOIs encoding plasminogen activators. Suitable NOIs for the treatment or prevention of rheumatoid arthritis or cerebral malaria include genes encoding anti-inflammatory proteins, antibodies directed against tumour necrosis factor (TNF) alpha, and anti-adhesion molecules (such as antibody molecules or receptors specific for adhesion molecules).

The expression products encoded by the NOIs may be proteins which are secreted from the cell. Alternatively the NOI expression products are not secreted and are active within the cell. In either event, it is preferred for the NOI expression product to demonstrate a bystander effector or a distant bystander effect; that is the production of the expression product in one cell leading to the killing of additional, related cells, either neighbouring or distant (e.g. metastatic), which possess a common phenotype.

Where macrophages or other haematopoietic cells are used, NOIs may be used which encode, for example, cytokines. These would serve to direct the subsequent differentiation of the HSCs containing a therapeutic NOI. Suitable cytokines and growth factors include but are not limited to: ApoE, Apo-SAA, BDNF, Cardiotrophin-1, EGF, ENA-78, Eotaxin, Eotaxin-2, Exodus-2, FGF-acidic, FGF-basic, fibroblast growth factor-10, FLT3 ligand, Fractalkine (CX3C), GDNF, G-CSF, GM-CSF, GF-β1, insulin, IFN-γ, IGF-I, IGF-II, IL-1α, II-1β, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin α, Inhibin β, IP-10, keratinocyte growth factor-2 (KGF-2), KGF, Leptin, LIF, Lymphotactin, Mullerian inhibitory substance, monocyte colony inhibitory factor, monocyte attractant protein, M-CSF, MDC (67 a.a.), MDC (69 a.a.), MCP-1 (MCAF), MCP-2, MCP-3, MCP-4, MDC (67 a.a.), MDC (69 a.a.), MIG, MIP-1α, MIP-1β, MIP-3α, MIP-3β, MIP-4, myeloid progenitor inhibitor factor-1 (MPIF-1), NAP-2, Neurturin, Nerve growth factor, β-NGF, NT-3, NT-4, Oncostatin M, PDGF-AA, PDGF-AB, PDGF-BB, PF-4, RANTES, SDF1α, SDF1β, SCF, SCGF, stem cell factor (SCF), TARC, TGF-α, TGF-β, TGF-β2, TGF-β3, tumour necrosis factor (TNF), TNF-α, TNF-β, TNIL-1, TPO, VEGF, GCP-2, GRO/MGSA, GRO-β, GRO-γ and HCC1.

For some applications, a combination of some of these cytokines may be preferred, in particular a combination which includes IL-3, IL-6 and SCF, for the maintenance and expansion of stem cell populations. For differentiation *in vitro,* further cytokines may be added such as GM-CSF and M-CSF to induce differentiation of macrophages or GM-CSF and G-CSF to obtain neutrophils. On reintroduction of the engineered cells into the individual from whom they were derived, the body's own mechanisms then permit the cells or their differentiated progeny to migrate into the affected area e.g. the tumour.

Optionally, another NOI may be a suicide gene, expression of which in the presence of an exogenous substance results in the destruction of the transfected or transduced cell. An example of a suicide gene includes the herpes simplex virus thymidine kinase gene (HSV *tk*) which can kill infected and bystander cells following treatment with ganciclovir.

Optionally another NOI may be a targeting protein (such as an antibody to the stem cell factor receptor (WO-A-92/17505; WO-A-92/21766). For example, recombinant (ecotropic) retroviruses displaying an antibody (or growth factor or peptide) against a receptor present on HSCs (CD34 or stem cell factor, for example) might be used for targeted cell delivery to these cells, either *ex vivo* by incubating unfractionated bone marrow with virus or by intravenous delivery of virus.

NOIs may also include marker genes (for example encoding β-galactosidase or green fluorescent protein) or genes whose products regulate the expression of other genes (for example, transcriptional regulatory factors including the tet repressor/VP16 transcriptional activator fusion protein described above). In addition, NOIs may comprise sequences coding fusion protein partners in frame with a sequence encoding a protein of interest. Examples of fusion protein partners include the DNA binding or transcriptional activation domain of GAL4, a 6xHis tag and β-galactosidase. It may also be desirable to add targeting sequences to target proteins encoding by NOIs to particular cell compartments or to secretory pathways. Such targeting sequences have been extensively characterised in the art.

In a preferred embodiment, at least one NOI, operably linked to an HRE/promoter of the present invention encodes a HIF-1 transcription factor or a homologue or fragment thereof capable of binding to a HIF-1 binding site. Such a construct will provide an autoregulated system since in the presence of hypoxia, expression of HIF-1 from the HRE construct will increase and serve to further increase transcription from the HRE construct and other HRE constructs present.

### Enhancement of the hypoxic response by inhibiting VHL-mediated degradation of HIF

Maxwell *et al*., (1999) have published evidence that suggests the tumour suppressor protein VHL targets hypoxia-inducible factors for oxygen-dependent proteolysis. Prior to this data has been published suggesting that in order to regulate hypoxia-inducible gene expression VHL requires binding to other proteins including elongins, B and C and Cul2 (Lonergan, 1998). Disruption of this multiprotein complex may be a route to block the degradation of HIFs and thereby increase longevity/degree of expression of HRE regulated genes. There are several possible methods for inhibiting VHL function.

A synthetic peptide derived from pVHL (for example amino acids 157-172) may be used to block the binding of full length endogenous pVHL to Elongin B and C. Introduction of this peptide into cells by standard transfection techniques preferably as part of a lipid complex, may prevent association of endogenous VHL with the rest of the complex resulting in the failure to degrade HIF.

Alternatively, the VHL peptide may be co-expressed under the control of an HRE/promoter construct with another NOI. The optional use of an IRES sequence will allow both the VHL construct and NOI to be expressed under the control of one regulatory sequence.

Typically The VHL peptide, or other peptide that inhibits VHL function may be used in conjunction with an HRE autoregulated system where HRE controls expression of the therapeutic/reporter gene in concert with expression of a HIF transcription factor. The latter may also be coordinately regulated by use of an IRES sequence.

With respect to suitable peptides for inhibiting VHL function, the domain structure of proteins allows the design of proteins that retain certain features but are functionally silent for other e.g. maintain binding to one member of the complex but not to others. Expression of such a mutant protein would have a dominant negative effect on the normal protein:protein interaction ie would compete for binding with the wild type endogenous protein.

An example of this would be to express mutant VHL (this could be designed using natural mutations found in patients with VHL-associated disease e.g. renal cell ca, hemangioblastomas) this would result in a reduction in proteolytic targeting of HIF and, as a consequence, increased HRE-dependent gene expression. In nature VHL mutation is frequently observed in the context of aberrant normoxic expression of HRE regulated genes such as those of the glycolytic enzymes.

Thus, in another preferred embodiment, the NOI encodes a polypeptide capable of inhibiting binding of the tumour suppressor protein VHL (Maxwell et al., 1999) to one or more of elongin B, elongin C and Cul2 (Lonergan, 1998). As discussed above, suitable polypeptides include variants of wild type VHL or its binding partners that can compete for binding to the multiprotein complex. Suitable polypeptides may be identified, by for example, *in vitro* binding assays using VHL and/or one of its binding partners. Other screening techniques include two hybrid screens to identify interacting sequences.

A particularly preferred peptide is a peptide consisting essentially of amino acids 157 to 172 of the full length VHL sequence.

Alternatively, the polypeptide capable of inhibiting binding of the tumour suppressor protein VHL to one or more of elongin B, elongin C and Cul2 may be introduced by other means, such as by separately transfecting a construct encoding the polypeptide into target cells, or by introducing pre-synthesised peptides.

Alternative approaches include expressing in a target cell inhibitory RNA molecules that effect the direct or indirect cleavage of VHL mRNA and/or precursor RNA. Inhibitory RNA molecules include antisense VHL constructs, ribozymes that cleave VHL mRNA and external guide sequences (EGSs). NOIs encoding said inhibitory RNA molecules may be introduced into nucleotide constructs, vectors and viral vectors of the present invention in a similar manner to that described above and below for other NOIs. Antisense VHL constructs are also included in a general sense since antisense inhibitory effects are mediated by mechanisms other than RNase cleavage. Thus NOIs that encode inhibitory RNA molecules that bind to and prevent VHL RNA processing and/or expression, for example splicing, transport and/or translation, are also included.

Thus in another embodiment of the invention, the NOI encodes (i) one or more inhibitory RNA molecules that effect the direct or indirect cleavage of VHL mRNA and/or precursor RNA or (ii) one or more inhibitory RNA molecules that bind to and prevent VHL RNA processing and/or expression.

### C. Nucleic Acid Vectors

Polynucleotides of the invention can be incorporated into a recombinant replicable vector. The vector may be used to replicate the nucleic acid in a compatible host cell. Thus, there is described a method of making polynucleotides of the invention by introducing a polynucleotide of the invention into a replicable vector, introducing the vector into a compatible host cell, and growing the host cell under conditions which bring about replication of the vector. The vector may be recovered from the host cell. Suitable host cells include bacteria such as *E. coli*, yeast, mammalian cell lines and other eukaryotic cell lines, for example insect Sf9 cells.

A vector comprising a polynucleotide of the invention which is operably linked to an NOI can be considered to be an expression vector since under suitable conditions, the NOI will be expressed under the control of the HRE/promoter construct of the present invention. However, it is not necessary for a vector of the invention to comprise an NOI. Nonetheless it is possible to introduce an NOI into the vector at a later stage. Thus a vector of the invention which lacks an NOI can be considered to be a cloning vector. Preferably, a cloning vector of the invention comprises a multiple cloning site downstream of the HRE/promoter sequences to enable an NOI to be cloned into the vector when required whereby it is then operably linked to the HRE/promoter sequences.

The vectors may be for example; plasmids, chromosomes, artificial chromosomes or virus vectors. The vectors may contain one or more selectable marker genes, for example an ampicillin resistance gene in the case of a bacterial plasmid or a neomycin resistance gene for a mammalian vector. Vectors may be used, for example, to transfect, transform or transduce a host cell either *in vitro* or *in vivo.*

Non-viral delivery systems include but are not limited to DNA transfection methods. Here, transfection includes a process using a non-viral vector to deliver a gene to a target mammalian cell. Typical transfection methods include electroporation, DNA biolistics, lipid-mediated transfection, compacted DNA-mediated transfection, liposomes, immunoliposomes, lipofectin, cationic agent-mediated, cationic facial amphiphiles (CFAs), multivalent cations such as spermine, cationic lipids or polylysine, 1, 2,-bis (oleoyloxy)-3-(trimethylammonio) propane (DOTAP)-cholesterol complexes and combinations thereof.

Viral delivery systems include but are not limited to an adenovirus vector, an adeno-associated viral (AAV) vector, a herpes viral vector, a retroviral vector, such as a lentiviral vector, a baculoviral vector and combination vectors such as an adenolenti viral vector. In the case of viral vectors, gene delivery is typically mediated by viral infection of a target cell.

### D. Viral Vectors

### Retroviruses

The retroviral vector of the present invention may be derived from or may be derivable from any suitable retrovirus. A large number of different retroviruses have been identified.

Examples include: murine leukemia virus (MLV), human immunodeficiency virus (HIV), simian immunodeficiency virus, human T-cell leukemia virus (HTLV). equine infectious anaemia virus (EIAV), mouse mammary tumour virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV). A detailed list of retroviruses may be found in Coffin et al., 1997, "Retroviruses", Cold Spring Harbour Laboratory Press Eds: JM Coffin, SM Hughes, HE Varmus pp 758-763.

Details on the genomic structure of some retroviruses may be found in the art. By way of example, details on HIV and Mo-MLV may be found from the NCBI Genbank (Genome Accession Nos. AF033819 and AF033811, respectively).

Retroviruses may be broadly divided into two categories: namely, "simple" and "complex". Retroviruses may even be further divided into seven groups. Five of these groups represent retroviruses with oncogenic potential. The remaining two groups are the lentiviruses and the spumaviruses. A review of these retroviruses is presented in Coffin *et al*., 1997 (*ibid*).

The lentivirus group can be split even further into "primate" and "non-primate". Examples of primate lentiviruses include human immunodeficiency virus (HIV), the causative agent of human auto-immunodeficiency syndrome (AIDS), and simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV).

A distinction between the lentivirus family and other types of retroviruses is that lentiviruses have the capability to infect both dividing and non-dividing cells. In contrast, other retroviruses - such as MLV - are unable to infect non-dividing cells such as those that make up, for example, muscle, brain, lung and liver tissue.

Each retroviral genome comprises genes called *gag, pol* and *env* which code for virion proteins and enzymes. In the provirus, these genes are flanked at both ends by regions called long terminal repeats (LTRs). The LTRs are responsible for proviral integration, and transcription. LTRs also serve as enhancer-promoter sequences and can control the expression of the viral genes. Encapsidation of the retroviral RNAs occurs by virtue of a *psi* sequence located at the 5' end of the viral genome.

The LTRs themselves are identical sequences that can be divided into three elements, which are called U3, R and U5. U3 is derived from the sequence unique to the 3' end of the RNA. R is derived from a sequence repeated at both ends of the RNA and U5 is derived from the sequence unique to the 5' end of the RNA. The sizes of the three elements can vary considerably among different retroviruses.

The basic molecular organisation of an infectious retroviral RNA genome is (5') R - U5 - *gag, pol, env -* U3-R (3'). In a defective retroviral vector genome *gag, pol* and *env* may be absent or not functional. The R regions at both ends of the RNA are repeated sequences. U5 and U3 represent unique sequences at the 5' and 3' ends of the RNA genome respectively.

Host range and tissue tropism varies between different retroviruses. In some cases, this specificity may restrict the transduction potential of a recombinant retroviral vector. For this reason, many gene therapy experiments have used MLV. A particular MLV that has an envelope protein called 4070A is known as an amphotropic virus, and this can also infect human cells because its envelope protein "docks" with a phosphate transport protein that is conserved between man and mouse. This transporter is ubiquitous and so these viruses are capable of infecting many cell types.

In some cases however, it may be beneficial, especially from a safety point of view, to target specifically restricted cells. Replacement of the *env* gene with a heterologous *env* gene is an example of a technique or strategy used to target specifically certain cell types.

This technique is called pseudotyping and examples may be found in WO-A-98/05759, WO-A-98/05754, WO-A-97/17457, WO-A-96/09400 and WO-A-91/00047.

The term "recombinant retroviral vector" (RRV) refers to a vector with sufficient retroviral genetic information to allow packaging of an RNA genome, in the presence of packaging components, into a viral particle capable of infecting a target cell. Infection of the target cell includes reverse transcription and integration into the target cell genome. The RRV in use typically carries non-viral coding sequences which are to be delivered by the vector to the target cell. An RRV is incapable of independent replication to produce infectious retroviral particles within the final target cell.

In a typical recombinant retroviral vector for use in gene therapy, at least part of one or more of the *gag, pol* and *env* protein coding regions essential for replication may be removed from the virus. This makes the retroviral vector replication-defective. The removed portions may even be replaced by an NOI to generate a virus capable of integrating its genome into a host genome but wherein the modified viral genome is unable to propagate itself due to a lack of structural proteins. When integrated in the host genome, expression of the NOI occurs - resulting in, for example, a therapeutic and/or a diagnostic effect. Thus, the transfer of an NOI into a site of interest is typically achieved by: integrating the NOI into the recombinant viral vector; packaging the modified viral vector into a virion coat; and allowing transduction of a site of interest - such as a targeted cell or a targeted cell population.

Replication-defective retroviral vectors are typically propagated, for example to prepare suitable titres of the retroviral vector for subsequent transduction, by using a combination of a packaging or helper cell line and the recombinant vector. That is to say, that the three packaging proteins can be provided *in trans*.

A "packaging cell line" contains one or more of the retroviral *gag, pol* and *env* genes. The packaging cell line produces the proteins required for packaging retroviral DNA but it cannot bring about encapsidation due to the lack of a *psi* region. However, when a recombinant vector carrying an NOI and a *psi* region is introduced into the packaging cell line, the helper proteins can package the *psi*-positive recombinant vector to produce the recombinant virus stock. This virus stock can be used to transduce cells to introduce the NOI into the genome of the target cells. It is preferred to use a *psi* packaging signal, called *psi* plus, that contains additional sequences spanning from upstream of the splice donor to downstream of the *gag* start codon (Bender *et al*., 1987) since this has been shown to increase viral titres.

The recombinant virus whose genome lacks all genes required to make viral proteins can tranduce only once and cannot propagate. These viral vectors which are only capable of a single round of transduction of target cells are known as replication defective vectors. Hence, the NOI is introduced into the host/target cell genome without the generation of potentially harmful retrovirus. A summary of the available packaging lines is presented in Coffin *et al*., 1997 (*ibid*).

Retroviral packaging cell lines in which the *gag, pol* and *env* viral coding regions are carried on separate expression plasmids that are independently transfected into a packaging cell line are preferably used. This strategy, sometimes referred to as the three plasmid transfection method (Soneoka *et al*., 1995), reduces the potential for production of a replication-competent virus since three recombinant events are required for wild type viral production. As recombination is greatly facilitated by homology, reducing or eliminating homology between the genomes of the vector and the helper can also be used to reduce the problem of replication-competent helper virus production.

An alternative to stably transfected packaging cell lines is to use transient transfected cell lines. Transient transfections may advantageously be used to measure levels of vector production when vectors are being developed. In this regard, transient transfection avoids the longer time required to generate stable vector-producing cell lines and may also be used if the vector or retroviral packaging components are toxic to cells. Components typically used to generate retroviral vectors include a plasmid encoding the gag/pol proteins, a plasmid encoding the env protein and a plasmid containing an NOI. Vector production involves transient transfection of one or more of these components into cells containing the other required components. If the vector encodes toxic genes or genes that interfere with the replication of the host cell, such as inhibitors of the cell cycle or genes that induce apotosis, it may be difficult to generate stable vector-producing cell lines, but transient transfection can be used to produce the vector before the cells die. Also, cell lines have been developed using transient transfection that produce vector titre levels that are comparable to the levels obtained from stable vector-producing cell lines (Pear *et al*., 1993).

It is highly desirable to use high-titre virus preparations in both experimental and practical applications. Techniques for increasing viral titre include using a *psi* plus packaging signal as discussed above and concentration of viral stocks. In addition, the use of different envelope proteins, such as the G protein from vesicular-stomatitis virus has improved titres following concentration to 10⁹ per ml (Cosset *et al*., 1995). Another technique is the split intron system for constructing retroviral vectors. This will be described later.

In addition to manipulating the retroviral vector with a view to increasing vector titre, retroviral vectors have also been designed to induce the production of a specific NOI in transduced cells. As already mentioned, the most common retroviral vector design involves the replacement of retroviral sequences with one or more NOIs to create replication-defective vectors. With regard to regulation of expression of the NOI, there are three main approaches currently in use.
1. The simplest approach has been to use the promoter in the retroviral 5' LTR to control the expresssion of a cDNA encoding an NOI or to alter the enhancer/promoter of the LTR to provide tissue-specific expression or inducibility. Where multiple NOIs are inserted, the additional downsteam NOIs can be expressed from a polycistronic mRNA by the use of internal ribosome entry sites.
2. The NOI may be operably linked to an internal heterologous promoter. This arrangement permits more flexibility in promoter selection. Additional NOIs can still be expressed from the 5'LTR or the LTR can be mutated to prevent expression following infection of a target cell.
3. The NOI is inserted together with regulatory control elements in the reverse orientation to the 5'LTR. Genomic sequences including enhancers, promoters, introns and 3' regions may be included. In this way it may be possible to achieve tightly regulated tissue-specific gene expression.

In addition, we have now shown that there is a particular advantage to configuring retroviral vectors, in particular lentiviral vectors, as single transcription unit vectors whereby the HRE/promoter construct of the invention is placed within the 3'LTR such that the resultant duplication of the 3'LTR also leads to duplication of the regulatory sequence (i.e. the 5'LTR of the provirus will contain the HRE/promoter construct duplicated from the 3'LTR). We have now shown that this arrangement enhances the activated response to hypoxia in a synergistic manner. Consequently, it is preferred to use a retroviral vector which comprises an HRE/promoter of the invention within its LTR. More specifically, the HRE/promoter construct (optionally together with any additional regulatory sequences such as tissue-specific enhancer elements) may be inserted into the 3' U3 region of the retroviral vector or the 5' U5 region, most preferably the 3' U3 region. Preferably, the NOI is not also inserted into the LTR since the resulting two copies in the provirus can decrease the size of the NOI which can be accommodated by the retroviral vector. Instead, the NOI is preferably inserted into the region of the retroviral vector which is normally occupied by the *env* gene.

The NOI may or may not include a selectable marker. If the vector contains an NOI that is not a selectable marker, the vector can be introduced into packaging cells by co-transfection with a selectable marker present on a separate plasmid. This strategy has an appealing advantage for gene therapy in that a single protein is expressed in the ultimate target cells and possible toxicity or antigenicity of a selectable marker is avoided. However, when the inserted gene is not selectable, this approach has the disadvantage that it is more difficult to generate cells that produce a high titre vector stock. In addition it is usually more difficult to determine the titre of the vector.

The current methodologies used to design retroviral vectors that express two or more proteins have relied on three general strategies. These include: (i) the expression of different proteins from alternatively spliced mRNAs transcribed from one promoter; (ii) the use of the promoter in the 5' LTR and internal promoters to drive transcription of different cDNAs and (iii) the use of internal ribosomal entry site (IRES) elements to allow translation of multiple coding regions from either a single mRNA or from fusion proteins that can then be expressed from an open reading frame.

Vectors containing internal heterologous promoters have been widely used to express multiple genes. An internal promoter makes it possible to exploit promoter/enhancer combinations other than the viral LTR for driving gene expression. Multiple internal promoters can be included in a retroviral vector and it has proved possible to express at least three different cDNAs each from its own promoter.

### Lentiviruses

The lentivirus group can be into "primate" and "non-primate". Examples of primate lentiviruses include human immunodeficiency virus (HIV), the causative agent of human auto-immunodeficiency syndrome (AIDS), and simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV).

A distinction between the lentivirus family and other types of retroviruses is that lentiviruses have the capability to infect both dividing and non-dividing cells. In contrast, other retroviruses - such as MLV - are unable to infect non-dividing cells such as those that make up, for example, muscle, brain, lung and liver tissue. Thus, lentiviral vectors may advantageously be used in the present invention since lentiviruses are capable of infecting a wide range of non-dividing cells, by contrast to certain other retroviruses that require cell division for stable integration.

A number of vectors have been developed based on various members of the lentivirus sub-familiy of the retroviridae and a number of these are the subject of patent applications (WO-A-98/18815; WO-A-97/12622). Preferred lentiviral vectors are based on HIV, SIV or EIAV. The simplest vectors constructed from HIV-1 have the complete HIV genome except for a deletion of part of the *env* coding region or replacement of the *nef* coding region. Notably these vectors express *gag*/*pol* and all of the accessory genes hence require only an envelope to produce infectious virus particles. Of the accessory genes *vif, vpr, vpu* and *nef* are non-essential. More recently however vectors have been described that are efficient yet lack most or all of the accessory factors, for example Blomer *et al*., 1997 and Kim *et al*., 1998. Thus a lentiviral vector of the invention preferably lacks at least one accessory gene, more preferably all accessory genes.

One preferred general format for HIV-based lentiviral vectors is, HIV 5'LTR and leader, some gag coding region sequences (to supply packaging functions), a reporter cassette, the rev response element (RRE) and the 3'LTR. In these vectors *gaglpol*, accessory gene products and envelope functions are supplied either from a single plasmid or from two or more co-transfected plasmids, or by co-infection of vector containing cells with HIV. More recently the lentiviral vector configurations have been further refined. For example self inactivating HIV vectors have been produced where the HIV LTR is deleted to restrict expression to the internal cassette (Myoshi *et al*., 1998).

In a preferred embodiment, the lentiviral vector of the present invention is a non-primate lentiviral vector, for example EIAV. We have shown recently (U.K. patent application nos. 9811037.2 and 9727135.7) that the amount of vector genomic sequence required from a non-primate lentivirus to produce an efficient cloning vector is substantially less than has been described for an HIV vector. We have shown that a minimal EIAV vector lacking large regions of the gag gene suffices for efficient packaging and indeed leads to higher viral titres. Thus, a minimal EIAV genome vector typically comprises a promoter and optionally an enhancer capable of directing expression of a retroviral vector genome comprising, in order, the following elements from an EIAV: part of the 5'LTR containing an R-region and a U5 region; sequences from the 5'untranslated region of the gag gene containing a functional packaging signal and a portion of the gag coding sequence; an insertion site for a gene of interest and a 3'LTR from EIAV. The 3' LTR may be a hybrid LTR containing at least the polypurine tract and R-U5 region from an EIAV and sequences from another source to replace all or part of the U3 region. Alternatively, the R region in the 5' and 3' LTRs may be replaced. WO-A-96/33623 describes a method for replacing both the U3 and R regions of retroviral vector genomes.

Preferably, the portion of the gag gene contains less than the first 350 nucleotides of the gag coding region, more preferably only the first 150 or 109 nucleotides of the gag coding region, only about the first 109 nucleotides being especially preferred.

In a particularly preferred embodiment of the first aspect of the invention, a hCMV-MIE promoter enhancer is used to direct expression of the retroviral RNA transcript. The U3 region enhancer may, for example, be replaced by a hypoxia responsive enhancer (HRE) of the present invention and an SV40 or MLV promoter.

The minimal EIAV vector also lacks S2, Tat, Rev and dUTPase genes but may still be used in vector production or for transduction of dividing and non-dividing cells. The viral genome may typically be packaged in cells by providing gagpol and env functions in *trans.*
For example DNA sequences encoding gagpol and env may be co-introduced into a cell along with the minimal vector as described above for retroviruses in general. Preferably the gagpol sequence is of non-primate lentiviral origin. It is particularly advantageous to include the leader sequences between the end of the 5' LTR and the ATG start codon of gag upstream of the gagpol coding sequence to provide for maximum gagpol expression.
It may also be desirable to include the Rev and/or RRE sequences although this is not essential and may be eliminated by codon optimisation of the EIAV gagpol.

### Retroviral Split Intron Technology

Transcription of the proviral DNA recreates the full length viral RNA genomic and subgenomic-sized RNA molecules that are generated by RNA processing. Typically, all RNA products serve as templates for the production of viral proteins. The expression of the RNA products is achieved by a combination of RNA transcript splicing and ribosomal frameshifting during translation.

RNA transcript splicing is carried out in higher eukaryotic cells by nuclear RNA processing machinery (the spliceosome) that recognises short consensus sequences at the boundaries of the sequences to be spliced. These consensus splice sites follow the so-called 'GT-AG rule' (although in the RNA sequence GT is of course GU). The 5' site, or the splice donor (SD), has a highly conserved GT dinucleotide at the first exon-intron boundary and the 3' site, or the splice acceptor (SA), has a highly conserved AG dinucleotide at the second exon-intron boundary. These consensus sequences confer directionality on the splicing process. Splice sites have been shown to be generic - they do not have specificity for particular RNA precursors and can be spliced by any cell. The splicing process also depends on the presence of a sequence within the region to be spliced out called a branch site. The branch site is usually lies between 18 to 40 nucleotides upstream of the 3' SA and is believed to identify the nearest SA as the correct splice site for connection to the 5' SD.

Unlike most cellular mRNAs, in which all introns are efficiently spliced, newly synthesised retroviral RNA must be diverted into two populations. One population remains unspliced to serve as the genomic RNA and the other population is spliced to provide subgenomic RNA.

In simple retroviruses, one population of newly synthesised retroviral RNA remains unspliced to serve as the genomic RNA and as mRNA for *gag* and *pol*. The other population is spliced, fusing the 5' portion of the genomic RNA to the downstream genes, most commonly *env*. Splicing is achieved with the use of a splice donor positioned upstream of *gag* and a splice acceptor near the 3' terminus of *pol*. The intron between the splice donor and splice acceptor that is removed by splicing contains the *gag* and *pol* genes. This splicing event creates the mRNA for envelope (Env) protein. Typically the splice donor is upstream of *gag* but in some viruses, such as ASLV, the splice donor is positioned a few codons into the *gag* gene resulting in a primary Env translation product that includes a few amino-terminal amino acid residues in common with Gag. The Env protein is synthesised on membrane-bound polyribosomes and transported by the cell's vesicular traffic to the plasma membrane, where it is incorporated into viral particles.

Complex retroviruses generate both singly and multiply spliced transcripts that encode not only the *env* gene products but also the sets of regulatory and accessory proteins unique to these viruses. Compex retroviruses such as the lentiviruses, and especially HIV, provide striking examples of the complexity of alternative splicing that can occur during retroviral infection. For example, it is now known that HIV-1 is capable of producing over 30 different mRNAs by sub-optimal splicing from primary genomic transcripts. This selection process appears to be regulated as mutations that disrupt competing splice acceptors can cause shifts in the splicing patterns and can affect viral infectivity.

The ability of higher eukaryotic cells to splice sequences between a 5' SD and a 3' SA can be utilised by retroviral vectors according to the present invention. In split-intron technology (described in PCT/GB98/02885), a retroviral vector is constructed which comprises a first nucleotide sequence (NS) capable of yielding a functional splice donor site (FSDS) and a second NS capable of yielding a functional splice acceptor site (FSAS); wherein the first NS is downstream of the second NS and is present in the sequences within the 3' LTR (the U3 region) of the retroviral vector that are duplicated in the 5' LTR as a result of reverse transcription of the RNA form of the retroviral vector into the DNA form that integrates into a host cell genome. Since the first NS capable of yielding an FSDS is downstream of the second NS capable of yielding an FSAS, splicing will not occur in the retroviral vector using these sites. However, in the reverse-transcribed DNA form of the vector, such as the integrated provirus, the first NS is now upstream of the second NS and thus a functional 5' splice donor - 3' splice acceptor pairing is generated. Consequently, when transcription occurs from the provirus, the resulting RNA is capable of being spliced to remove intervening sequences between the FSDS and the FSAS. Depending on the nature of the sequences which are spliced from RNA transcripts produced from the provirus, this process has several applications.

A preferred embodiment in which split-intron technology may preferably be used relates to a hybrid vector system (see above). In this system, the retroviral genome delivered by the primary viral vector, such as an adenoviral vector, to a primary target celll is in a configuration such the first NS is downstream of the second NS. Thus, splicing out of sequences between the first NS and second NS using the first NS as a splice donor and the second NS as a splice acceptor should not occur since they are in the wrong orientation. However, the viral particles packaged by the primary target cell may be used to infect a secondary target cell. In the secondary target cell, reverse transcription and integration of the viral genome will typically occur. This will result in a functional splice donor/splice acceptor configuration since the FSDS will then be located in the 5' LTR, i.e. upstream of the FSAS. Splicing may then occur to remove the sequences between the FSDS and FSAS.

The nucleotide sequence between the 5' LTR and the second NS in the retroviral vector that is found between the FSAS and FSDS in the provirus may contain an NOI. The NOI may contain sequences that are essential for production of viral particles, for example env or gagpol sequences. Since the NOI will be spliced out from proviral transcripts, the provirus will not be able to give rise to viable viral particles. This will, for example, allow construction of adeno retroviral vectors that contain NOIs encoding viral components and gene products of interest, such as therapeutic polypeptides, that have the added safety feature of not being able to produce said viral components when the viral particles assembled in the primary target cells are introduced into secondary target cells.

A further embodiment allows the expression of an NOI in a secondary target cell and not a primary target cell. In one alternative of this embodiment, removal of the intervening sequences between the FSDS and the FSAS in the proviral RNA transcript brings a regulatory sequence that is 5' to the FSDS in the proviral genome near to the 5' end of an NOI that was 3' to the FSAS in the proviral genome such that the regulatory sequence is now operably linked to the NOI and can direct transcription from the NOI whereas when the intervening sequences were present, the regulatory sequence was not operably linked to the NOI. Although the regulatory sequence may be upstream of the NOI in the retroviral vector, in a preferred embodiment the regulatory sequence is upstream of the first NS in the retroviral vector, within the U3 region of the 3' LTR such that it is downstream of the NOI. Thus, only when reverse transcription of the retroviral RNA takes place in the secondary target cells is the regulatory sequence positioned 5' of the NOI.

In summary, therefore, NOIs which are positioned between the FSDS and the FSAS (and are therefore upstream of the second NS in the retroviral vector) are sequences that will typically be spliced out of the viral RNA transcript produced by the provirus in the secondary target cell. Such NOIs may therefore include, but are not limited to, retroviral sequences required for assembly of retroviral particles in the primary cells. These include env sequences, gagpol sequences, sequences encoding essential accessory genes and/or packaging sequences. NOIs which are positioned downstream of the FSAS in the proviral genome (and therefore also downstream of the second NS in the retroviral vector) will therefore typically include, but are generally not limited to, sequences encoding gene products that it is desired to express in the secondary target cells, such as therapeutic products.

It will be appreciated that in the context of an adenoretroviral hybrid system, the reference to retroviral vector includes the retroviral genome sequences present in a primary adenoretroviral vector as well as the subsequent RNA genome produced in the primary target cells and subsequently packaged into viral particles.

As discussed above, functional splicing sequences also require a branch sequence which is found 5' and near to the FSAS (and consequently to the second NS) and therefore a suitable branch sequence should be present. It should also be appreciated that construction of split-intron vectors may required functional inactivation of existing functional splice donor sites, for example to prevent splicing in the retroviral RNA genome transcribed in the primary target cells. Typically, this may be achieved by mutating the GT dinucleotide consensus, for example to GC, using standard techniques.

### Adenoviruses

The adenovirus is a double-stranded, linear DNA virus that does not go through an RNA intermediate. There are over 50 different human serotypes of adenovirus divided into 6 subgroups based on the genetic sequence homology all of which exhibit comparable genetic organisation. Human adenovirus group C serotypes 2 and 5 (with 95% sequence homology) are most commonly used in adenoviral vector systems and are normally associated with upper respiratory tract infections in the young.

The adenoviruses/adenoviral vectors of the invention may be of human or animal origin. As regards the adenoviruses of human origin, preferred adenoviruses are those classified in group C, in particular the adenoviruses of type 2 (Ad2), 5 (Ad5), 7 (Ad7) or 12 (Ad12). More preferably, it is an Ad2 or Ad5 adenovirus. Among the various adenoviruses of animal origin, canine adenovirus, mouse adenovirus or an avian adenovirus such as CELO virus (Cotton *et al*., 1993) may be used. With respect to animal adenoviruses it is preferred to use adenoviruses of canine origin, and especially the strains of the CAV2 adenoviruses [manhattan strain or A26/61 (ATCC VR-800) for example]. Other adenoviruses of animal origin include those cited in application WO-A-94/26914 incorporated herein by reference.

As mentioned above, the organisation of the adenovirus genome is similiar in all of the adenovirus groups and specific functions are generally positioned at identical locations for each serotype studied. The genome of adenoviruses comprises an inverted terminal repeat (ITR) at each end, an encapsidation sequence (Psi), early genes and late genes. The main early genes have been classified into an array of intermediate early (E1a), delayed early (E1b, E2a, E2b, E3 and E4), and intermediate regions (see diagram below). Among these, the genes contained in the E1 region in particular are necessary for viral propagation. The main late genes are contained in the L1 to L5 regions. The genome of the Ad5 adenovirus has been completely sequenced and is available on a database (see particularly Genbank Accession No. M73260). Likewise, parts, or even all of other adenoviral genomes (such as Ad2, Ad7, Ad12) have also been sequenced.

For use as recombinant vectors, an adenovirus is typically modified so as to make it incapable of replicating in an infected cell.

Thus, constructs described in the prior art include adenoviruses deleted for the E1 region, essential for viral replication, into which are inserted the heterologous DNA sequences (Levrero *et al*., 1991; Gosh-Choudhury *et al*., 1986). Moreover, to improve the properties of the vector, it has been proposed to create other deletions or modifications in the adenovirus genome. Thus, a heat-sensitive point mutation has been introduced into the ts125 mutant, making it possible to inactivate the 72 kDa DNA-binding protein (DBP). Preferably, a recombinant adenoviral vector used in the invention comprises a deletion in the E 1 region of its genome. More particularly, it comprises a deletion in the E1a and E1b regions. According to a particularly preferred mode, the E1 region is inactivated by deletion of a PvuII-BglII fragment stretching from nucleotide 454 to nucleotide 3328, in the Ad5 adenovirus sequence (Genbank Accession No. M73260). In another preferred embodiment, the E1 region is inactivated by deletion of an HinfII-Sau3A fragment stretching from nucleotide 382 to nucleotide 3446.

Other adenoviral vectors comprise a deletion of another region essential for viral replication and/or propagation, the E4 region. The E4 region is involved in the regulation of the expression of the late genes, in the stability of the late nuclear RNAs, in decreasing host cell protein expression and in the efficiency of the replication of the viral DNA. Adenoviral vectors in which the E1 and E4 regions are deleted therefore possess very reduced viral gene expression and transcriptional background noise. Such vectors have been described for example in applications WO-A-94/28152, WO-A-95/02697, WO-A-96/22378. In addition, vectors carrying a modification of the IVa2 gene have also been described (WO-A-96/10088).

According to a preferred variant, a recombinant adenoviral vector used in the invention comprises, in addition, a deletion in the E4 region of its genome. More particularly, the deletion in the E4 region affects all the open reading frames. There may be mentioned, by way of a precise example, deletions of nucleotides 33466-35535 or 33093-35535. In particular, preferred vectors comprise a deletion of the whole of the E4 region. This may be carried deletion or excision of an MaeII-MscI fragment corresponding to nucleotides 35835-32720. Other types of deletions in the E4 region are described in applications WO-A-95/02697 and WO-A-96/22378, incorporated herein by reference.

Alternatively, only a functional part of E4 is deleted. This part comprises at least the ORF3 and ORF6 frames. By way of example, these coding frames can be deleted from the genome in the form of PvuII-AluI and BglII-PvuII fragments respectively, corresponding to nucleotides 34801-34329 and 34115-33126 respectively. The deletions of the E4 region of the virus Ad2 dl808 or of viruses Ad5 dl1004, Ad5 dl1007, Ad5 dl1011 or Ad5 dl1014 can also be used within the framework of the invention.

The positions given above refer to the wild-type Ad5 adenovirus sequence as published and accessible on a database. Although minor variations may exist between the various adenovirus serotypes, these positions are generally applicable to the construction of recombinant adenoviruses according to the invention from any serotype, and especially the adenoviruses Ad2 and Ad7.

Moreover, the adenoviruses produced may possess other alterations in their genome. In particular, other regions may be deleted to increase the capacity of the virus and reduce its side effects linked to the expression of viral genes. Thus, all or part of the E3 or IVa2 region in particular may be deleted. As regards the E3 region, it may however be particularly preferred to conserve the part encoding the gp19K protein. This protein indeed makes it possible to prevent the adenoviral vector from becoming the subject of an immune reaction which (i) would limit its action and (ii) could have undesirable side effects. According to a specific mode, the E3 region is deleted and the sequence encoding the gp19K protein is reintroduced under the control of a heterologous promoter.

The polynucleotide of the invention/NOI can be inserted into various sites of the recombinant genome. It can be inserted at into the E1, E3 or E4 region, as a replacement for the deleted or surplus sequences. It can also be inserted into any other site, outside the sequences necessary *in cis* for the production of the viruses (ITR sequences and encapsidation sequence).

The E2 region is essential as it encodes the 72 kDa DNA binding protein, DNA polymerase and the 80 kDa precurser of the 55 kDa Terminal Protein (TP) needed for protein priming to initiate DNA synthesis.

An alternative approach to making a more defective virus has been to "gut" the virus completely maintaining only the terminal repeats required for viral replication. The "gutted" or "gutless" viruses can be grown to high titres with a first generation helper virus in the 293 cell line.

The recombinant adenoviruses are typically produced in an encapsidation cell line, which is a cell line capable of complementing *in trans* one or more of the functions deficient in the recombinant adenoviral genome. One of these lines is for example line 293, into which part of the adenovirus genome has been integrated. More precisely, line 293 is a human kidney embryonic cell line containing the left end (about 11-12%) of the genome of serotype 5 adenovirus (Ad5), comprising the left ITR, the encapsidation region, the E1 region, including E1a and E1b, the region encoding protein pIX and part of the region encoding protein pIVa2. This line is capable of transcomplementing recombinant adenoviruses defective for the E1 region, that is to say lacking all or part of the E1 region, and of producing viral stocks having high titres. This line is also capable of producing, at a permissive temperature (32°C), virus stocks comprising, in addition, the heat-sensitive E2 mutation.

Other cell lines capable of complementing the E1 region have been described, based in particular on human lung carcinoma cells A549 (WO-A-94/28152) or on human retinoblasts (Hum. Gen. Ther. (1996) 215). Moreover, cell lines capable of transcomplementing several adenovirus functions have also been described, for example cell lines complementing the E1 and E4 regions (Yeh *et al*., 1996a, b; Krougliak *et al*., 1995) and lines complementing the E1 and E2 regions (WO-A-94/28152, WO-A-95/02697, WO-A-95/27071).

The recombinant adenoviruses are usually produced by introducing the viral DNA into the encapsidation line, followed by lysis of the cells after about 2 or 3 days (the kinetics of the adenoviral cycle being 24 to 36 hours). For carrying out the process, the viral DNA introduced may be the complete recombinant viral genome, optionally constructed in a bacterium (WO-A-96/25506) or in a yeast (WO-A-95/03400), transfected into the cells. It may also be a recombinant virus used to infect the encapsidation line. The viral DNA may also be introduced in the form of fragments each carrying part of the recombinant viral genome and a region of homology which makes it possible, after introduction into the encapsidation cell, to reconstitute the recombinant viral genome by homologous recombination between the various fragments.

Replication-competent adenoviruses can also be used for gene therapy. For example, the E1a gene can be inserted into a first generation virus under the regulation of a tumour-specific promoter. In theory, following injection of the virus into a tumour, it could replicate specifically in the tumour but not in the surrounding normal cells. This type of vector could be used either to kill tumour cells directly by lysis or to deliver a "suicide gene" such as the herpes-simplex-virus thymidine-kinase gene (HSV *tk*) which can kill infected and bystander cells following treatment with ganciclovir.

Thus, given that the HRE construct of the present invention may be preferentially active in certain tumour tissue by virtue of the hypoxic conditions that exist within many solid tumour masses, the present invention provides an adenovirus vector comprising a polynucleotide of the invention operably linked to a nucleic acid sequence encoding an adenoviral E1a polypeptide. The E1a polypeptide under the control of the HRE enhancer would only be expressed under hypoxic conditions and therefore the adenovirus would only be replication competent under hypoxic conditions. The adenovirus lacks an endogenous E1 gene, and preferably also lacks an endogenous E3 gene. Other regions of the adenovirus genome which may be deleted are described above. It may also be desirable to include all or part of the E3 gene under the control of a hypoxia response element such that host cell immune modulation is balances to obtain the correct viral spread within the tumour and immune response to infected cells.

An adenovirus defective only for E1b has been used specifically for antitumour treatment in phase-1 clinical trials. The polypeptides encoded by E1b are able to block p53-mediated apoptosis, preventing the cell from killing itself in response to viral infection. Thus, in normal non tumour cells, in the absence of E1b, the virus is unable to block apoptosis and is thus unable to produce infectious virus and spread. In tumour cells deficient in p53, the E1b defective virus can grow and spread to adjacent p53-defective tumour cells but not to normal cells. Again, this type of vector could also be used to deliver a therapeutic gene such as HSV *tk*.

Consequently, it is preferred that the E1a-expressing adenoviruses of the present invention lack a functional E1b gene.

Other essential viral genes may also be placed under the control of a hypoxia responsive regulatory element.

### Hybrid vector systems

Hybrid adenovirus/retrovirus systems may also be used whereby the features of adenoviruses are combined with the genetic stability of retroviruses. These hybrid viral vectors use the adenovirus to transduce target cells when then become transient retroviral producer cells that can stably infect neighbouring cells.

A hybrid viral vector system of the present invention comprises one or more primary viral vectors which encode a secondary viral vector, the primary vector or vectors being capable of infecting a first target cell and of expressing therein the secondary viral vector, which secondary vector is capable of transducing a secondary target cell.

Thus a genetic vector of the invention consists of a primary vector manufactured *in vitro* which encodes the genes necessary to produce a secondary vector *in vivo.* In use, the secondary vector carries one or more selected genes for insertion into the secondary target cell. The selected genes may be one or more marker genes and/or therapeutic genes (see above).

The primary viral vector or vectors may be a variety of different viral vectors, such as retroviral (including lentiviral), adenoviral, herpes virus or pox virus vectors, or in the case of multiple primary viral vectors, they may be a mixture of vectors of different viral origin. In whichever case, the primary viral vectors are preferably defective in that they are incapable of independent replication. Thus, they are capable of entering a target cell and delivering the secondary vector sequences, but not of replicating so as to go on to infect further target cells.

In the case where the hybrid viral vector system comprises more than one primary vector to encode the secondary vector, all of the primary vectors will be used to infect a primary target cell population, usually simultaneously. Preferably, there is a single primary viral vector which encodes all components of the secondary viral vector.

The preferred single or multiple primary viral vectors are adenoviral vectors. Adenovirus vectors have significant advantages over other viral vectors in terms of the titres which can be obtained from *in vitro* cultures. The adenoviral particles are also comparatively stable compared with those of enveloped viruses and are therefore more readily purified and stored.

The secondary viral vector is preferably a retroviral vector, more preferably a lentiviral vector. The construction of an adenolentiviral system is described in the Examples. The secondary vector is produced by expression of essential genes for assembly and packaging of a defective viral vector particle, within the primary target cells. It is defective in that it is incapable of independent replication. Thus, once the secondary retroviral vector has transduced a secondary target cell, it is incapable of spreading by replication to any further target cells.

The secondary vector may be produced from expression of essential genes for retroviral vector production encoded in the DNA of the primary vector. Such genes may include a gag-pol gene from a retrovirus, an envelope gene from an enveloped virus and a defective retroviral genome containing one or more therapeutic genes. The defective retroviral genome contains in general terms sequences to enable reverse transcription, at least part of a 5' long terminal repeat (LTR), at least part of a 3'LTR and a packaging signal.

Importantly, the secondary vector is also safe for *in vivo* use in that incorporated into it are one or more safety features which eliminate the possibility of recombination to produce an infectious virus capable of independent replication.

To ensure that it is replication defective the secondary vector may be encoded by a plurality of transcription units, which may be located in a single or in two or more adenoviral or other primary vectors. Thus, there may be a transcription unit encoding the secondary vector genome, a transcription unit encoding *gag-pol* and a transcription unit encoding *env.* Alternatively, two or more of these may be combined. For example, nucleic acid sequences encoding *gag-pol* and *env,* or *env* and the genome, may be combined in a single transcription unit. Ways of achieving this are known in the art.

Transcription units as described herein are regions of nucleic acid containing coding sequences and the signals for achieving expression of those coding sequences independently of any other coding sequences. Thus, each transcription unit generally comprises at least a promoter, an enhancer and a polyadenylation signal. The promoter and enhancer of the transcription units encoding the secondary vector are preferably strongly active, or capable of being strongly induced, in the primary target cells under conditions for production of the secondary viral vector. The promoter and/or enhancer may be constitutively efficient, or may be tissue or temporally restricted in their activity.

Safety features which may be incorporated into the hybrid viral vector system are described below. One or more such features may be present.

Firstly, sequence homology between the sequences encoding the components of the secondary vector may be avoided by deletion of regions of homology. Regions of homology allow genetic recombination to occur. In a particular embodiment, three transcription units are used to construct a secondary retroviral vector. A first transcription unit contains a retroviral *gag-pol* gene under the control of a non-retroviral promoter and enhancer. A second transcription unit contains a retroviral *env* gene under the control of a non-retroviral promoter and enhancer. A third transcription unit comprises a defective retroviral genome under the control of a non-retroviral promoter and enhancer. In the native retroviral genome, the packaging signal is located such that part of the *gag* sequence is required for proper functioning. Normally when retroviral vector systems are constructed therefore, the packaging signal, including part of the *gag* gene, remains in the vector genome. In the present case however, the defective retroviral genome contains a minimal packaging signal which does not contain sequences homologous to *gag* sequences in the first transcription unit. Also, in retroviruses, for example Moloney Murine Leukaemia virus (MMLV), there is a small region of overlap between the 3' end of the *pol* coding sequence and the 5' end of *env.* The corresponding region of homology between the first and second transcription units may be removed by altering the sequence of either the 3' end of the *pol* coding sequence or the 5' end of *env* so as to change the codon usage but not the amino acid sequence of the encoded proteins.

Secondly, the possibility of replication competent secondary viral vectors may be avoided by pseudotyping the genome of one retrovirus with the envelope protein of another retrovirus or another enveloped virus so that regions of homology between the *env* and *gag-pol* components are avoided. In a particular embodiment the retroviral vector is constructed from the following three components. The first transcription unit contains a retroviral *gag-pol* gene under the control of a non-retroviral promoter and enhancer. The second transcription unit contains the *env* gene from the alternative enveloped virus, under the control of a non-retroviral promoter and enhancer. The third transcription unit comprises a defective retroviral genome under the control of a non-retroviral promoter and enhancer. The defective retroviral genome contains a minimal packaging signal which does not contain sequences homologous to *gag* sequences in the first transcription unit.

Pseudotyping may involve for example a retroviral genome based on a lentivirus such as an HIV or equine infectious anaemia virus (EIAV) and the envelope protein may for example be the amphotropic envelope protein designated 4070A. Alternatively, the retroviral genome may be based on MMLV and the envelope protein may be a protein from another virus which can be produced in non-toxic amounts within the primary target cell such as an Influenza haemagglutinin or vesicular stomatitis virus G protein. In another alternative, the envelope protein may be a modified envelope protein such as a mutant or engineered envelope protein. Modifications may be made or selected to introduce targeting ability or to reduce toxicity or for another purpose.

Thirdly, the possibility of replication competent retroviruses can be eliminated by using two transcription units constructed in a particular way. The first transcription unit contains a *gag-pol* coding region under the control of a promoter-enhancer active in the primary target cell such as a hCMV promoter-enhancer or a tissue restricted promoter-enhancer. The second transcription unit encodes a retroviral genome RNA capable of being packaged into a retroviral particle. The second transcription unit contains retroviral sequences necessary for packaging, integration and reverse transcription and also contains sequences coding for an *env* protein of an enveloped virus and the coding sequence of one or more therapeutic genes.

In a preferred embodiment the hybrid viral vector system according to the invention comprises single or multiple adenoviral primary vectors which encodes or encode a retroviral secondary vector. Adenoviral vectors for use in the invention may be derived from a human adenovirus or an adenovirus which does not normally infect humans. Preferably the vectors are derived from Adenovirus Type 2 or adenovirus Type 5 (Ad2 or Ad5) or a mouse adenovirus or an avian adenovirus such as CELO virus. The vectors may be replication competent adenoviral vectors but are more preferably defective adenoviral vectors. Adenoviral vectors may be rendered defective by deletion of one or more components necessary for replication of the virus. Typically, each adenoviral vector contains at least a deletion in the E1 region. For production of infectious adenoviral vector particles, this deletion may be complemented by passage of the virus in a human embryo fibroblast cell line such as human 293 cell line, containing an integrated copy of the left portion of Ad5, including the E1 gene. The capacity for insertion of heterologous DNA into such vectors can be up to approximately 7 kb. Thus such vectors are useful for construction of a system according to the invention comprising three separate recombinant vectors each containing one of the essential transcription units for construction of the retroviral secondary vector.

Alternative adenoviral vectors are known in the art which contain further deletions in other adenoviral genes and these vectors are also suitable for use in the invention. Several of these second generation adenoviral vectors show reduced immunogenicity (eg E1 + E2 deletions Gorziglia *et al*., 1996; E1 + E4 deletions Yeh *et al*., 1996). Extended deletions serve to provide additional cloning capacity for the introduction of multiple genes in the vector. For example a 25 kb deletion has been described (Lieber *et al*., 1996) and a cloning vector deleted of all viral genes has been reported (Fisher *et al*., 1996) which will permit the introduction of more than 35 kb of heterologous DNA. Such vectors may be used to generate an adenoviral primary vector according to the invention encoding two or three transcription units for construction of the retroviral secondary vector.

Embodiments of the invention described solve one of the major problems associated with adenoviral and other viral vectors, namely that gene expression from such vectors is transient. The retroviral particles generated from the primary target cells can infect secondary target cells and gene expression in the secondary target cells is stably maintained because of the integration of the retroviral vector genome into the host cell genome. The secondary target cells do not express significant amounts of viral protein antigens and so are less immunogenic than the cells transduced with adenoviral vector.

The use of a retroviral vector as the secondary vector is also advantageous because it allows a degree of cellular discrimination, for instance by permitting the targeting of rapidly dividing cells. Furthermore, retroviral integration permits the stable expression of therapeutic genes in the target tissue, including stable expression in proliferating target cells.

Preferably, the primary viral vector preferentially infects a certain cell type or cell types. More preferably, the primary vector is a targeted vector, that is it has a tissue tropism which is altered compared to the native virus, so that the vector is targeted to particular cells. The term "targeted vector" is not necessarily linked to the term "target cell". "Target cell" simply refers to a cell which a vector, whether native or targeted, is capable of infecting or transducing.

Primary target cells for the vector system according to the invention include but are not limited to haematopoietic cells (including monocytes, macrophages, lymphocytes, granulocytes or progenitor cells of any of these); endothelial cells; tumour cells; stromal cells; astrocytes or glial cells; muscle cells; and epithelial cells.

Thus, a primary target cell according to the invention, capable of producing the second viral vector, may be of any of the above cell types. In a preferred embodiment, the primary target cell according to the invention is a monocyte or macrophage infected by a defective adenoviral vector containing a first transcription unit for a retroviral gag-pol and a second transcription unit capable of producing a packageable defective retroviral genome. In this case at least the second transcription unit is preferably under the control of a promoter-enhancer which is preferentially active in a diseased location within the body such as an ischaemic site or the micro-environment of a solid tumour. In a particularly preferred embodiment of this aspect of the invention, the second transcription unit is constructed such that on insertion of the genome into the secondary target cell, an intron is generated which serves to reduce expression of the viral *env* gene and permit efficient expression of a therapeutic gene.

The secondary viral vectors may also be targeted vectors. For retroviral vectors, this may be achieved by modifying the envelope protein. The envelope protein of the retroviral secondary vector needs to be a non-toxic envelope or an envelope which may be produced in non-toxic amounts within the primary target cell, such as for example a MMLV amphotropic envelope or a modified amphotropic envelope. The safety feature in such a case is preferably the deletion of regions or sequence homology between retroviral components.

The secondary target cell population may be the same as the primary target cell population. For example delivery of a primary vector of the invention to tumour cells leads to replication and generation of further vector particles which can transduce further tumour cells. Alternatively, the secondary target cell population may be different from the primary target cell population. In this case the primary target cells serve as an endogenous factory within the body of the treated individual and produce additional vector particles which can infect the secondary target cell population. For example, the primary target cell population may be haematopoietic cells transduced by the primary vector *in vivo* or *ex vivo.* The primary target cells are then delivered to or migrate to a site within the body such as a tumour and produce the secondary vector particles, which are capable of transducing for example tumour cells within a solid tumour.

The invention permits the localised production of high titres of defective retroviral vector particles *in vivo* at or near the site at which action of a therapeutic protein or proteins is required with consequent efficient transduction of secondary target cells. This is more efficient than using either a defective adenoviral vector or a defective retroviral vector alone.

The invention also permits the production of retroviral vectors such as MMLV-based vectors in non-dividing and slowly-dividing cells *in vivo.* It had previously been possible to produce MMLV-based retroviral vectors only in rapidly dividing cells such as tissue culture-adapted cells proliferating *in vitro* or rapidly dividing tumour cells *in vivo.* Extending the range of cell types capable of producing retroviral vectors is advantageous for delivery of genes to the cells of solid tumours, many of which are dividing slowly, and for the use of non-dividing cells such as endothelial cells and cells of various haematopoietic lineages as endogenous factories for the production of therapeutic protein products.

### Herpes Simplex Viruses

### 1. Viral Strains

The HSV vectors of the invention comprising a polynucleotide of the invention may be derived from, for example, HSV1 or HSV2 strains, or derivatives thereof, preferably HSV1. Derivatives include inter-type recombinants containing DNA from HSV1 and HSV2 strains. Derivatives preferably have at least 70% sequence homology to either the HSV 1 or HSV2 genomes, more preferably at least 90%, even more preferably 95%.

The use of HSV strains in therapeutic procedures will require the strains to be attenuated so that they cannot establish a lytic cycle. In particular, if HSV vectors are to be used for gene therapy in humans the polynucleotide should preferably be inserted into an essential gene. This is because if a vector virus encounters a wild-type virus transfer of a heterologous gene to the wild-type virus could occur by recombination. However as long as the polynucleotide is inserted into an essential gene this recombinational transfer would also delete the essential gene in the recipient virus and prevent 'escape' of the heterologous gene into the replication competent wild-type virus population.

Attenuated strains may be used to produce the HSV strain of the present invention, here given as examples only, including strains that have mutations in either ICP34.5 or ICP27, for example strain 1716 (MacLean *et al*., 1991), strains R3616 and R4009 (Chou and Roizman, 1992) and R930 (Chou *et al*., 1994) all of which have mutations in ICP34.5, and d27-1 (Rice and Knipe, 1990) which has a deletion in ICP27. Alternatively strains deleted for ICP4, ICP0, ICP22, ICP6, ICP47, *vhs* or gH, with an inactivating mutation in VMW65, or with any combination of the above may also be used to produce HSV strains of the invention.

The terminology used in describing the various HSV genes is as found in Coffin and Latchman, 1996. Herpes simplex virus-based vectors. In: Latchman DS (ed). Genetic manipulation of the nervous system. Academic Press: London, pp 99-114.

### 2. Complementing cell lines

HSV viruses defective in ICP27 are propagated in a cell line expressing ICP27, for example V27 cells (Rice and Knipe, 1990) or 2-2 cells (Smith *et al*., 1992). ICP27-expressing cell lines can be produced by co-transfecting mammalian cells, for example the Vero or BHK cells, with a vector, preferably a plasmid vector, comprising a functional HSV ICP27 gene capable of being expressed in said cells, and a vector, preferably a plasmid vector, encoding a selectable marker, for example neomycin resistance. Clones possessing the selectable marker are then screened further to determine which clones also express functional ICP27, for example on the basis of their ability to support the growth of ICP27⁻ mutant HSV strains, using methods known to those skilled in the art (for example as described in Rice and Knipe, 1990).

Cell lines which do not allow reversion of an ICP27⁻ mutant HSV strain to a strain with functional ICP27 are produced as described above, ensuring that the vector comprising a functional ICP27 gene does not contain sequences that overlap with (i.e. are homologous to) sequences remaining in the ICP27⁻ mutant virus.

Where HSV strains of the invention comprise inactivating modifications in other essential genes, for example ICP4, complementing cell lines will further comprise a functional HSV gene which complements the modified essential gene in the same manner as described for ICP27.

### 3. Methods of mutation

HSV genes may be rendered functionally inactive by several techniques well known in the art. For example, they may be rendered functionally inactive by deletions, substitutions or insertions, preferably by deletion. Deletions may remove portions of the genes or the entire gene. Inserted sequences may include the expression cassette described above.

Mutations are made in the HSV strains by homologous recombination methods well-known to those skilled in the art. For example, HSV genomic DNA is transfected together with a vector, preferably a plasmid vector, comprising the mutated sequence flanked by homologous HSV sequences. The mutated sequence may comprise deletions, insertions or substitutions, all of which may be constructed by routine techniques. Insertions may include selectable marker genes, for example *lacZ*, for screening recombinant viruses by, for example, β-galactosidase activity.

Mutations may also be made in other HSV genes, for example genes such as ICP0, ICP4, ICP6, ICP22, ICP47, VMW65, gH or *vhs*. In the case of the VMW65 gene, the entire gene is not deleted since it encodes an essential structural protein, but a small inactivating insertion is made which abolishes the ability of VMW65 to transcriptionally activate IE genes (Ace *et al*., 1989).

### 4. HSV strains comprising a polynucleotide of the invention

A polynucleotide of the invention may be inserted into the HSV genome at any location provided that the virus can still be propagated, which may require the use of a cell line carrying another HSV essential gene (as described in 2.) if the NOI is inserted into an essential gene. For example, if the polynucleotide is inserted into the ICP27 gene of the HSV strain, then a cell-line expressing ICP27 would be needed.The polynucleotide of the invention is preferably inserted into the region of the ICP27 mutation as in the unlikely event that the mutation is repaired by recombination with a wild-type virus, the repair would remove the inserted polynucleotide.

The polynucleotide of the invention may be inserted into the HSV genome by homologous recombination of HSV strains with, for example, plasmid vectors carrying the expression cassette flanked by HSV sequences, as described above for introducing mutations. The polynucleotide may be introduced into a suitable plasmid vector comprising HSV sequences using cloning techniques well-known in the art.

It is particularly preferred that the polynucleotide of the invention is operably linked to LAT P2 sequences as described in WO-A-98/30707 (some of the disclosure of which is reproduced above) since this has been shown to dramatically improve long term expression in mammalian cells.

### E. Host cells and target cells

Polynucleotide constructs, nucleic acid vectors and viral vectors of the invention may be introduced into a variety of host cells. Host cells include both prokaryotic, for example bacterial, and eukaryotic, for example yeast and higher eukaryotic cells (such as insect, mammalian, for example human, cells). Host cells may be used to propagate both non-viral and viral vectors, for example to prepare nucleic acid vectors comprising a polynucleotide of the invention or to prepare high titre viral stocks.

Alternatively, host cells comprising a polynucleotide of the invention/NOI may be used in therapy, such as the use of macrophages discussed below in, for example, *ex vivo* therapy.

Non-viral nucleic acids and viral vectors are typically introduced into host cells using techniques well known in the art such as transformation or transfection. Viral vectors may also be introduced into host cells using by infection.

Target cells, in the context of the present invention, means cells of, or from, an organism that typically it is desired to treat, rather than simply cell lines. Target cells may be removed from the organism and subsequently returned after treatment, or targeted *in vivo.* Thus for example, tumour cells *in vivo* can be considered to be target cells.

Examples of target cells include tumour cells (see below for list of tumours), in particular, tumour cells under conditions of hypoxia. The target cell may be a growth-arrested cell capable of undergoing cell division such as a cell in a central portion of a solid tumour mass or a stem cell such as an HSC or a CD34⁺ cell. As a further alternative, the target cell may be a precursor of a differentiated cell such as a monocyte precursor, a CD33⁺ cell, or a myeloid precursor. The target cell may also be a differentiated cell such as a neuron, astrocyte, glial cell, microglial cell, macrophage, monocyte, epithelial cell, endothelial cell or hepatocyte. Target cells may be transfected or transduced either *in vitro* after isolation from an individual or may be transfected or transduced directly *in vivo.*

Preferred host cells/target cells include cells in which EPAS is expressed, more preferably cells in which EPAS is expressed but HIF-1 is not (or at much lower levels).

In a particularly preferred embodiment, haematopoietic stem cells such as macrophages are used as host cells/target cells. HSCs are pluripotent stem cells that give rise to all blood cell lineages in mammals. HSCs differentiate into various cell lineages under the influence of microenvironmental factors such as cell-to-cell interactions and the presence of soluble cell cytokines. Four major cell lineages arise from the HSCs. These include: erythroid (erythrocytes); megakaryocytic (platelets); myeloid (granulocytes and monocytes); and lymphoid (lymphocytes). Maturation of these cells occurs under the influence of a network of tissue specific protein regulators which have been given a variety of names including growth factors, cytokines and interleukins.

Macrophages, derived from monocytes from the bloodstream, have been used as a delivery vehicle for targeting drugs and therapeutic genes to solid tumours. It has been shown that macrophages continuatly enter solid tumours and congregate in poorly vascularised, ischaemic sites in breast carcinomas. Moreover, the degree of ischaemia-induced necrosis in these tumours was positively correlated with the degree of intra-tumoral macrophage infiltration.

Monocytes and macrophages also infiltrate ischaemic lesions which are a feature of other disease states including cerebral malaria, coronary heart disease and rheumatoid arthritis. Thus monocytes and macrophages are suitable best cells for use in the present invention. In particular, monocytes and/or macrophages comprising polynucleotides and/or vectors, such as viral vectors, of the invention are suitable for use in *ex vivo* and *in vivo* methods for treating diseases associated with hypoxia.

Methods for isolation of HSCs and their maintenance and differentiation in culture are known in the art (Santiago-Schwartz *et al* 1992; Charbord *et al*., 1996; Dao *et al*., 1997; Piacibello *et al*., 1997) and in WO-A-91/09938. Retroviral gene transfer into human HSCs in the general sense has been reported (Duphar and Emmons, 1994). Methods for retroviral mediated transduction of HSCs and transfer to patients are also described by Dunbar *et al*., 1996.

*In vivo* murine studies have indicated that the pretreatment of donor mice with 5-fluorouracil prior to harvest of bone marrow can improve transduction efficiencies by inducing the cycling of primitive cells and increasing the susceptibility to retroviral infection and integration. The co-culture of target cells with a retroviral producer cell line and the use of cell lines capable of producing at least 10⁵ viral particles per ml has also improved efficiencies (Bodine *et al*., 1991). Successful gene transfer into long term re-copulating cells has been achieved in virtually all recipient mice with reconstitution of multiple haematopoietic lineages stably with 1-50% or more cells carrying the proviral genome (Fraser *et al*., 1990).

Where the invention uses a vector for delivery of an NOI to HSCs *in vivo*, the vector is preferably a targeted vector capable of targeting CD34⁺ HSCs.

The term "targeted vector" refers to a vector whose ability to infect/transfect a cell or to be expressed in the target cell is restricted to certain cell types within the host organism, usually cells having a common or similar phenotype. An example of a targeted vector is a targeted retroviral vector with a genetically modified envelope protein which binds to cell surface molecules found only on a limited number of cell types in the host organism. Another example of a targeted vector is one which contains promoter and/or enhancer elements which only-permit expression of one or more retroviral transcripts in a proportion of the cell types of the host organism. Thus, the vector may be provided with a ligand specific for CD34, such as an antibody or an immunoglobulin-like molecule directed against CD34. On introduction into an individual to be treated such a vector will specifically transfect CD34⁺ HSCs. The vector may be administered systemically, to the peripheral circulation.

### F. Therapeutic Uses

The polynucleotides, nucleic acid vectors, viral vectors and host cells of the present invention may be used in the treatment of tumours. Examples of tumours that may be treated by the present invention include but are not limited to: sarcomas including osteogenic and soft tissue sarcomas, carcinomas such as breast, lung, bladder, thyroid, prostate, colon, rectum, pancreas, stomach, liver, uterine, and ovarian carcinoma, lymphomas including Hodgkin and non-Hodgkin lymphomas, neuroblastoma, melanoma, myeloma, Wilms tumour, and leukemias, including acute lymphoblastic leukemia and acute myeloblastic leukemia, gliomas and retinblastomas.

As a further example of targeting of tumours, patients with mutations in the VHL locus are known to have an increased incidence of haemagiomas and renal cell carcinomas. Furthermore, certain renal cell carcinomas have spontaneous atterations in the VHL gene. Tumour cells in these patients will have constitutively increased levels of HIF expression, which will in turn stimulate expression driven by an HRE construct regardless of the normoxia and hypoxia in the cell. Consequently, introduction of a nucleotide sequence comprising an NOI encoding a therapeutic gene product operably linked to an HRE, such as an HRE of the invention, into a cell having increased levels of HIF expression, for example as a result in a mutation at the VHL locus, may be useful in treating or prevent a condition associated with one or more mutations at the VHL locus which mutations result in inhibition of VHL function. Examples of such conditions include haemagiomas and renal cell carcinomas.

A particularly advantageous feature of the present invention is that NOIs may be expressed in hypoxic cells and not cells under normoxic conditions. Thus polynucleotides, nucleic acid vectors, viral vectors and host cells of the present invention may be used in the clinical management of a range of conditions characterised by hypoxia. Example of conditions which are characterised by symptoms of hypoxia include stroke, deep vein thrombosis, pulmonary embolus and renal failure. The cell death of cardiac tissue, called myocardial infarction, is due in large part to tissue damage caused by ischemia and/or ischemia followed by reperfusion. Other examples include cerebral malaria and rheumatoid arthritis. It is especially preferred to use polynucleotides, nucleic acid vectors, viral vectors and host cells of the present invention in the clinical management of solid tumours such as ovarian tumours, in particular tumours comprising tumour cells under hypoxic conditions. Treatment may effect a slowdown in the rate of tumour growth, a cessation in the rate of tumour growth or indeed shrinkage of tumour mass without necessarily resulting in complete apoptotic/necrotic death of all malignant cells in an affected patient.

The polynucleotides, nucleic acid vectors, viral vectors and host cells of the present invention may also be used in preventative medicine. Thus the NOIs used in the invention may have a therapeutic effect via prophylaxis. For example, where an increased risk of developing cancer is diagnosed, the invention may be used to vaccinate the at-risk individual.

Suitability for prophylaxis may be based on genetic predisposition to cancer, for example cancer of the breast or ovary because of one or more mutations in a BRCA-1 gene, a BRCA-2 gene (Cornelisse *et al*., 1996) or another relevant gene.

### G. Administration

The polynucleotide, nucleic acid vectors and viral vectors of the invention may thus be used to deliver therapeutic genes to a human or animal in need of treatment.

The polynucleotide of the invention may be administered directly as a naked nucleic acid construct, preferably further comprising flanking sequences homologous to the host cell genome. Uptake of naked nucleic acid constructs by mammalian cells is enhanced by several known techniques including biolistic transformation and lipofection. Alternatively, the polynucleotide may be administered as part of a nucleic acid vector, including a plasmid vector or viral vector, preferably a lentiviral vector.

Preferably the delivery vehicle (i.e. naked nucleic acid construct or viral vector comprising the polynucleotide for example) is combined with a pharmaceutically acceptable carrier or diluent to produce a pharmaceutical composition. Thus, the present invention also provides a pharmaceutical composition for treating an individual by gene therapy, wherein the composition comprises a therapeutically effective amount of the polynucleotide, vector or viral vector of the present invention comprising one or more deliverable therapeutic and/or diagnostic NOI(s) or a viral particle produced by or obtained from same, together with a pharmaceutically acceptable carrier, diluent, excipient or adjuvant. The pharmaceutical composition may be for human or animal usage.

The choice of pharmaceutical carrier, excipient or diluent can be selected with regard to the intended route of administration and standard pharmaceutical practice. Suitable carriers and diluents include isotonic saline solutions, for example phosphate-buffered saline. The pharmaceutical compositions may comprise as - or in addition to - the carrier, excipient or diluent any suitable binder(s), lubricant(s), suspending agent(s), coating agent(s), solubilising agent(s), and other carrier agents that may aid or increase the viral entry into the target site (such as for example a lipid delivery system).

The pharmaceutical composition may be formulated for parenteral, intramuscular, intravenous, intracranial, subcutaneous, intraocular or transdermal administration.

Where appropriate, the pharmaceutical compositions can be administered by any one or more of: inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intracavemosally, intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

The pharmaceutical composition is administered in such a way that the polynucleotide/vector containing the therapeutic gene for gene therapy, can be incorporated into cells at an appropriate area.

When the polynucleotide of the invention is delivered to cells by a viral vector of the invention, the amount of virus administered is in the range of from 10³ to 10¹⁰ pfu, preferably from 10⁵ to 10⁸ pfu, more preferably from 10⁶ to 10⁷ pfu. When injected, typically 1-10 µl of virus in a pharmaceutically acceptable suitable carrier or diluent is administered.

When the polynucleotide/vector is administered as a naked nucleic acid, the amount of nucleic acid administered is typically in the range of from 1 µg to 10 mg, preferably from 100 µg to 1 mg.

Where the NOI is under the control of an inducible regulatory sequence, it may only be necessary to induce gene expression for the duration of the treatment. Once the condition has been treated, the inducer is removed and expression of the NOI is stopped. This will clearly have clinical advantages. Such a system may, for example, involve administering the antibiotic tetracycline, to activate gene expression via its effect on the tet repressor/VP16 fusion protein.

The use of tissue-specific promoters will be of assistance in the treatment of disease using the polynucleotides/vectors of the invention. For example, several neurological disorders are due to aberrant expression of particular gene products in only a small subset of cells. It will be advantageous to be able express therapeutic genes in only the relevant affected cell types, especially where such genes are toxic when expressed in other cell types.

Modified HSCs of the invention are administered to a patient or an at-risk individual in a suitable formulation. The formulation may include an isotonic saline solution, a buffered saline solution or a tissue-culture medium. The cells are administered by bolus injection or by infusion intravenously or directly to the site of a tumour or to the bone marrow at a concentration of for example between approximately 10⁶ and of the order of 10¹² cells / dose, preferably at least 10⁸ or 10¹⁰ cells per dose.

The individual may first be treated to deplete the bone marrow of stem cells or may be treated with one or more cytokines such as G-CSF to increase the mobilisation of stem cells into the peripheral blood or one or more cytokines to enhance repopulation of bone marrow. Combinations of such treatments are also envisaged. The treatments of the invention may also be combined with currently available anti-cancer therapies.

In the event that the vector used for stem cell engineering encodes a pro-drug activating enzyme, the individual suffering from cancer is additionally treated with the corresponding pro-drug, administered using an appropriate regimen according to principles known in the art.

Where the HSCs are removed from the individual to be treated and are transfected or transduced with the vector *in vitro*, the cells are generally expanded in culture prior to and after introduction of the NOI or NOIs. When cultured *in vitro* under appropriate conditions or when appropriate signals are received *in vivo*, HSC have the capacity to differentiate into, among other cell types, endothelial cells, myeloid cells, dendritic cells and immune effector cells such as neutrophils, lymphocytes, mononuclear phagocytes and NK cells.

This involves the use of tissue culture methods which are known in the art and include exposure to cytokines and/or growth factors for the maintenance of HSCs (Santiago-Schwartz *et al*., 1992; Charbord *et al*., 1996; Dao *et al*., 1997; Piacibello *et al*., 1997). Agents which induce the differentiation of the HSCs may also be added.

In addition to responding to hypoxia the HRE elements are known to respond to chemical inducers that mimic hypoxia. Two of these are known, these are cobalt chloride and desferrioxamine (Meliillo *et al*., 1996; Wang and Semenza 1993b). Thus, the products of the invention may also be used to treat a disorder where compounds that mimic hypoxia are administered, such as the chemical activator desferrioxamine or analogous chemicals used to treat neuroblastoma (Blatt, 1994), beta thalassemia (Giardina and Grady, 1995), Alzheimers disease (Crapper *et al*., 1991), VEGF deficiency (Beerrepoot *et al*., 1996), Erythropoetin deficiency (Wang and Semenza, 1993b) and for enhancement of tumour chemotherapy (Voest *et al*., 1993). The products of the invention may be adminstered concomitantly, sequentially or separately with the compounds that mimic hypoxia.

The routes of administration and dosages described are intended only as a guide since a skilled practitioner will be able to determine readily the optimum route of administration and dosage for any particular patient and condition.

In accordance with the invention, standard molecular biology techniques may be used which are within the level of skill in the art. Such techniques are fully described in the literature. See for example; Sambrook et al (1989) Molecular Cloning; a laboratory manual; Ausubel et al., Current Protocols in Molecular Biology (1999), John Wiley & Sons, Inc.; Hames and Glover (1985 - 1997) DNA Cloning: a practical approach, Volumes I- IV (second edition); Methods for the engineering of immunoglobulin genes are given in McCafferty et al., 1996, "Antibody Engineering: A Practical Approach".

It should be appreciated that features from various sections, aspects and embodiments of the invention as described above are generally equally applicable to other sections, aspects and embodiments *mutatis mutandis*.

The invention will now be further described by way of Examples, which are meant to serve to assist one of ordinary skill in the art in carrying out the invention and are not intended in any way to limit the scope of the invention. The Examples refer to the Figures. In the Figures:
Figure 1 is a pictorial representation of plasmid OB37. This plasmid contains the OBHRE1 or the OBHRE11promoter;
Figure 2a is a graph illustrating the relative strengths of synthetic hypoxic responsive promoters. The bars indicate the level of reporter gene activity. The numbers above the bars give the fold induction;
Figure 2b is a graph illustrating the hypoxia-mediated activation of a synthetic hypoxia responsive promoter in combination with the SV40 minimal promoter as compared with a CMV promoter.
Figure 3 is a graph illustrating that OBHRE11 mediates hypoxic induction in primary human macrophages;
Figure 4 shows photographs illustrating the induction of β-galactosidase expression in HT1080 cells transduced with Xiavector;
Figure 5 is a graph illustrating the induction of β-galactosidase expression in breast cancer cells transduced with Xiavector;
Figure 6 is a graph illustrating the effect on hypoxia-mediated expression of mutations in the 5' and/or 3' HRE of an artificial promoter construct linked to a sequence encoding luciferase.
Figure 7 shows a pictorial representation of a pKAHRE construct;
Figure 8 shows a schematic map of a retroviral XiaGen-P450 vector comprising a therapeutic gene under the control of an HRE;
Figure 9 shows a graph illustrating hypoxia activation of a reporter gene driven by the XiaMac promoter (labelled OBHREMAC) relative to the CMV promoter in macrophages and breast cancer cell lines;
Figure 10 is a graph showing that the HRE is critical for the hypoxic response of the XiaMac promoter. The HRE was deleted in XiaMac (XiaMac-HRE) and no induction by hypoxia is observed;
Figure 11a shows a pictorial representation of a plasmid map of pEGASUS-4(+);
Figure 11b shows a pictorial representation of a plasmid map of pONY2.11acZ;
Figure 11c shows a pictorial representation of a plasmid map of pONYHRELucLac;
Figure 11d shows a pictorial representation of a plasmid map of pEGHRELacZ;
Figure 12 is a graph illustrating viral titre when a Pegasus vector expressing LacZ was plated onto cells in culture. Cells were then placed in normoxia or hypoxia.;
Figure 13 is a graph showing hypoxia mediated activation of a luciferase reporter in a lentiviral vector;
Figure 14 is a schematic of a PCR approach to introducing the HRE into an EIAV vector.
Figure 15 is a schematic of a hypoxia responsive EIAV vector configured as a single transcription unit;
Figure 16 is a schematic of a hypoxia responsive autoregulated EIAV vector configured as a single transcription unit;
Figure 17 shows a scheme for constructing recombinant adenoviral vectors Ad.OBHRElacZ is the OBHRElacZ cassette from OB37 inserted into the Microbix transfer vector pE1sp1A;
Figure 18 is a graph illustrating hypoxic induction (0.1%) from Ad.OBHRElacZ transduced Chiang Liver cells;
Figure 19 shows photographs of hypoxic regulation of β-galactosidase gene expression in primary human macrophages transduced with AdOBHRE11 LacZ;
Figure 20a shows a pictorial representation of a plasmid map of pE1HREPG;
Figure 20b shows a pictorial representation of a plasmid map of pE1CMVPG;
Figure 21 shows a pictorial representation of a plasmid map of pE1RevE;
Figure 22 shows a pictorial representation of a plasmid map of pE1HORSE3.1;

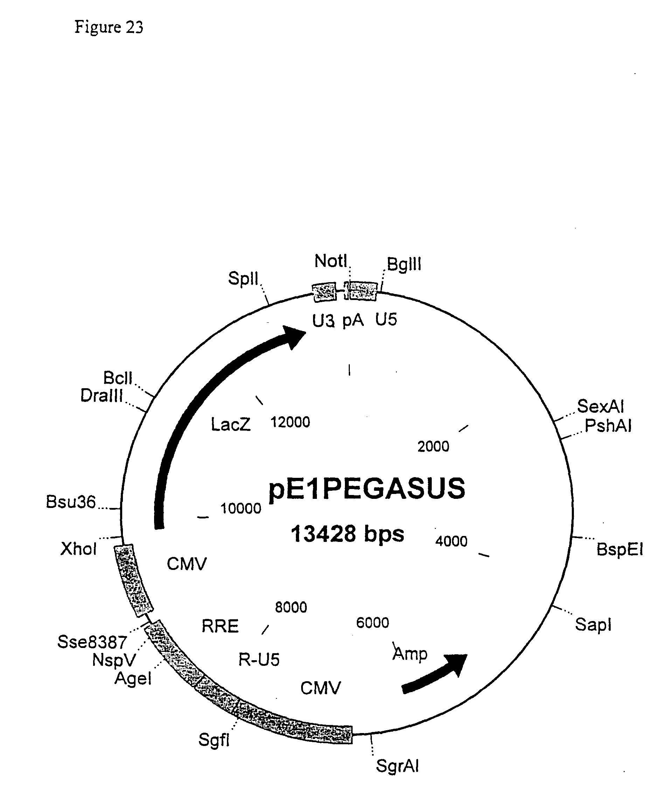

Figure 23 shows a pictorial representation of a plasmid map of pE 1PEGASUS;
Figure 24 shows a pictorial representation of a plasmid map of pCI-Neo;
Figure 25 shows a pictorial representation of a plasmid map of pCI-Rab; and
Figure 26 shows a pictorial representation of a plasmid map of pE1Rab.
Figure 27 is a graph illustrating hypoxic induction using various HRE constructs with or without HIF-1. LTR = 3 x PGK HRE upstream of an intact MLV promoter. -CAAT means core promoter lacks a CAAT box.
Figure 28 is a representation of an HRE HIF-1 autoregulated cassette.
Figure 29 is a graph illustrating hypoxic induction in T47D cells using various HRE constructs.
Figure 30 is a graph illustrating hypoxic induction in CTC12 cells using various HRE constructs.

### EXAMPLES

### Example 1 - Construction of an optimised hypoxia response element - OBhre11: a promoter based on the murine PGK HRE

A variety of HRE sequences are known in the art. However, as discussed above, the degree of hypoxia-restricted expression directed by these sequences in constructs used to date has not been entirely satisfactory. A suitable HRE construct should be capable of directing high levels of expression in a hypoxia-restricted manner. Consequently, we have tested several different constructs, including two novel constructs based on three mouse PGK HRE direct repeats cloned upstream of an SV40 promoter sequence, for hypoxia-specific activity to try an obtain a sequence with the desired characteristics.

### Materials and Methods

### Plasmid construction

Synthetic oligonucleotides are synthesised encompassing hypoxia response element (HRE) sequences and cloned as BglII/BamH1 fragments into the BamH1 site of the pGL3 promoter plasmid (Promega; Genbank accession no U47298). PGK sequences are synthesised as Xba1/Nhe1 fragments and cloned into the Nhe1 site of this vector. pGL3 is an enhancerless expression plasmid with a minimal SV40 promoter upstream of a luciferase coding sequence. Insertion of the HRE at this site places it upstream of the minimal SV40 promoter.

### The various enhancer/promoter sequences used in this assay are listed below:

### OBhre1

Trimer encompassing -307/-290 sequence of murine PGK in the natural orientation (Firth *et al*., 1995) linked to the SV40 promoter (italicised) (SEQ ID. No. 3).

GCTAGAGTCGTGCAGGACGTGACATCTAGTGTCGTGCAGGACGTGACA
NheI HRE HRE
TCTAGTGTCGTGCAGGACGTGACAGCTAGCCCGGGCTCGAGATCTGCG
HRE XbaI

ATCTGCATCTCAATTAGTCAGCAACCATAGTCCCGCCCCTAACTCCGCC
CATCCCGCCCCTAACTCCGCCCAGTTCCGCCCATTCTCCGCCCCATCG
CTGACTAATTTTTTTTATTTATGCAGAGGCCGAGGCCGCCTCGGCCTCTG
start start

Although the results given herein are for OBhre11 (see below), very similar results were obtained with constructs based on OBhre1.

### OBhre11

Similar to Obhre1 except that the middle HRE contains a deletion in the HIF-1 binding site (deleted residues between the two nucleotides shown in bold. (SEQ ID. No. 9)).

GCTAGAGTCGTGCAGGACGTGACATCTAGTGTCGTGCAGG          CA
NheI                    HRE                    mutant HRE
TCTAGTGTCGTGCAGGACGTGACAGCTAGCCCGGGCTCGAGATCTGCG
                        HRE          XbaI
ATCTGCATCTCAATTAGTCAGCAACCATAGTCCCGCCCCTAACTCCGCC
CATCCCGCCCCTAACTCCGCCCAGTTCCGCCCATTCTCCGCCCCATCG
CTGACTAATTTTTTTTATTTATGCAGAGGCCGAGGCCGCCTCGGCCTCTG
                                                    start  start

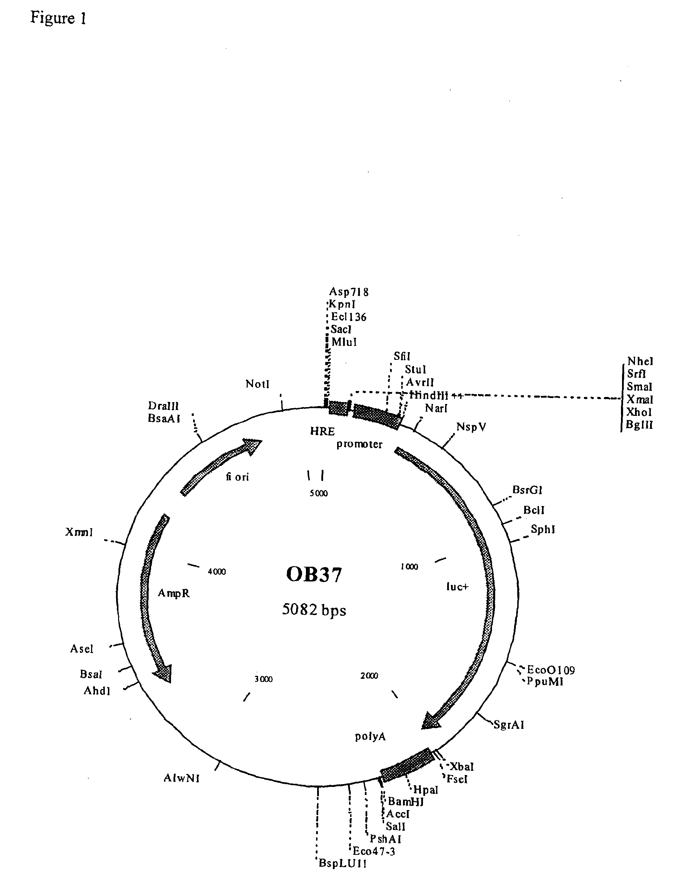

This promoter sequence is defined as OBhre11. The resulting plasmid is designated plasmid OB37 (Figure 1).

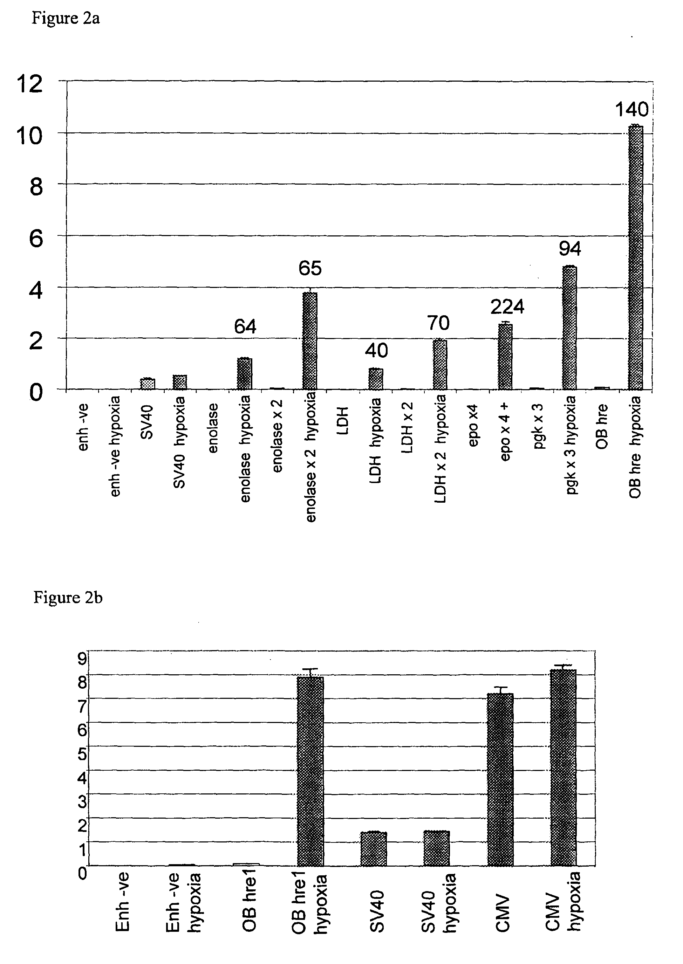

### PGK (PGK x 3 in Figure 2a).

As Obhre 1 but in the reverse orientation to the natural configuration. This construct is also novel.

CTAGCTGTCACGTCCTGCACGACACTAGATGTCACGTCCTGCACGACA
HRE HRE
CTAGATGTCACGTCCTGCACGACTCTAGCCCGGGCTCGAGATCTGCGA
HRE
TCTGCATCTCAATTAGTCAGCAACCATAGTCCCGCCCCTAACTCCGCCC
ATCCCGCCCCTAACTCCGCCCAGTTCCGCCCATTCTCCGCCCCATCGCT
GACTAATTTTTTTTATTTATGCAGAGGCCGAGGCCGCCTCGGCCTCTG

### Enolase A

Sequence includes three HRE consensus sites spaced as in the native human gene positions -413/-351. (Semenza *et al*., 1996).

GATCTGAGGGCCGGACGTGGGGCCCCAGAGCGACGCTGAGTGCGTGC
(HRE)                                    HRE

GGGACTCGGAGTACGTGACGGAGCCCCGGATCTGCATCTCAATTAGTC
HRE
AGCAACCATAGTCCCGCCCCTAACTCCGCCCATCCCGCCCCTAACTCC

GCCCAGTTCCGCCCATTCTCCGCCCCATCGCTGACTAATTTTTTTTATTT

ATGCAGAGGCCGAGGCCGCCTCGGCCTCTG

### Enolase x 2

As above but two copies of the synthetic sequence inserted.

GATCTGAGGGCCGGACGTGGGGCCCCAGAGCGACGCTGAGTGCGTGC
(HRE) HRE
GGGACTCGGAGTACGTGACGGAGCCCCGGATCTGAGGGCCGGACGTG
HRE (HRE)
GGGCCCCAGAGCGACGCTGAGTGCGTGCGGGACTCGGAGTACGTGAC
HRE (HRE)
GGAGCCCCGGATCT etc. of SV40 sequence

### Epo x 4

Tetramer of the human erythropoietin enhancer (positions +3065/3089, (HRE?) marks a site common to LDH that binds a constitutive factor that contributes to hypoxic response, Wang and Semenza, 1993.

GATCTGCCCTACGTGCTGTCTCACACAGCCTGGCCCTACGTGCTGTCTC
HRE (HRE?) HRE
ACACAGCCTGGATCTGCCCTACGTGCTGTCTCACACAGCCTGGCCCTA
(HRE?) HRE (HRE?)
CGTGCTGTCTCACACAGCCTGGGATCTGCATCTCAATTAGTCAGCAACC
HRE (HRE?)
ATAGTCCCGCCCCTAACTCCGCCCATCCCGCCCCTAACTCCGCCCAGT

TCCGCCCATTCTCCGCCCCATCGCTGACTAATTTTTTTTATTTATGCAGA

GGCCGAGGCCGCCTCGGCCTCTG

### LDH

This sequence derived from murine lactate dehydrogenase A (-88/-40) encompasses a consensus also found adjacent to the epo HRE, an HRE, and a CRE site that contribute to enhancer activity. (Firth *et al*., 1995). This is the natural orientation.

Note: In Firth *et al*. the sequence of the complementary strand is described CCAGCGGACGTGCGGGAACCCACGTGTAGG (-50/-88) written in the conventional 5' to 3' direction with the HRE underlined. An additional regulatory element is highlighted in italics.

GATCTCTACACGTGGGTTCCCGCACGTCCGCTGGGCTCCCACTCTGAC
(HRE?)                    HRE                    CRE
GTCAGCGGGATCTGCATCTCAATTAGTCAGCAACCATAGTCCCGCCCC
TAACTCCGCCCATCCCGCCCCTAACTCCGCCCAGTTCCGCCCATTCTCC
GCCCCATCGCTGACTAATTTTTTTTATTTATGCAGAGGCCGAGGCCGCC
TCGGCCTCTG

### LDH x 2

As above with 2 copies of the synthetic sequence.

GATCTCTACACGTGGGTTCCCGCACGTCCGCTGGGCTCCCACTCTGAC
(HRE?)          HRE
GTCAGCGGATCTCTACACGTGGGTTCCCGCACGTCCGCTGGGCTCCCA
CRE          (HRE?)          HRE
CTCTGACGTCAGCGGGATC etc. of SV40 sequence
CRE

### Cell lines

MCF-7 (ECACC) and HT1080 (ECACC) cell lines were maintained in Dulbecco's modified Eagle's medium. The T47D cell line was maintained in RPMI 1640 Media. All media was supplemented with 10% (v/v) Foetal calf serum, 2mM glutamine and 2mM non-essential amino acids. For transient assays the media was also supplemented with the antibiotics. All tissue culture media/solutions were purchased from Sigma (Dorset,UK)

### Luciferase Assays

Typically, cells seeded in a 24 well dish at a confluency of 60-80% were transfected with 0.21 µg of the reporter vector (pGL3 series of vectors purchased from Promega) using the Fugene-6 transfection reagent (Boehringer Mannheim, East Sussex, UK.). When required the reporter vector was co-transfected with 1/50th of the Renilla-based pRL-SV40 (Promega) vector to control for transfection efficiency. Following 16 hours (overnight) incubation (21% O₂, 5% CO₂, 74% N₂) transfected cells were either incubated for a further 16 hours under normoxic conditions in a standard incubator (21 % O₂, 5% CO_{2,} 74% N₂) or under hypoxic conditions (0.1% O₂/5% O₂, 5% CO_{2,} 74% N₂) using multigas incubators purchased from Heto (Surrey, UK)

Luciferase or beta-galactosidase chemiluminescent reporter assays were carried out using either the Promega Dual-Luciferase™ reporter assay sytem respectively with a Dynex Technologies (West Sussex) MLX Microtiter® plate luminometer. All reporter assays were carried out in triplicate.

### Protein Assays

These were carried out using the Biorad Detergent Compatible protein assay kit (BioRad, UK) together with a Dynex Technologies MRX Plate reader using a bovine serum albumin protein standard.

### Results

Luciferase assays are performed using T47D cells transfected with one of the above constructs to compare function of the different constructs in normoxia and hypoxia (0.1% oxygen. The results are shown in Figure 2a as relative expression levels. All these sequences confer on the minimal SV40 promoter hypoxia induced expression. However what is clear is the different induction ratios and the scale of expression under hypoxia. The OBhre11 construct shows over 2.5 times the relative levels of expression under hypoxic conditions compared with the best prior art construct (enolase x 2). The PGK x 3 construct (reverse orientation) also gives good results.

The OBhre11 construct is also tested against the strong viral promoters CMV and SV40 in the absence of HRE elements using a luciferase assay to measure expression levels. The results shown in Figure 2b demonstrate that OBhre11 directs low basal activity in normal oxygen tensions and is strongly induced in hypoxia such that expression levels are equivalent or greater than the CMV immediate early promoter. It should also be noted that the HRE elements silence the activity of the downstream SV40 promoter under normoxic conditions as is evidenced by the significant basal levels of transcription produced in normoxic conditions by the SV40 promoter alone which are dramatically reduced in the HRE/SV40 construct.

The extent of activation with the OBhre11 sequence was unexpected according to the published data on activation of HREs. Thus the OBhre11 promoter construct is a good candidate for development of vectors for use in delivering NOIs to tissues under hypoxic conditions. To test the properties of the OBhre11 promoter in the context of a viral vector delivery system, a fragment of the OBhre11 pGL3 plasmid (OB37) is isolated as a Mlu1/Xba1 fragment containing the PGK trimer/minimal SV40 promoter described above upstream of the luciferase coding sequence. This fragment is used as a starting point for the construction of the regulated lentiviral and adenoviral vectors described later.

### Example 2 - The PGK HRE elements in OBhre11, which contain a consensus sequence for transcription factor HIF-1, are capable of directing hypoxia mediated expression in macrophages, a cell type that lacks HIF-1 expression.

The HRE promoters described above all contain DNA sequence motifs for the classical HRE binding transcription factor, HIF-1. This is a heterodimer consisting of HIF 1α and HIF 1β, a member of the basic helix-loop-helix (bHLH)/PAS domain protein family (from the founding members of this gene family; Period, ARNT, and SIM). HIF 1α was originally identified as a protein binding to the HRE of the erythropopietin gene in hepatoma cells (Wang *et al*., 1995) but has since been implicated in the regulation of an expanding family of genes that are regulated by hypoxia in most cell types.

Macrophages infiltrate tumours to such an extent that they can form a significant proportion of the total tumour mass. In particular macrophages accumulate in areas of necrosis and thus hypoxia. Other ischemic diseases typified by hypoxia e.g. RA and cadiovascular disease also have significant macrophage infiltrates.

Thus macrophages may be used to deliver genes to therapeutically relevant areas and would in particular target areas of hypoxia. Consequently, hypoxia regulation of therapeutic genes within this cell mediated delivery system may improve targeting of therapy and therapeutic index.

It was therefore important to establish that macrophages can respond to hypoxia and have the regulatory systems to drive gene expression through HRE control since there is no data in the literature that primary human macrophages can respond to hypoxia via a HIF pathway. Consequently, we have carried out studies to assess whether macrophages possess HIF protein and if so are these transcription factors induced under hypoxic conditions. Furthermore can genes under HRE control respond to hypoxia in the macrophage context.

### Materials and Methods

### Preparation of Macrophage Lysates for Western Blotting

7 day old macrophages were plated on Primera 24 well plastic plates at a density of 3x10⁵ cells/well and allowed to adhere. The cells were then exposed for 16 hours to either 0.1 % oxygen, 5% CO₂ (hypoxia) or 21% oxygen, 5% CO₂ at 37°C. The plates were removed from the incubator and the cells immediately lysed in SDS buffer (2% SDS, 10% glycerol, 0.0625 M Tris-HCl pH 6.8, 5% β-mercaptoethanol) and heated at 99°C for 5 min. Lysates were stored at -70°C until a protein gel and blot could be performed (as described in Weisener *et al*., 1998). Blots were probed with antibodies to HIF-1 and a related factor, EPAS-1.

### Results

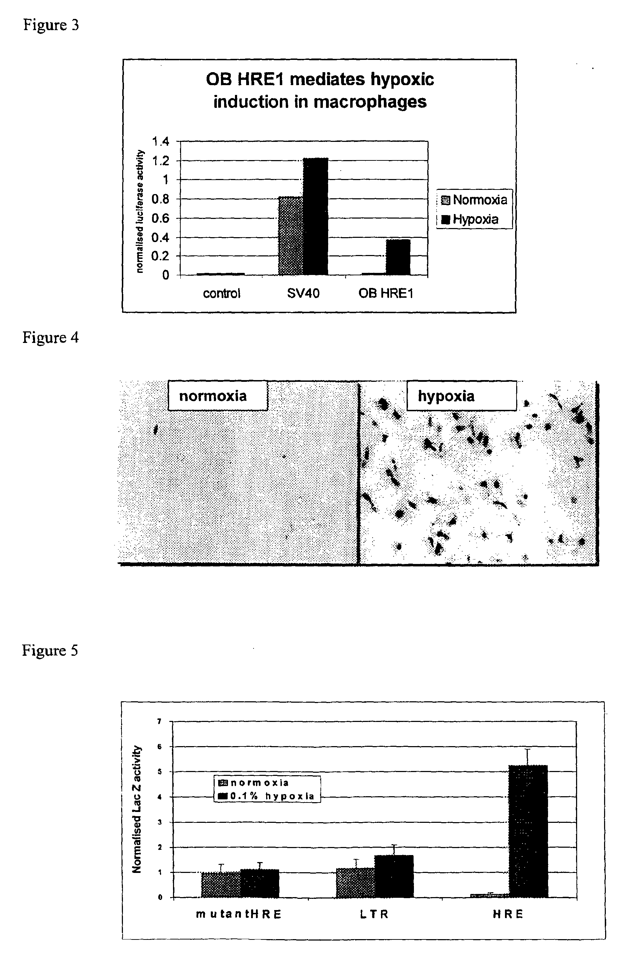

We now show that, surprisingly, in macrophages it is not possible to detect the HIF-1 protein, by Western blotting, even under hypoxia, and yet the OBHRE promoter (PGK) is activated in these cells when they are placed in hypoxic conditions (Figure 3). We also now show, by Western blotting, that macrophages do express a related factor of the bHLH/PAS family member termed EPAS-1 (endothelial PAS domain protein) or HRF (HIF-related factor) (Ema *et al*., 1997; Flamme *et al*., 1997; Tian *et al*., 1997). As predicted from the conservation of the DNA binding domain of the two proteins, EPAS-1 appears to bind to the same consensus sequence (defined as the HRE) as HIF-1. Published data have however indicated that EPAS expression is restricted to endothelial cells and this is the first time that EPAS has been found in non-epithelial cells.

Thus the induction of OBhre11 in macrophages is either principally or entirely mediated by EPAS-1 and as such represents an EPAS responsive enhancer. Consequently, OBhre11 may be used to drive expression in cell types that don't express HIF1 but do express EPAS.

### Example 3 - PGK derived enhancer sequences in the context of the MLV retroviral promoter

To characterise further the PGK HRE trimer in the context of other strong viral promoters, the PGK HRE trimer is configured upstream of the MLV retroviral core promoter. Apart from assessing the wider utility of this HRE in the background of other core promoters this was useful to predict the behaviour of the HRE when further configured into a retroviral vector system that is based on the MLV genome. Thus the PGK HRE trimer is placed in the context of an MLV retroviral promoter, forward (natural) orientation. This construct is identical to OBhre11 but with the HRE sequences placed upstream of the Moloney MLV retroviral promoter instead of SV40. Sequence shown up to transcription start. (SEQ ID. No. 4). MLV sequences shown in italics.

AGCTAGCCTAGCGTCGTGCAGGACGTGACATCTAGTGTCGTGCAGGACGTGAC
ATCTAGTGTCGTGCAGGACGTGACATCTAGAGAACCATCAGATGTTTCCAGGGT

GCCCCAAGGACCTGAAATGACCCTGTGCCTTATTTGAACTAACCAATCAGTTCG
CTTCTCGCTTCTGTTCGCGCGCTTCTGCTCCCCGAGCTCAATAAAAGAGCCCAC
AACCCCTCACTCGG

The PGK HRE trimer is also placed in the context of the above MLV retroviral promoter but in the reverse orientation. Sequence shown up to transcription start. (SEQ ID. No. 5).

AAGCTAGCTGTCACGTCCTGCACGACACTAGATGTCACGTCCTGCACGACACTA
GATGTCACGTCCTGCACGACTCTAGAGAACCATCAGATGTTTCCAGGGTGCCCC
AAGGACCTGAAATGACCCTGTGCCTTATTTGAACTAACCAATCAGTTCGCTTCTC
GCTTCTGTTCGCGCGCTTCTGCTCCCCGAGCTCAATAAAAGAGCCCACAACCCC
TCACTCGG

Both of these novel constructs are tested using the luciferase assay described in Example 1 and give induction levels of greater than 2 logs under hypoxia. Thus, these results indicate that the MoMLV promoter my be used instead of the SV40 promoter and confirm the results obtained with the SV40 promoter in Example 1 showing that the PGK HREs function well in either orientation with respect to the promoter element.

### Example 4 - Construction of Hypoxia Regulated Retroviral Vectors

To test the HRE/promoter constructs of the present invention in the context of a retroviral vector delivery system, we constructed MLV based retroviral vectors.

### HRE retroviral vector plasmid construction

Plasmid pLNSX (A D Miller, Fred Hutchinson Cancer Center: Miller and Rosman, 1989; Genbank Accession No. M28246) is cut in the 5' and 3' LTR's with Nhe1 and religated to remove the retroviral genome. The resulting plasmid, pLNhe, was cut with Nhe1 and Xba1 to remove a 267 bp fragment containing the retroviral enhancer from the 3' LTR and ligated to a 72 bp oligomer consisting of a trimer of the phosphoglycerate kinase (PGK) hypoxia response element to produce pLNheHRE. The plasmid pMOI (Kim *et al*., 1998) was cut with Nhe1 and the resulting retroviral genome ligated into the pLNheHRE to create plasmid pMOIHRE. A nuclear localising LacZ sequence was ligated into pMOIHRE as a Stu1-Xho1 fragment 3' to the IRES to create pHRE. The lacZ sequence was prepared by PCR, the PCR primers being designed such that the 5' end of the LacZ gene could be modified by the addition of the SV40 large T antigen nuclear localising signal, amino acid sequence MGTAPKKKRKV.

pMUTHRE, a variation of pHRE was constructed by inserting a 72 bp mutant PGK oligomer, which contained a point mutation in the HIF1 binding site (see below). As a positive control a similar plasmid, pLTR is constructed which contained an intact 3' LTR. A similar group of plasmids are also constructed with the luciferase reporter gene instead of LacZ.

| | |
|---|---|
| PGK | CTAGCTGTCACGTCCTGCACGACACTAGATGTCACGTC CTGCACGACACTAGATGTCACGTCCTGCACGACT |
| PGK (mut HRE) | CTAGCTGTCCATTCCTGCACGACACTAGATGTCCATTCCTGCAC GACACTAGATGTCCATTCCTGCACGACT |
| | Point mutation in HIF site in bold |

### Beta-galactosidase chemiluminescent reporter assays

These were carried out using the Tropix Galacton-Plus® reporter assay system with a Dynex Technologies (West Sussex) MLX Microtiter® plate luminometer. All reporter assay were carried out in triplicate.

### Protein Assays

These were carried out using the Biorad Detergent Compatible protein assay kit (BioRad, UK) together with a Dynex Technologies MRX Plate reader using a bovine serum albumin protein standard.

### Transient Recombinant Retroviral Production

Recombinant retroviral vectors were produced using the three-plasmid expression system previously described *(Soneoka et al*., 1995) with a few modifications: 16 µg of the genome plasmids described above were co-transfected with 16 µg of the gag-pol plasmid pHIT60 *(Soneoka et al*., 1995) and 8 µg of the VSV-G/env plasmid (Kim *et al*., 1998) onto a 10 cm dish of 70% confluent 293T cells. After approximately 6 hours the medium was removed. The viral supernatant was harvested 36 hours later and filtered through a 0.45µm filter.

Retroviral titre was determined using D17 cells. The cells were plated onto a twelve-well culture dish at a density of 6 x 10⁴ the day before transduction. Supernatant (1 ml at appropriate dilutions of the original stock) containing 8 µg/ml polybrene (Sigma) was added to each well. After 24 hours incubation in either normoxic or hypoxic conditions, as previously described, the viral titre was determined by X-gal staining and counting the number of beta-galactosidase positive cells.

### Results

The results obtained with these hypoxia regulated MLV based retroviruses demonstrate that a pool of stable transductants show a surprisingly high degree of regulation and show more than a two log increase in viral titre and a 40 fold induction of reporter gene activity under hypoxia (see Figures 4 and 5).

### Example 5 - Duplication and generation of flanking HREs enhances hypoxia response

It is known that sequences inserted into regions of the 3' LTR of retroviral vectors are duplicated when the retrovirus integrates into the host genome. To determine whether the surprising results obtained in Example 5 with MLV retroviral constructs were as a consequence of a duplication of the HRE sequences inserted into the 3' LTR, we have produced a series of retroviral vector plasmids to mimic the proviral genome. The HRE in the 5' and 3' position are sequentially mutated and analysed in a transient reporter system for use in transient transfection assays

A series of vectors are constructed to analyse the activity of the HRE linked to the MoMLV promoter in a transient assay. The HRE and MoMLV promoter are removed from pLNheHRE as an Nhe1-Sma1 fragment and inserted into the MCS of pGL3 basic (Promega) to produce pGLHRE and pGLMUT. pGL3 promoter and pGL3 control are used as negative and positive controls respectively.

The vectors p5'HRE3'MUT, p5'MUT3'HRE, p5'MUT3'MUT and p5'HRE3'HRE are constructed by digesting pGLHRE and pGLMUT with Sac1 and ligating the resulting retroviral genome with linearised pLNheHRE cut with the same enzyme.

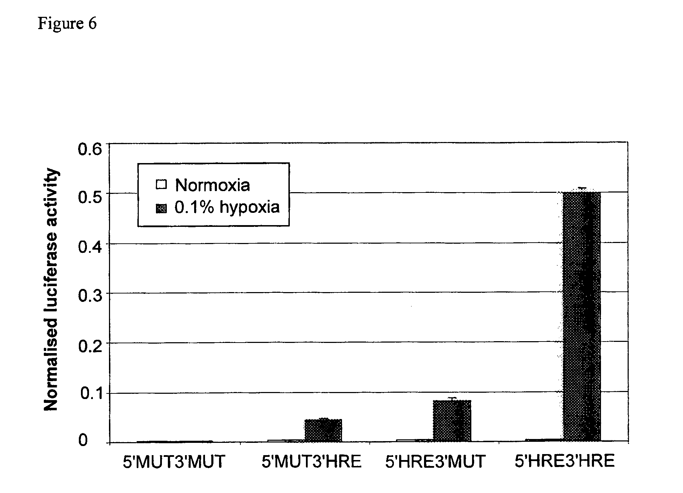

The data shown (Figure 6) clearly demonstrate that both the 5' and 3' HRE are involved in transcriptional regulation and that the surprising results obtained in Example 5 are due to a synergistic effect between the 5' and 3' HRE sequences. Thus, we have now demonstrated in an MLV retroviral vector system that duplication of the HRE sequences enhances the transcriptional response to hypoxia in a synergistic manner. The strategy of configuring the HRE into the 3' LTR results in duplication of the enhancer during reverse transcription in the transduced cell such that the proviral genome has identical flanking HREs. It is therefore likely that in the retroviral system, it is this duplication that underlies the strength of the transcriptional response to hypoxia. Consequently, retroviruses offer key advantages in the delivery of hypoxia regulated genes.

The biology of retroviruses provides strategies for optimising the control of other delivery systems. For example if non viral gene delivery is required plasmid vectors can be configured with flanking enhancers to mimic the proviral genome structure of a retrovirus.

It is important to note that these data can be extrapolated to other non HRE enhancer systems where a similar synergy can be envisaged.

### Example 6 - Construction of hypoxia regulated retroviral vectors containing NOIs.

RRVs may be constructed using a packaging cell line system such as FLYRD 18 (Cosset *et al*., 1995). A plasmid vector containing the vector genome to be packaged is transfected into the packaging cell line as described (Cosset *et al*., 1995) to derive the producer cell line. A suitable plasmid containing vector genome is pHIT111 (Soneoka *et at*., 1995). The required NOI is inserted in place of the LacZ gene in pHIT111 using standard molecular biology techniques. The HRE regulatory elements may similarly be introduced into the retroviral LTR in pHIT111 in place of the retroviral enhancer to ensure hypoxia regulated expression of the NOI. The plasmid is then co-transfected with a selectable marker NOI appropriate for FLYRD18 cells (e.g. pSV2neo; Genbank Accession No. U02434) and transfected cells are selected in 1 mg/ml G418 (Sigma).

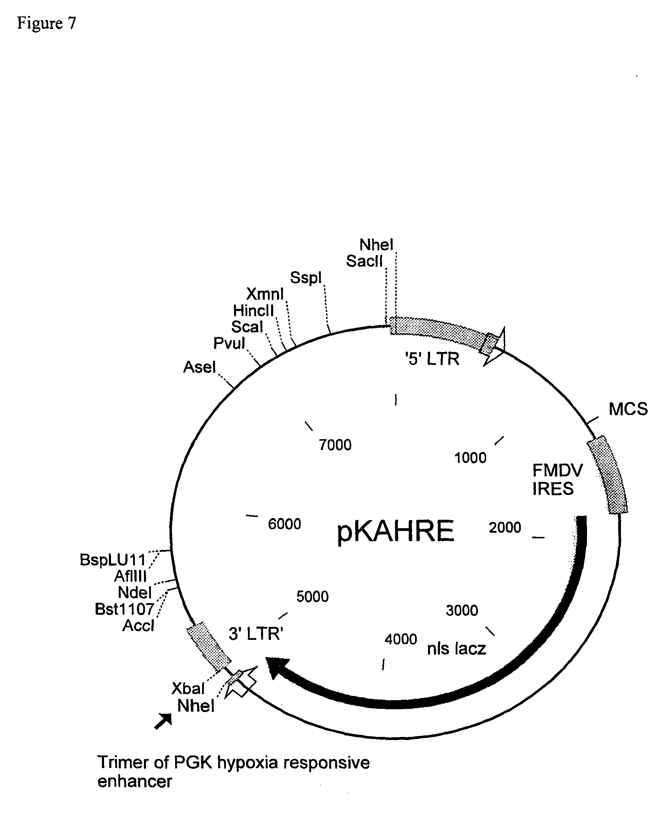

A suitable HRE-containing enhancer consists of three copies of the HRE from the PGK gene (Firth *et al*., 1994). An example, WT PGK18+++, (SEQ ID. No. 6) is shown below. Also shown below is MUT PGK18+++, a mutant HRE-containing sequence to be used as a control which is not regulated by hypoxia. The synthetic oligonucleotides shown below are inserted between the Nhe1 and Xba1 sites in the 3' LTR of pHIT111 to generate a retroviral vector in which gene expression in the target cell is under hypoxia control. An alternative retroviral vector, constructed in the same way, is pKAHRE shown in Figure 7.

WT PGK18+++ (SEQ ID. No. 6)

5′ CTA GCT GTC ACG TCC TGC ACG ACA CTA GAT GTC ACG TCC TGC
(Nhe1) GA CAG TGC AGG ACG TGC TGT GAT CTA CAG TGC AGG ACG

ACG ACA CTA GAT GTC ACG TCC TGC ACG ACT
TGC TGT GAT CTA CAG TGC AGG ACG TGC TGA GATC 3′ (Xba1)

MT PGK18+++

5′ CTA GCT GTC CAT TCC TGC ACG ACA CTA GAT GTC CAT TCC TGC
(Nhe1) GA CAG GTA AGG ACG TGC TGT GAT CTA CAG GTA AGG ACG

ACG ACA CTA GAT GTC CAT TCC TGC ACG ACT
TGC TGT GAT CTA CAG GTA AGG ACG TGC TGA GATC 3′ (Xba1)

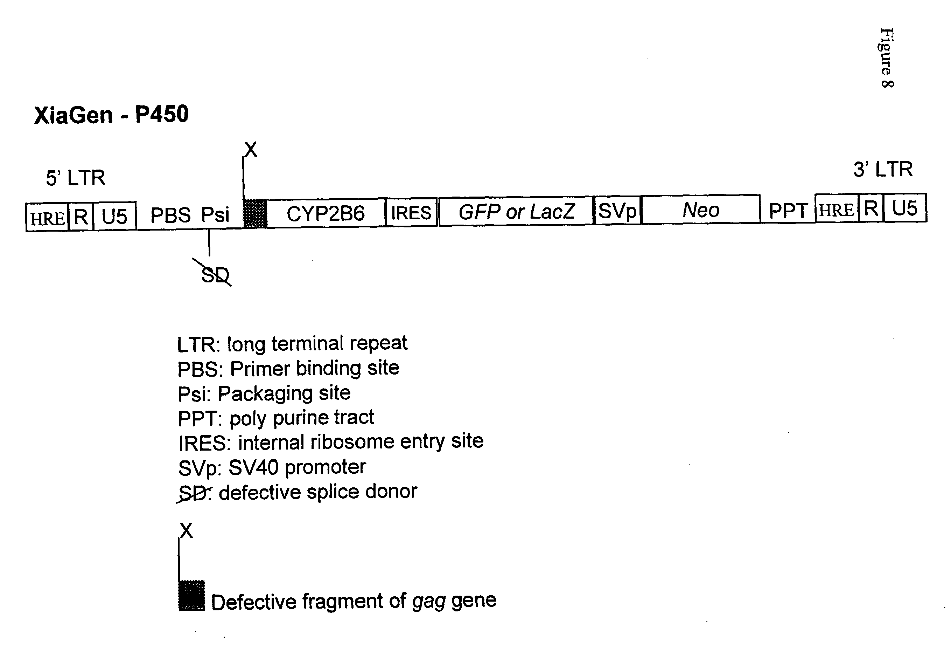

Another configuration constructed according to the principles outlined above is XiaGen-P450 (see Figure 8). This vector contains a therapeutic gene for human cytochrome P450 (CYP2B6) which activates the anti-cancer compound cyclophosphamide. Any other therapeutic gene as recited above could also be used. XiaGen-P450 also contains a reporter gene that encodes green fluorescent protein and a selectable gene that encodes neomycin resistance.

The CYP2B6 gene is obtained by PCR amplification from human hepatocyte derived mRNA as follows.

First strand cDNA containing the coding region of P450 CYP2B6 was obtained by reverse transcription from commercial liver total RNA (Clontech) using an oligo-dT primer. The corresponding double-stranded cDNA fragment was amplified by PCR using the primers P450F (sequence: CAG ACC ATG GAA CTC AGC GT) and P450R (sequence: GGA CAC TGA ATG ACC CTG GA). Due to the low abundance of CYP2B6 mRNA in the liver RNA preparation, a second round of PCR was performed on the first PCR product using oligonucleotide primers P450FOR (sequence: TCA TGC TAG CGG ATC CAC CAT GGA ACT CAG CGT C) and P450REV (sequence: AAA ATC ACA CTC TAG ATT CCC TCA GCC CCT TCA GC) for the plus and minus strands respectively.

The cDNA fragment generated from the PCR amplification was cloned into the pCRII-TOPO vector using the TOPO TA Cloning Kit from Invitrogen. This was then cloned upstream of the IRES sequence in pHRE as outlined in Example 4. The correct gene sequence is confirmed by comparison to the established sequence (Yamano *et al*., 1989 GenBank Accession No. M29874).

### Example 7 - Construction of tissue-restricted hypoxia responsive promoters

To further regulate the expression of NOIs using the hypoxia regulated expression system of the present invention, tissue-specific regulatory sequences may be operably linked to the HRE/promoter sequences. We have therefore constructed and tested constructs that confine expression of an NOI to cells that have differentiated into the granulocyte-monocyte lineage, in addition to hypoxia-regulated expression. Using promoters that are specific to, or upregulated in, the monocyte/macrophage lineage will restrict therapeutic gene expression to these cell types. Suitable promoters for this purpose include those with consensus sequences that bind myeloid specific transcription factors such as PU1 or transcription factors that are up-regulated during differentiation into macrophages such as CEBP-β (NF IL 6) (See Clarke and Gordon 1998).

Two constructs are used. The first is based on cellular macrophage regulatory sequences. The second is based on HIV regulatory sequences.

### a) Cellular promoter

An oligonucleotide is synthesised in two parts based on tissue specific control elements residing in the M CSF receptor promoter (Zhang *et al*., 1994) flanked with a *Hind*III/*Nco*1 site for cloning into pGL3 basic vector (Promega) and subsequent analysis in luciferase reporter assays. Sites of transcription factor binding are indicated.

AGCTTCCTGCCCCAGACTGCGACCCCTCCCTCTTGGGTTCAAGGCTTTGTTTTCTT
SP1
CTTAAAGACCCAAGATTTCCAAACTCTGTGGTTGCCTTGCCTAGCTAAAGGGGAA
CEBP? PEBP2/CBF PU. 1
GAAGAGGATCAGCCCAAGGAGGAC

### b) HIV promoter

The transcription control sequences of HIV reside in the LTR, this generally consists of two NFkβ sites and three SP1 sites upstream of the ATATAA box (Koong *et al*., 1994).

An Entrez database nucleotide search is performed for HIV LTR sequences and these are analysed for the integrity of the motifs outlined above. Submission U63538 (Zacharova *et al*., 1997) describes an isolate that shows the well conserved motifs of two NFkβ sites and three SP1 sites. However we observed that this particular isolate also had the consensus sequence for binding of NF-IL6 (C/EBPβ). As discussed above this transcription factor is known to be active in macrophages.

Oligonucleotides are synthesised in two parts to generate the sequence from Genbank Accession No U63538 positions 279 - 447. These are synthesised with Nhe1/Smal ends to facilitate cloning. The annealed and ligated oligonucleotide is cloned into OB37. The final construction is shown below (SEQ ID. No. 7).

### XiaMac sequence

GCTAGAGTCGTGCAGGACGTGACATCTAGTGTCGTGCAGGACGTGACA
HRE HRE
TCTAGTGTCGTGCAGGACGTGACAGCTAGCATTCCATCACGTGGCCCG
HRE NF-IL6
AGAGAAGCATCCGGAGTACTACAAGGACTGCTGACAGCGAGATTTCTA

CAAGGGACTTTCCGCTGGGGACTTTCCAGGGAGGTGTGGCCTGGGCG
NFkβ NFkβ Sp1 Sp1
GGACTGGGGAGTGGCGAGCCCTCAGATGCTGCATATAAGCAGCAGCTGCTT
Sp1
TTGCCCC

We have therefore constructed a novel synthetic promoter (XiaMac) that combines the HRE mediated response to hypoxia with tissue specificity (macrophage).

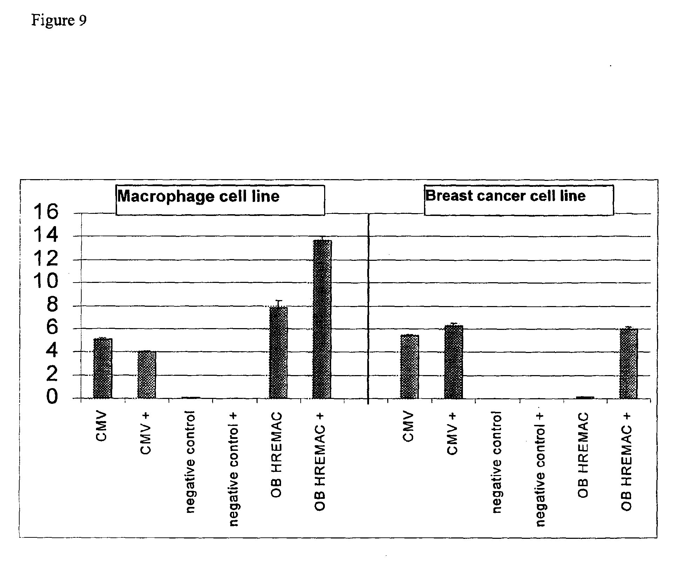

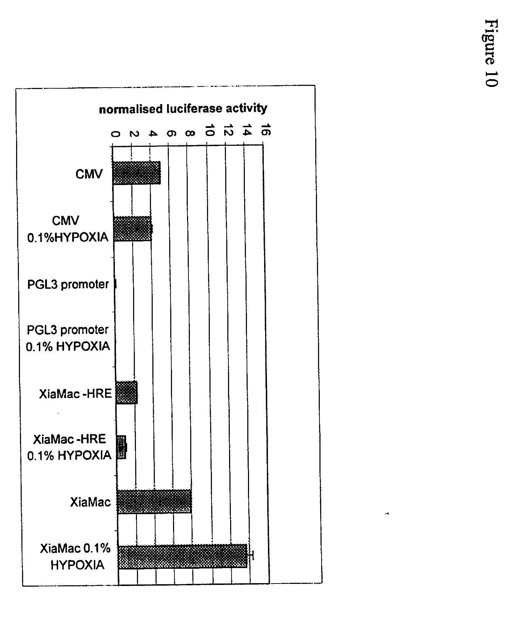

As shown in Figure 9 when the XiaMac promoter is tested in non-macrophage cells such as breast cancer cells there is no activity at all. However if the cells are subjected to hypoxia then activation is observed (negative control is pGL3 promoter only, CMV control is CMV promoter without HREs). In contrast the promoter is active in macrophage cell line and the activity is boosted by hypoxia. The XiaMac promoter response to hypoxia is abolished if the HRE is inactivated (Figure 10).

### Example 8 - Construction of a macrophage-specific promoter restricted by repression.

Additional sequences may also be added to the HRE enhancer/promoter sequences of the present invention to further restrict the expression to particular cells, for example macrophages, under defined conditions. An example of this approach would be the IRF system (Taniguchi *et al*., 1995; Kuhl *et al*., 1987). In this situation an interferon response element (IRE) is used that can bind the transcription factors IRF1 and IRF2. IRF2 is constitutively bound to this IRE in macrophages and lacks the ability to activate transcription. Interferon activates IRF 1 expression that can then compete for binding with IRF2. The ability of IRF1 to activate transcription thereby reverses IRF2 repression. IRF1 also induces IRF2 gene expression thereby limiting activation of transcription by 'auto shut off. Inclusion of a tetramer of the IRE can block SV40 promoter function in the absence of IRF1 activation. We have now shown that inclusion of this tetrameric sequence downstream of the HRE 5' to the ATATAA (i.e. the TATA box-like element in the SV40 promoter) confers repression in the absence of activation by interferon.

The IRE is cloned into the XiaMac promoter as follows:-
Oligonucleotides are designed consisting of a tetramer of the IRE site as follows with BanII sticky ends: C(AAGTGA)₄GAGCC.

Within the XiaMac promoter there is a BanII site which is 3' to the last SP 1 site and 15 bp upstream of the TATAA. Insertion of the IRE elements at this site produces a repressed promoter (XiaMac-IRE (SEQ ID. No. 8)) that can only become active in the presence of interferon and hypoxia.

### XiaMac-IRE sequence

GCTAGAGTCGTGCAGGACGTGACATCTAGTGTCGTGCAGGACGTGACA
HRE HRE
TCTAGTGTCGTGCAGGACGTGACAGCTAGCATTCCATCACGTGGCCCG
HRE NF-IL6
AGAGAAGCATCCGGAGTACTACAAGGACTGCTGACAGCGAGATTTCTA
CAAGGGACTTTCCGCTGGGGACTTTCCAGGGAGGTGTGGCCTGGGCG
NFkβ NFkβ Sp1 Sp1
GGACTGGGGAGTGGC AAGTGAAAGTGAAAGTGAAAGTGA
Sp1 IRE IRE IRE IRE
GAGCCCTCAGATGCTGCATATAAGCAGCAGCTGCTTTTGCCCC

### Example 9 - Construction of hypoxia regulated lentiviral vectors

Lentiviral vectors offer significant advantages over conventional MLV based retroviral vectors because they can transfer genes to non-dividing and slowly dividing cells.

The data show above in Examples 5 and 6 indicate that the optimum configuration of a hypoxia regulated vector is one where there is a duplication of the HRE in the 5' and 3' LTRs. This has led us to design regulated single transcription unit lentiviral vectors.

### Materials and Methods

### Plasmid construction

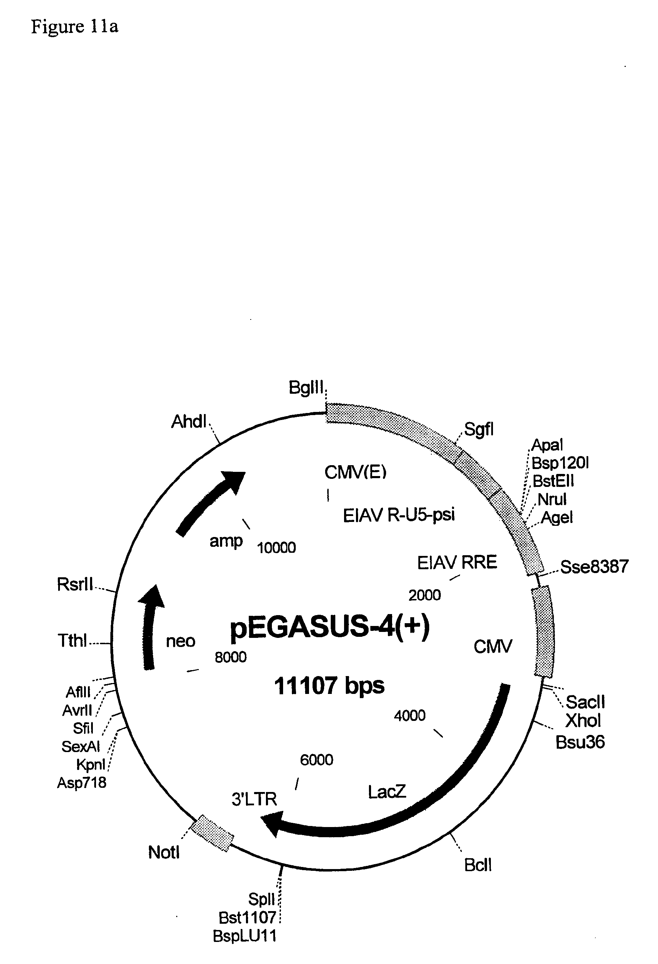

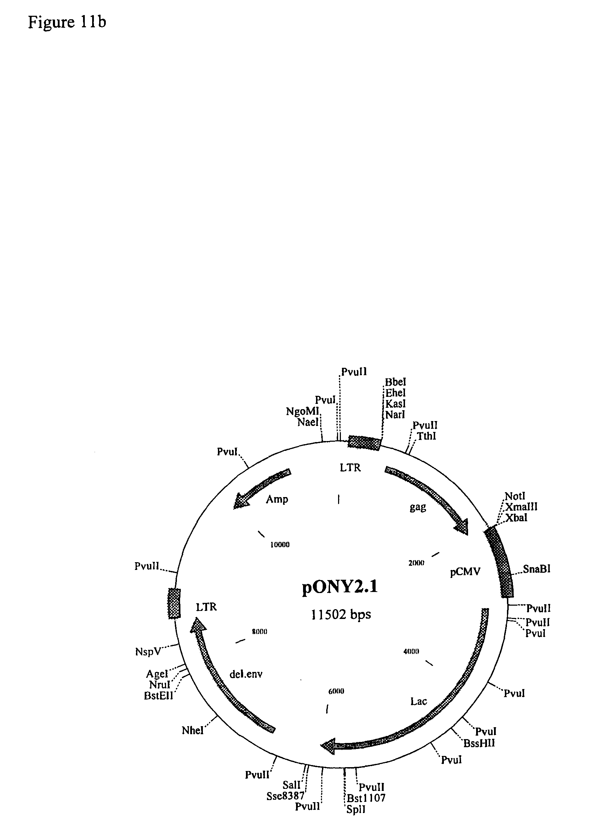

The hypoxia responsive promoter has been configured into a lentiviral vector, pEGASUS-1 (also pEGASUS-4(+) - Figure 11a) and the related vector pONY2.1 (Figure 11b). Both are derived from infectious proviral EIAV clone pSPEIAV19 (Payne *et al*., 1998; Accession No. U01866). The construction of these plasmids is described below (taken from U.K. patent application no. 9727135.7).

### pONY2.1

Plasmid pONY1 was constructed by inserting the EIAV 5' LTR into pBluescript II KS⁺ (Stratagene). The EIAV 5' LTR was amplified by PCR from pSPEIAV19 using pfu polymerase and the following primers - 5' GCATGGACCTGTGGGGTTTTTATGA GG and 3' GCATGAGCTCTGTAGGATCTCGAACAGAC. The amplification product was blunt-ended by 5' overhang fill-in and inserted into pBluescript II KS⁺ cut with BssHII which had been blunt ended by 3' overhang removal using T4 DNA polymerase This construct was called pONY1 and the orientation was 5' to 3' in relation to β-galactosidase of pBluescript II KS⁺. Sequencing of pONY1 revealed no mutations.

The *env* region of pSPEIAV19 was deleted by removal of the *Hind* III/*Hind* III fragment to generate pSPEIAV19ΔH. The MluI/MluI (216/8124) fragment of pSPEIAV19ΔH was then inserted into pONY1 cut with MluI to generate a wild type proviral clone (pONY2ΔH) in pBluescript II KS⁺. A BssHII digest (619/792) of pBluescript II KS⁺ was carried out to obtain the multiple cloning site. This was blunt ended by 5' overhang fill-in and ligated to pONY2ΔH cut with BglII and NcoI (1901/4949 - within *pol*) and blunt-ended by 5' overhang fill-in. The orientation was 3' to 5' in relation to the EIAV sequence. This was called pONY2.1.

pSPCMV was created by inserting pLNCX (Genbank Accession number: M28246) (PstI/HindIII) into pSP72 (Promega; Genbank Accession No. X65332). The β-galactosidase gene was inserted from pTIN414 (Cannon *et al*., 1996) into pSPCMV (XhoI/SphI) to make pSPlacZ. The 5' end to the β-galactosidase gene was replaced by the SV40 T-antigen nuclear localization signal from pAD.RSVbgal (Stratford-Perricaudet *et* al., 1992) to make pSPnlslacZ (XhoI/ClaI). The CMV nuclear localizing and non-nuclear localizing β-galactosidase from pSPlacZ and pSPnlslacZ was cut out with PstI and inserted into the PstI site of pONY2.1 in the 5' to 3' orientation of EIAV. These were called pONY2.1nlslacZ and pONY2.11acZ (Figure 11b).

### pEGASUS-1

This is an EIAV-based vector that contains only 759 nt of EIAV sequences (268 nt-675 nt and 7942 nt-8292 nt). Sequences encompassing the EIAV polypurine tract (PPT) and the 3'LTR were obtained by PCR amplification from pONY2.1 using primers PPTEIAV+ (Y8198): GACTACGACTAGTGTATGTTTAGAAAAACAAGG, and 3'NEGSpeI (Y8199): CTAGGCTACTAGTACTGTAGGATCTCGAACAG. The product was purified, digested with SpeI and ligated into pBS II KS⁺ which had been prepared by digestion with SpeI and treatment with alkaline phosphatase. Colonies obtained following transformation into *E. coli*, XL-1Blue were screened for the presence of the 3'LTR in the orientation in which the U5 region of the 3'LTR was proximal to the NotI site of the pBS II KS⁺ linker. The sequence of the cloned insert was determined and showed that it contained only one change from the EIAV clone pSPEIAV19. This was a 'C' insertion between bases 3 and 4 of the R region. The same change was found in the template used in the PCR reaction. The clone was termed pBS.3'LTR.

Next a reporter gene cassette, CMV promoter/LacZ, was introduced into the PstI site of pBS.3'LTR. The CMV/LacZ cassette was obtained as a PstI fragment from pONY2.1. The ligation reaction to join the above fragments was transformed into *E. coli*, XL-1Blue. A number of clones in which the CMV/LacZ insert was orientated so that the LacZ gene was proximal to the 3'LTR were assessed for activity of the CMV/LacZ cassette by transfection into the cell line 293T using standard procedures. A clone which gave blue cells at 48 hours post-transfection following development with X-gal was selected for further use and termed pBS CMVLacZ.3'LTR.

The 5' region of the EIAV vector was constructed in the expression vector pCI-ENeo which is a derivative of pCI-Neo (Promega; Genbank Accession No. U47120) modified by the inclusion of approximately 400 base pairs derived from the 5' end of the full CMV promoter as defined previously. This 400 base pair fragment was obtained by PCR amplifcation using primers VSAT1 (GGGCTATATGAGATCTTGAATAATAAAATG TGT) and VSAT2 (TATTAATAAC TAGT) and pHIT60 *(Soneoka et al*., 1995) as template. The product was digested with BglII and SpeI and ligated into pCI-Neo which had been digested similarly.

A fragment of the EIAV genome running from the R region to nt 150 of the gag coding region (nt 268 to 675) was amplified with primers CMV5'EIAV2 (GCTAC GCA**GAGCTC**GTTTAGTGAACCGGGCACTCAGATTCTG: [sequences underlined anneal to the EIAV R region]) and 3'PSI.NEG (GCTGAGCTCTAGAGTCCTTTTCTTT TACAAAGTTGG) using pSPEIAV19 as template DNA. The 5' region of the primer CMV5'EIAV2 contains the sequences immediately upstream of the CMV promoter transcriptional start site and can be cut with SacI (bold). 3'PSI.NEG binds 3' of the EIAV packaging sequences as defined by deletion analysis and contains an XbaI site.

The PCR product was trimmed with SacI and XbaI and ligated into pCI-Eneo which had been prepared for ligation by digestion with the same enzymes. This manipulation places the start of the EIAV R region at the transcriptional start point of the CMV promoter and transcripts produced thus start at the genuine start position used by EIAV and extend to the 3'-side of the packaging signal. Clones which appeared to be correct as assessed by restriction analysis were sequenced. A clone termed pCI-Eneo.5'EIAV was selected for further work.

In the next step the CMVLacZ and 3'LTR cassette in pBS.CMVLacZ.3'LTR was introduced into pCI-Eneo.5'EIAV. pBS.CMVLacZ.3'LTR was digested with ApaI, the 3'overhangs removed with T4 DNA polymerase, then digested with NotI. The fragment containing the CMVLacZ.3'LTR was purified by standard molecular biology techniques. The vector for ligation with this fragment was prepared from pCIEneo.5'EIAV by digestion with SalI, followed by filling-in of the 5'overhangs using T4 DNA polymerase. The DNA was then digested with NotI and purified prior to use in ligation reactions. Following transformation into *E*.*coli*, XL-1Blue colonies were screened for the presence of the insert by restriction analysis to identify the required clone, designated pEGASUS-1 (see Figure 11a which actually shows pEGASUS-4(+), a version of pEGASUS-1 containing an EIAV RRE immediately downstream of the EIAV R-U5-psi region - see below for details).

### pEGASUS-4(+)

Further improvements to the EIAV vector pEGASUS-1 may be made by introduction of additional elements to improve titre. A convenient site for the introduction of such elements is the SalI site which lies between the XbaI to the 3' of the packaging signal and upstream of the CMV/LacZ cassette of pEGASUS-1. For example the RRE from EIAV can be inserted at this site.

The EIAV RRE as defined previously was obtained by PCR amplification as follows. Using pONY2.10LacZ as template two amplifications were performed to obtain the two parts of the EIAV RRE. The 5'-element was obtained using primers ERRE1 (TTCTGTCGACGAATCCCAGGGGGAATCTCAAC) and ERRE2 (GTCACCTTCCAG AGGGCCCTGGCTAAGCATAACAG) and the 3'element with ERRE3 (CTGTTATG CTTAGCCAGGGCCCTCTGGAAGGTGAC) and ERRE4 (AATTGCTGACCCCCAAA ATAGCCATAAG). These products will anneal to each other hence can be used in second PCR reaction to obtain a DNA which 'encodes' the EIAV RRE. The second PCR amplification is set up without primers ERRE1 and ERRE4 for the first 10 cycles and then these primers are added to the reaction and a further 10 cycles of amplification carried out. The resulting PCR product and pEGASUS-1 were digested with SalI, ligated and transformed into E.coli XL-1Blue. Clones in which the EIAV RRE was in either the positive or negative orientations were selected for further work. These vectors plasmids were called pEGASUS-4(+) (see Figure 11a) and pEGASUS-4(-).

### pONY HRE luc/lac

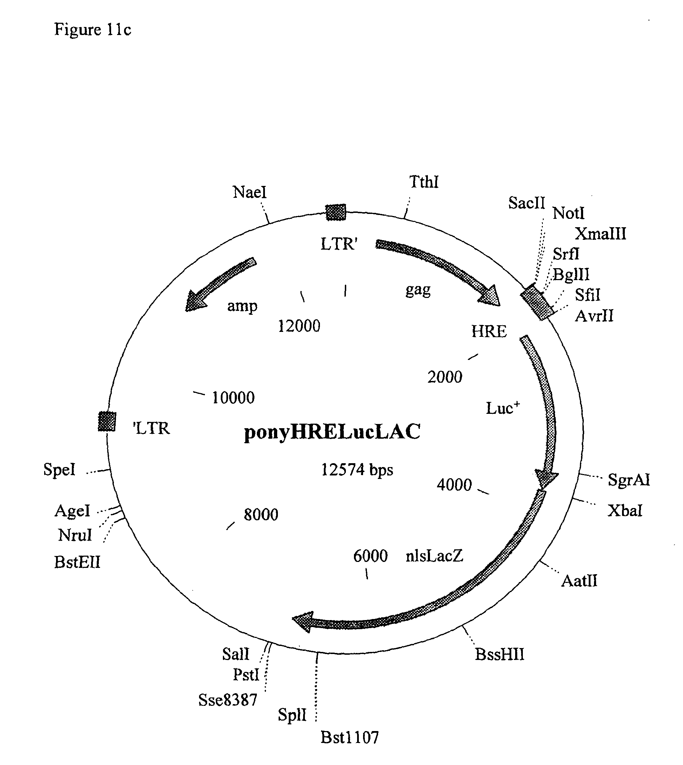

The CMV promoter in pONY2.1 is excised as an XbaI/AscI fragment and replaced with an oligonucleotide containing a MluI/XbaI site. This consequently allows insertion of the MluI/XbaI fragment isolated from OB37 (Figure 1) creating pONY HRE luc/lac (Figure 11c). Luciferase coding sequence is removed as an Nco1 fragment and the backbone religated creating pONY HRElac. Similarly, lacZ is removed as XbaI/SalI then the backbone religated to create pONY HREluc.

### pEGHRELacZ/luc

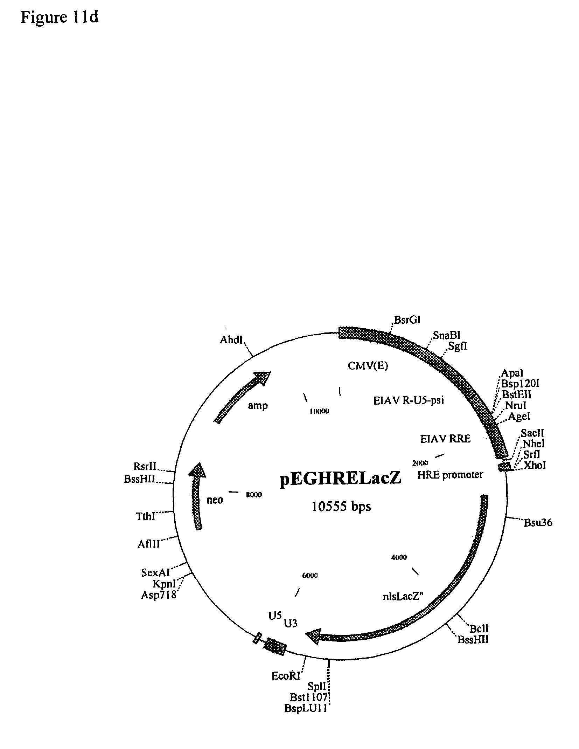

The CMV promoter lacZ cassette is excised from the EIAV vector plasmid pEGASUS-1 with EcoRl and replaced with a synthetic oligonucleotide containing a SacI and a Bsu36 site. This allows the cloning of the HRE luc/HRE lac cassette from pONY HRE luc and pONY HRE lac as SacI/Bsu36 and SacI/EcoR1 fragments respectively. The final vectors are designated pEG-HRE-lacZ (shown in Figure 11d) and pEG-HRE-luc.

Using the same approach pEGHRE vectors may be constructed to express therapeutic genes, such as any of the therapeutic genes listed above, in place of the lacZ or Luc genes. These may be cloned into pEGHRE vectors by excision of the lacZ/luc fragments from pEG-HRE-lacZ or pEG-HRE-luc with SacI/Bsu36 and SacI/EcoR1, respectively, and ligation of a suitable fragment containing a coding sequence. An example of a therapeutic gene which may be used is the gene encoding anti-angiogenic factor thrombospondin-1 (Genbank Accession No. X04665).

### Luciferase and beta-galactosidase assays using cells producing viral particles from pEGHRE plasmids

Viral particles with the pEGHRE genome are produced in a transient three plasmid system *(Soneoka et al*., 1995). 48 hours post-transfection tissue culture fluid is collected and filtered through 0.45 µm filters. Ten-fold dilutions are made in culture medium containing polybrene at 8 mg/ml and then 500 µl aliquots titred on D17 canine osteosarcoma on parallel plates seeded at 0.8 x 10⁵/well in 12 well plates on the previous day. The transduced cell population is split and incubated overnight in normoxia (21% oxygen) or hypoxia (0.1% oxygen).

Cells transduced with lacZ-expressing viruses are stained by X-gal histochemistry (MacGregor *et al*., 1991) and end point titres calculated. Titre is a measure of β-galactosidase gene expression and reflects changes in gene expression between cell populations under conditions of normoxia and hypoxia. Cells transduced with luc-expressing viruses are tested for luciferase activity.

### Results

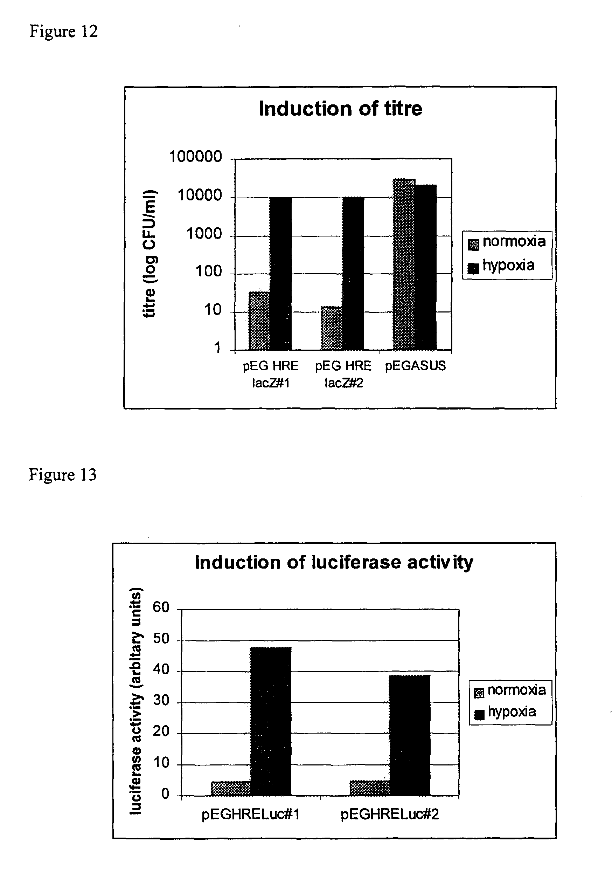

In the experiment shown the titre obtained from the parent 'pEGASUS-1' vector (with a CMV lacZ transcriptional unit) does not change significantly under hypoxia, whereas the regulated vector, pEG-HRE-lacZ, titre is induced by at least 100 fold (Figure 12). Luciferase activity of cells transduced with pEG-HRE-luc increases 12 fold under hypoxic conditions (Figure 13). These data indicate for the first time that it is possible to obtain highly efficient hypoxia regulation in the context of a lentiviral vector.

### Example 10 - Construction of hypoxia regulated lentiviral single transcription unit vectors

An alternative EIAV vector for use in this invention is where the therapeutic gene and a marker gene are expressed from a single transcription unit. Single transcription unit vectors are generally preferred over SIN vectors and vectors containing internal transcription unit because of advantages in vector production. These advantages are that the SIN vector must be introduced into a producer cell by transfection rather than transduction and this often reduces the number of high efficiency producer clones that can be obtained. It is also observed that vectors that contain internal transcription units generally give lower yields than single transcription unit vectors. Furthermore, as we have described above, the duplication of the HRE that can be achieved in a single transcription unit vector optimises hypoxia regulation. A single transcription unit lentiviral vector regulated by hypoxia vector is described below:-

### Vector Construction

The IRES is generated by PCR from pIRES-1hyg (Clontech) to incorporate flanking restriction sites to enable subsequent cloning;
ST1 (lead) - ATCGCTCGAGCTGCAGGGCCGCACTAGAGGAATTCGC
ST2 (complement) - GGTTGTGGCCCATGGTATCATCGTGTTTTTCAAAGG

This results in a XhoI IRES NcoI cassette. This cassette is then cloned into pSPlacZ (see Example 9) upstream of lacZ such that the NcoI site coincides with the ATG initiator of lacZ (pSPIRESlacZ). The IRES lacZ cassette is isolated as a PstI/Bst1107 fragment and used to replace the CMVlacZ cassette of pPEGASUS4(+) (excised as sse8387/Bst1107 fragment). This plasmid is designated pEG4+ IRES Z.

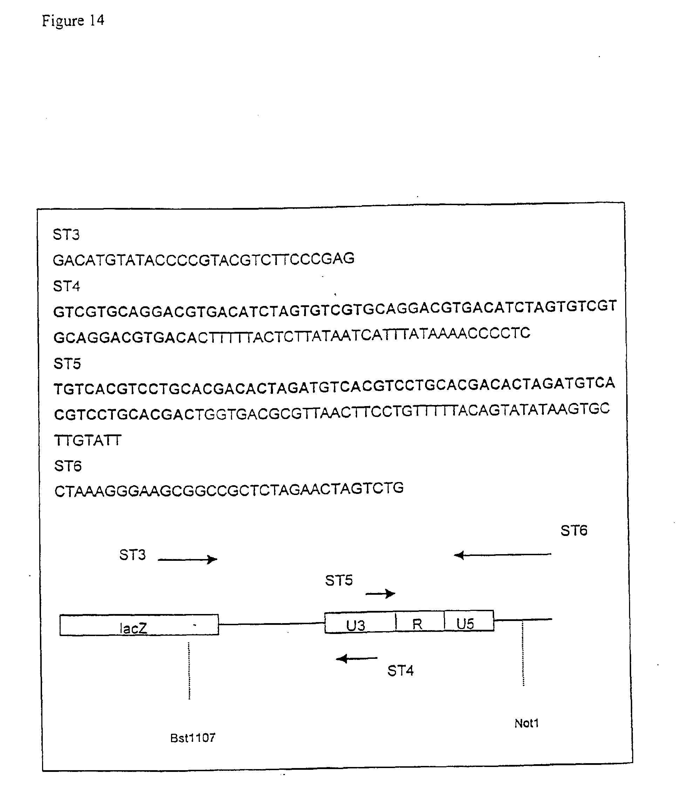

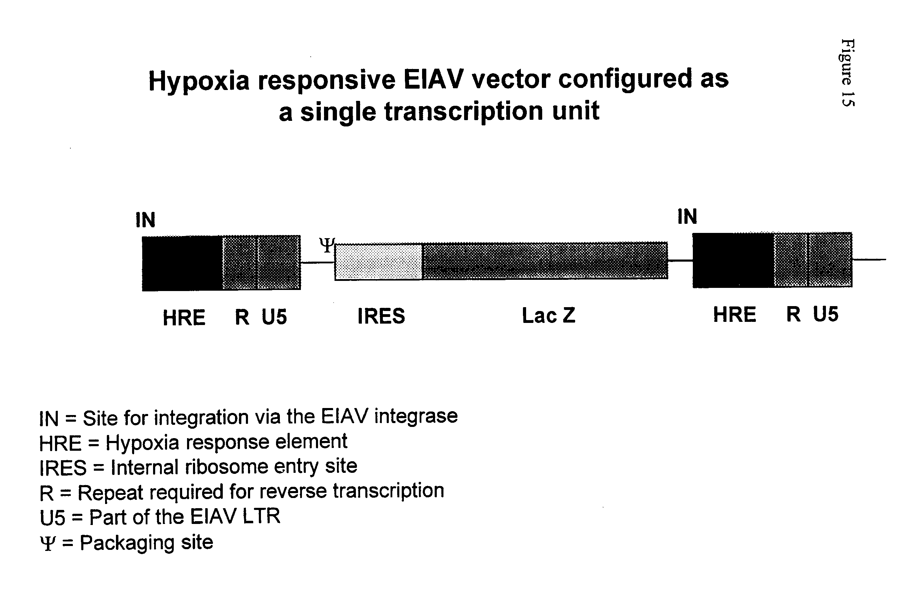

An overlapping PCR approach is used to replace part of the EIAV U3 enhancer region in the 3' LTR with an HRE (see Figure 14). A similar vector can be constructed where the U3 region is largely derived from any retrovirus or lentivirus or and other hybrid promoter such as those described above. The only limitations are that the terminal nucleotides of the parental vector are retained to ensure compatability with the lentiviral integrase and that the two R regions are homologous to allow efficient reverse transcription. A typical configuration for such a hybrid LTR is described in detail in WO-A-96/33623 and WO-A-98/17816. The final configuration of this vector is shown in Figure 15.

### Example 11 - Construction of an autoregulated hypoxia responsive lentiviral vector

It has previously been shown that the over-expression of HIF-1α can increase the expression from a number of hypoxia regulated promoters. This protein is known to be unstable in normal cells but this can be bypassed by over-expression. Such over expression would therefore compromise the specificity of the hypoxia response. The discovery that the OBHRE promoter is responsive to E-PAS opens up a new route to amplifying the response to hypoxia. To avoid the problem of constitutive expression of HIF-1 we have configured the E-PAS gene into a lentiviral vector that is itself responsive to hypoxia. In this way the vector is activated by hypoxia to express the E-PAS gene and the E-PAS protein then acts further on the HRE to augment expression. Given that HIF-1 is known to be labile once oxygen levels return to normal this vector system has the advantage of providing a longer term response to hypoxia. The initial priming of the vector is specific by virtue of the HRE interacting with HIF-1 or E-PAS. The production of E-PAS maintains the expression after the initial hypoxia stimulus is finished. Ultimately the response will decay according to the half life of the E-PAS protein.

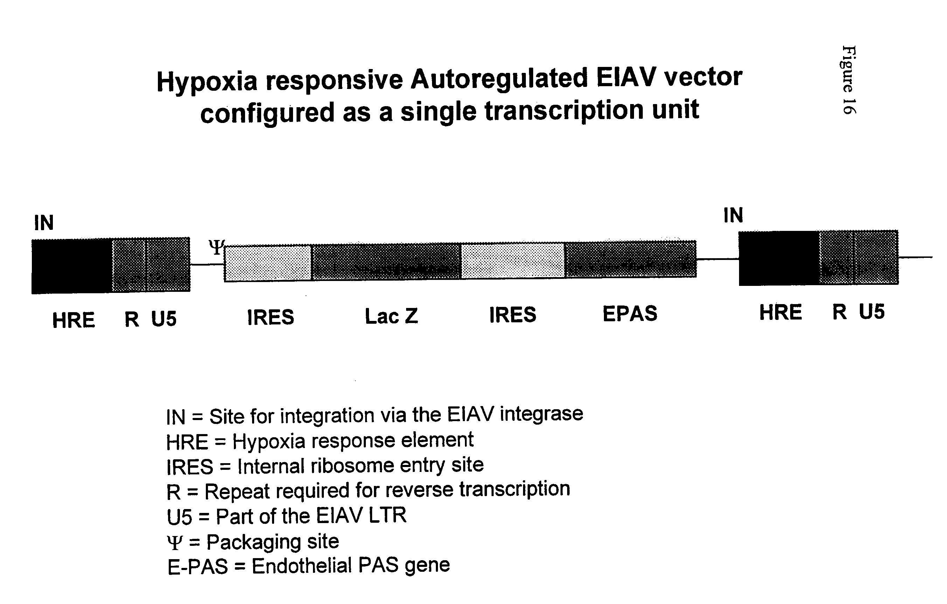

The auto-regulated vector that expresses E-PAS and lacZ is shown in Figure 16. The E-PAS coding sequence is obtained by PCR amplification using primers according to the known sequence of the cDNA (Accession number U81984; Tian *et al*., 1997) and is cloned downstream of the EMCV IRES. The subsequent IRES EPAS1 cassette can then be inserted downstream of the IRES lacZ cassette described in Example 10 and shown in Figure 15.

This approach is not limited to lentiviral systems and may be used in any of the HRE regulated NOI delivery systems, viral and non-viral referred to throughout.

### Example 12 - Construction of a hypoxia responsive adenoviral vector for the expression of a therapeutic gene.

Another viral vector system widely used to deliver genes to cells is the adenovirus-based system. Adenoviruses are capable of infecting a wide variety of cell types, including non-dividing cells and can be grown to high titres. Adenoviruses do not integrate into the host cell genome and are thus generally only suitable for short-term gene delivery. However, transient gene therapy may be a more appropriate approach in some circumstances.

In particular, adenoviruses may be advantageously used to infect stem cells for use in gene therapy. When the progeny of the stem cells differentiate and migrate to target tissue they are still capable of delivering a therapeutic gene without division and differentiation. Since the adenovirus does not integrate into the cell genome, as the stem cell divides the vector is gradually lost from the cell population. However significant vector is still present in the progeny cells following differentiation into various lineages. Thus a population of differentiated cells can be derived from the stem cell which contain the adenovirus without permanently modifying the stem cell.

A first generation recombinant adenoviral vector (E1/E3 deleted) has been constructed such that the bacterial β-galactosidase reporter gene is under the control of a hypoxically regulated promoter.

The first generation adenovirus vectors consist of a deletion of the E1 and E3 regions of the virus allowing insertion of foreign DNA, usually into the left arm of the virus adjacent to the left Inverted Terminal Repeat (ITR). The viral packaging signal (194-358 nt) overlaps with the E1a enhancer and hence is present in most E1 deleted vectors. This sequence can be translocated to the right end of the viral genome (Hearing and Shenk, 1983). Therefore, in an E1 deleted vector 3.2 kb can be deleted (358-3525 nt).

Adenovirus is able to package 105% length of the genome, thus allowing for addition of an extra 2.1kb. Therefore, in an E1/E3 deleted viral vector the cloning capacity becomes 7 - 8 kb (2.1 kb +1.9 kb (removal of E3) and 3.2 kb (removal of E1)). Since the recombinant adenovirus lacks the essential E1 early gene it is unable to replicate in non-E1 complementing cell lines. The complementing 293 cell line has been developed by Graham *et al*. (1977) and contains approximately 4 kb from the left end of the Ad5 genome including the ITR, packaging signal, E1a, E1b and pIX. The cells stably express E1a and E1b gene products, but not the late protein IX, even though pIX sequences are within E1b.

In non-complementing cells the E1 deleted virus transduces the cell and is transported to the nucleus but there is no expression from the E1 deleted genome.

The general strategy involves cloning the foreign DNA into an E1 shuttle vector, where the E1 region from 402-3328 bp is replaced by the foreign DNA cassette. The recombinant plasmid is then co-transfected into 293 cells with the pJM17 plasmid (Microbix Biosystems Inc. (Ontario, Canada)).

pJM17 is used to construct adenovirus type 5 vectors with inserts/mutations in early region 1 (E1). pJM17 contains a deletion of the E3 region and an insertion of the prokaryotic pBR322 vector derivative pBRX (including the ampicillin resistance and bacterial ori sequences) into the E1 region at 3.7 map units. This 40 kb plasmid is therefore too large to be packaged into adenonucleocapsids but can be propagated in bacteria. Intracellular recombination in 293 cells results in replacement of the amp^{r} and ori sequences with the insert of foreign DNA.

In the examples described below two transfer vectors have been used. The first obtained from Microbix is called pElsplA and the second obtained from Quantum Biotechnologies is called pAdBN. The pAdBN plasmid has the advantage that the new (foreign) DNA can be inserted in either orientation. This places the insert in a different spatial relationship with the resident adenoviral genes which can in some cases adversely affect expression. In both cases the second DNA is a defective version of the adenoviral genome, either as a plasmid for example pJM17 or as a part of the viral DNA for example the so-called right arm of Ad5. Homologous recombination generates the final gene transfer vector.

The construction of the hypoxia regulated adenoviral vector is described below:
The luciferase gene in OB37 (Figure 1) is replaced by the bacterial β-galactosidase encoding gene via an NcoI-XbaI fragment swap from the pONY2.1 vector (Figure 11b). The resulting OBHRELacZ cassette is removed from the OB37 vector as a KpnI-SalI fragment and cloned into the Quantum Biotechnologies™ pAdBN transfer vector producing Ad.OBHRElacZ.

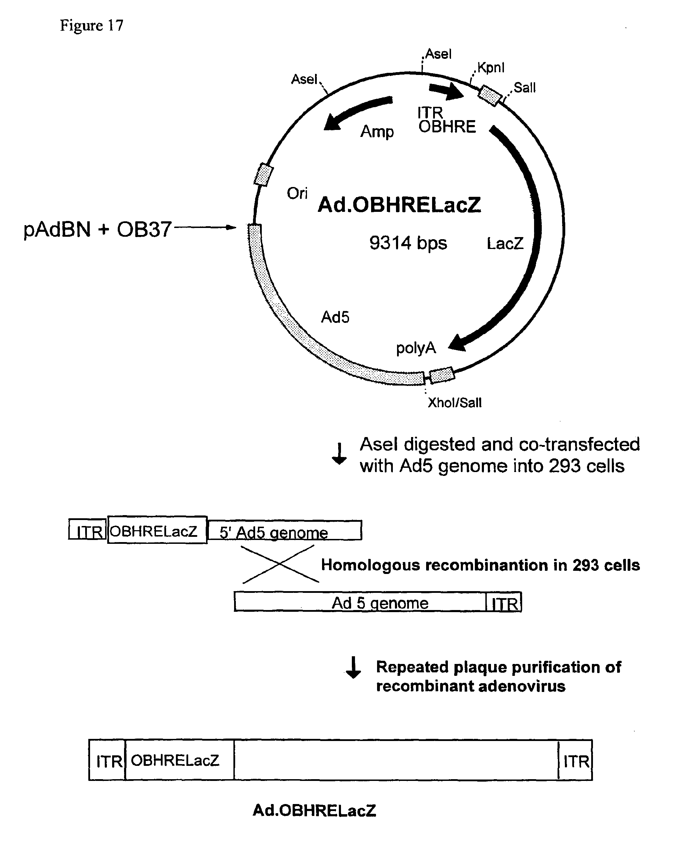

The recombinant Ad.OBHRElacZ transfer vector is linearised (AseI) and co-transfected into 293 cells along with the purified right arm of the Ad5 virus (from the ClaI site) to allow *in vivo* homologous recombination to occur resulting in the formation of the desired recombinant adenovirus. This is outlined in Figure 17. Adenoviral vectors containing the HRE are referred to as AdHRE followed by the inserted gene, for example Ad.OBHRElacZ has the bacterial β-galactosidase gene expressed by the OBhre11 promoter. Various different nucleic acid sequenes of interest may conveniently be cloned into the multiple cloning site downstream of the OBhre11 promoter. The MCS consists of 5' BgIII-AscI-Asp718-KpnI-XhoI-HindIII-EcoRV-SnaBI-BcII-MluI-NotI-3'.

The inserted DNA construct present in the adenoviral transfer vector is in the form of an autonomous expression cassette containing the OBhre11 promoter, the LacZ coding sequence and the SV40 polyadenylation signal (splice sites can also be included if necessary). In this system described for the construction of AdHRE vectors we have observed that a consistently higher level of protein expression is obtained if the expression cassette is directed in the orientation of the E1 genes. Therefore, to get tight regulation from the OBhre11 promoter (i.e. low basal level of expression in normoxia) the OBHRELacZ expression cassette was cloned in the direction of the E1 genes (minimising any transcriptional interference from the remaining adenoviral genome).

A range of different cell lines are transduced with Ad.OBHRElacZ. Exponentially growing cells were transduced with an MOI of 20-100 of the recombinant adenovirus in DMEM supplemented with 5% FCS for 90 minutes with frequent gentle shaking for 6-8 hours before the virus was removed and replaced with fresh media appropriate for the cell line. Transduced cells were then either incubated for 16 hours under normoxic (20% O₂, 5% CO₂, 75% N₂) or hypoxic conditions as described in the results section using multigas incubators purchased from Heto, or in the presence of 150 µM of either cobalt chloride or desferrioxamine (Sigma).

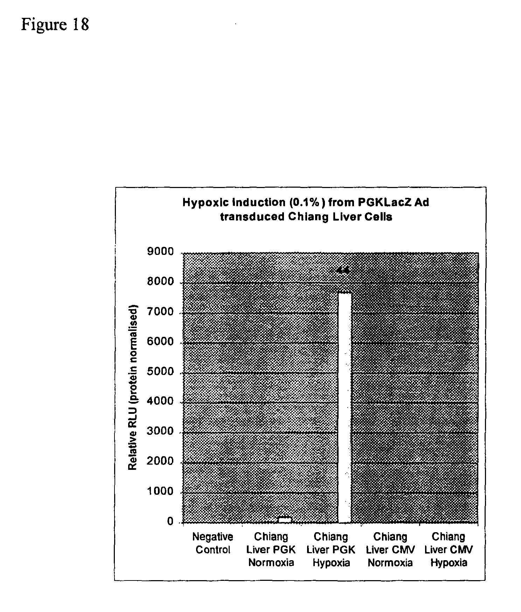

The results for two cell types shown in Figure 18 demonstrate the hypoxic inducibility of the LacZ reporter gene within the adenoviral vector in Chiang Liver and the MCF-7 human breast cancer cell line.

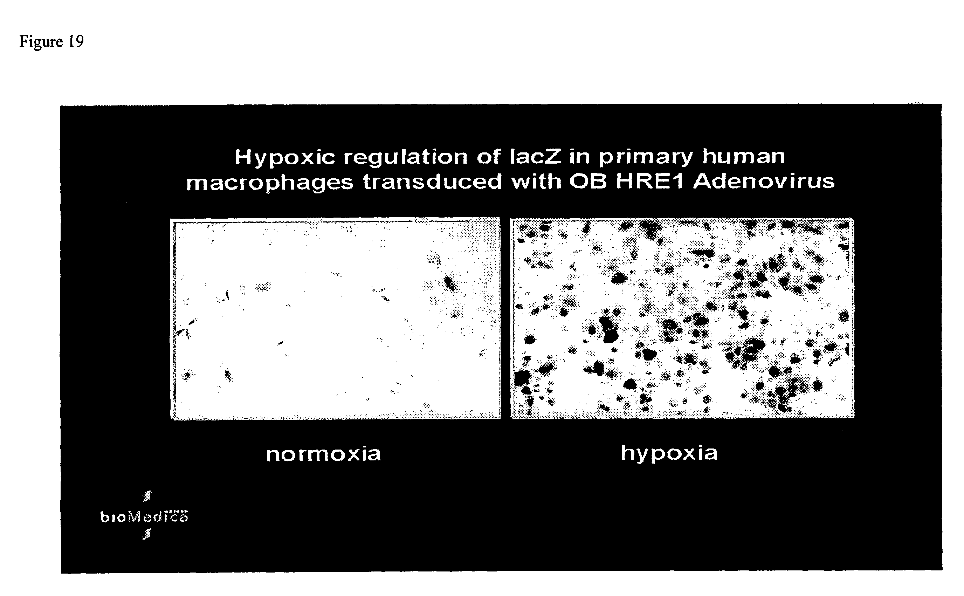

In addition, 7-14 day old primary human macrophages are similarly transduced with Ad.OBHRElacZ and incubated under either normoxic or hypoxic conditions. Primary human macrophages (isolated from buffy coats and cultured in teflon bags in serum free AIM-V medium supplemented with male AB serum) were transduced (on teflon) overnight with an MOI of 150-200 of the Ad.OBHRElacZ virus at a cell density of 2 x 10⁵ 100 µl in DMEM supplemented with 5% fetal calf serum. The cells were then split for incubation under either normoxic or hypoxic conditions. Results showing the hypoxic induction of the lacZ reporter gene are shown in Figures 18 and 19.

These results demonstrate not only the transducibility but also the utility of a HRE regulated recombinant adenovirus in cells in the haematopoietic lineage.

### Example 13 - Construction of hypoxia regulated adenoviral vectors comprising NOIs encoding therapeutic products

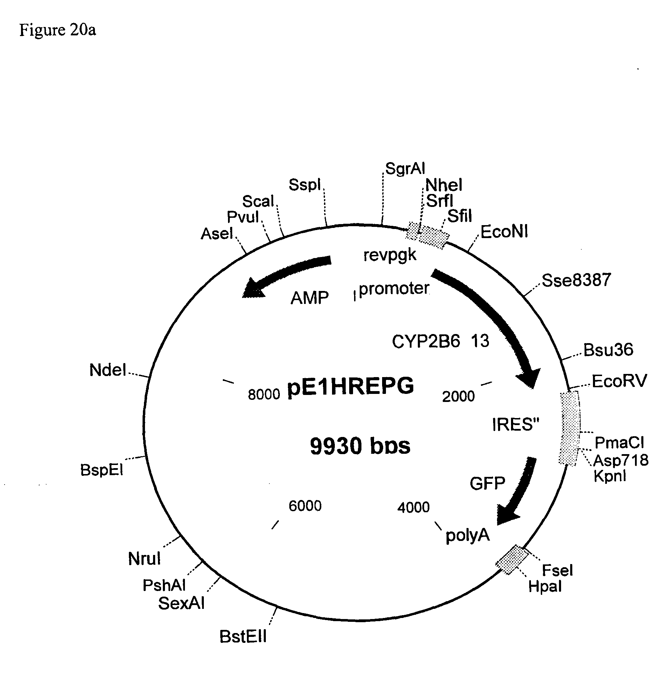

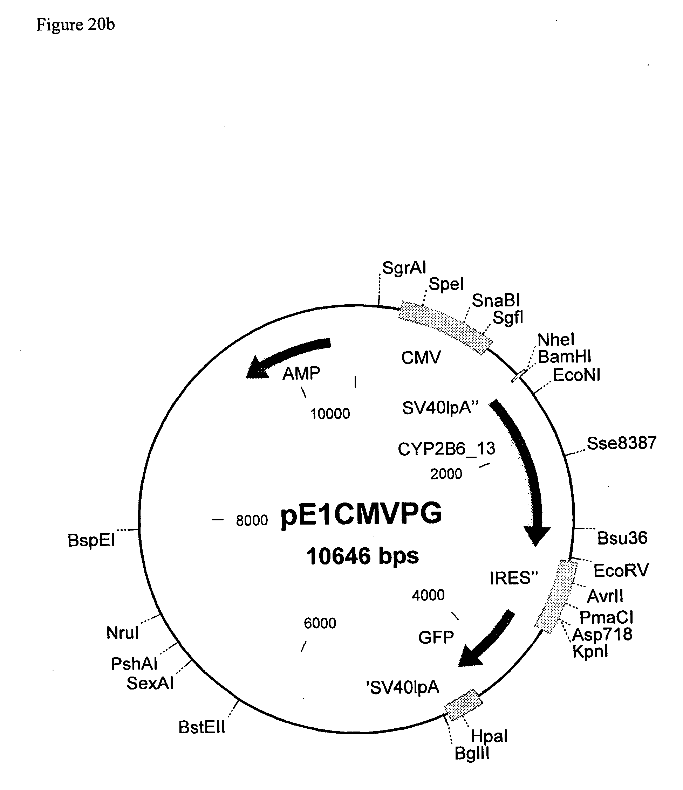

An example is described below for the construction of AdHRE-2B6 and AdCMV-2B6 recombinant adenoviral vectors using the Microbix Biosystems construction system. Plasmids are shown in Figure 20 and are as follows:
pE1HREPG - OBHRE-2B6 transfer vector (engineered to contain the OBhre11 driven 2B6 expression cassette)
   The EMCV IRES GFP was taken from pIRES2-EGFP (obtained from Clontech Laboratories UK Limited (cat no. 6064-1)) as an EcoRI-HpaI fragment and blunt cloned into the XbaI-HpaI sites of pGL30BHRE11p450 vector 3' to the 2B6 coding sequence (pGL3OBHRE11p450 was obtained by cloning the 2B6/p450 coding sequence - see Example 6 - as a HindIII-XbaI fragment into OB37 to replace the luciferase reporter gene).
   The complete expression cassette is cloned into the Microbix transfer vector pΔE1sp1B as *Mlu*I-*PshA*I fragment to give pE1HREPG (Figure 20a).
   pJM17 and the pΔE1sp1B transfer vector were all obtained from Microbix Biosystems Inc. (Ontario, Canada). pJM17 is used to construct adenovirus type 5 vectors with inserts/mutations in early region 1 (E1). The plasmid has an insertion of a pBR322 derivative in the E1 region making it non-infectious in single transfections of helper cells such as 293 since the viral genome is too large to be packaged. Co-transfection with a derivative of transfer vector, pΔE1sp1B, allows *in vivo* recombination to occur giving rise to packagable E1- recombinant adenoviral vectors.
pE1CMVPG - CMV-2B6 transfer vector (engineered to contain the CMV driven 2B6 expression cassette )

The Bglll-NaeI CMV2B6 fragment from pCI-2B6 is cloned into the BamHI-EcoRV site of pΔE1sp1B. The EMCV IRES2 EGFP EcoI-XbaI fragment from is blunt-cloned into the XbaI site 3' to the 2B6 coding sequence in the resulting plasmid to create pE1CMVPG (Figure 20b).

pCI-2B6 is obtained by cloning the 2B6 cDNA as described above such that the PCR product could be cloned into the NheI-XbaI sites of the Promega pCI-neo mammalian expression vector.

Note: The use of the EMCV IRES2 EGFP reporter allows easier plaque purification of the recombinant adenovirus and provides viable cell marker for studying gene expression during different physiological conditions.

Any NOI or combination of NOIs may be inserted into the adenoviral vector as described above.

### Example 14 - Construction of Adenoviral vectors to deliver lentiviral components

Combination adenovirus/lentivirus vectors have been described. To produce lentiviral vectors, four adenolentiviral constructs generally need to be made: genome, *gagpol*, envelope (for example rabies G) and, optionally, Rev. These are typically contained in four different adenovirus constructs. The lentiviral components are expressed from heterologous promoters they contain introns where needed (for high expression of gagpol, Rev and Rabies envelope) and a polyadenylation signal. When the four viruses are transduced into Ela minus cells the adenoviral components will not be expressed but the heterologous promoters will allow the expression of the lentiviral components. By way of example the production of an adenoviral vector that can produce a lentiviral vector is now described.

An example is outlined below of the construction of an EIAV adenoviral system (U.K. patent application no. 9727135.7). The EIAV is based on a minimal system, that is one lacking any of the non-essential EIAV encoded proteins (S2, Tat or envelope). The envelope used to pseudotype the EIAV is the rabies envelope (G protein). This has been shown to pseudotype EIAV well (U.K. patent application no. 9811152.9).

### Transfer Plasmids

Described below is the construction of transfer plasmids, based on transfer plasmid pE1sp1A, containing the EIAV components. The recombinant transfer plasmids can then be used to make recombinant adenoviruses by homologous recombination in 293 cells.

### Plasmid construction details

### pONY3

This EIAV *gagpol* expression plasmid (pONY3) is made by inserting the MluI/MluI fragment from pSPEIAV19ΔH (see Example 9) into the mammalian expression plasmid pCI-neo (Promega) cut with MluI such that the *gagpol* gene is expressed from the hCMV-IE promoter-enhancer. Plasmid pONY3 should not produce a functional genome because it lacks the appropriate LTR sequences.

### pONY3.1

The remaining 5' LTR of pONY3 is removed by cutting pONY3 with NarI and EcoRV. The 2.4 kb fragment is inserted into pBluescript KS⁺ (Stratagene) at ClaI and EcoRV sites to make construct pBSpONY3.0. pBSpONY3.0 is cut with XhoI and EcoRV and the 2.4 kb fragment is inserted into pONY3 at XhoI and EcoRV to make pONY3.1. This manipulation removes the 5' LTR up to the NarI site within the primer binding region (386nt).

### A. pE1RevE - provides the Rev protein required for the efficient expression of gag and pol

Firstly, for construction of pCI-Rev, the EIAV REV encoding sequences were derived by PCR amplification. The EIAV REV was obtained by PCR amplification as follows. Using pONY3 as the template, two amplifications were performed to obtain the two parts of the EIAV RRE. The 5' element was obtained using primers EIAV.REV-5'O (CCATGCA CGTCTGCAGCCAGCATGGCAGAATCGAAG) and EAIV.REV-IN (CCTGAGGA TCTATTTTCCACCAGTCATTTC) and the 3' element with EAIV.REV-IP (GTGGAAA ATAGATCCTCAGGGCCCTCTGG) and EIAV.REV3'O (GCAGTGCCGGATCCTCAT AAATGTTTCCTCCTTCG These products will anneal to each other hence can be used in the second PCR reaction to obtain a DNA which 'encodes' the EIAV REV. The second PCR amplification was carried out initially with primers EAIV.REV-IN and EAIV. REV-IP, with primers EIAV REV5'O and EIAV REV3'O being added after 10 cycles.

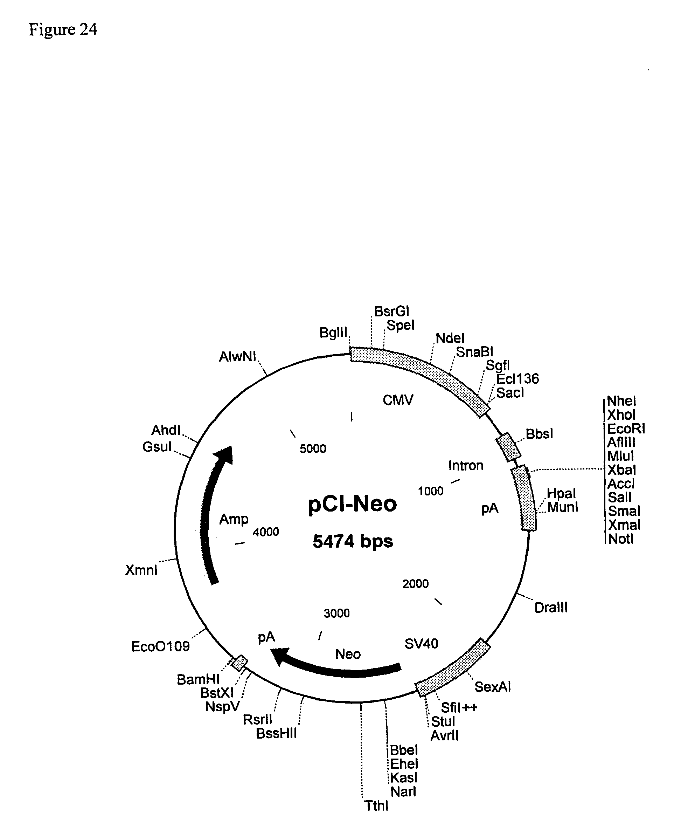

The resulting product was ligated with the PCR fragment 'TA' cloning vector pCR2.1 (Invitrogen) the orientation of the EIAV REV insert was assessed by restriction enzyme analysis and the presence of the correct EIAV REV sequence confirmed. The EIAV REV insert was excised from pCR2.1 by digestion with SpeI and NotI and ligated into pCI-Neo (Figure 24), which had been digested with NheI and NotI, to produce pCI-Rev.

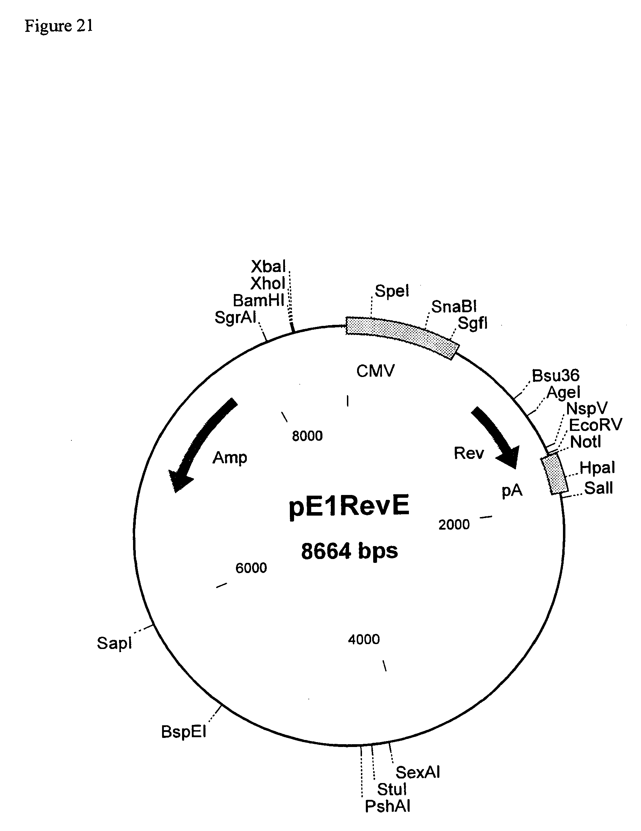

The plasmid pCI-Rev is then cut with ApaLI and ClaI. The 2.3 kb band encoding EIAV Rev is blunt ended with Klenow polymerase and inserted into the EcoRV site of pE1sp1A to give plasmid pE1RevE (Figure 21).

### B. pE1HORSE3.1 - gagpol construct

Firstly, for construction of pHORSE, the EIAV gagpol encoding sequences were derived by PCR amplification as follows. Using pONY3 as the template, two amplifications were performed to obtain two fragments of the EIAV gagpol. The 5' element was obtained using primers EGAGP.5'OUTER (CCATGCACGTCTCGAGCCAGCATGGGAGACC CTTTGAC) and EGAGP.INNER3 (GCAATGGAATGACATCCCTCAGCTGCCAGT CC) and the 3' element with EGAGP.3'OUTER (CGAGCTAGAGGTCGACTCA ATTTGGTTTATTAGTAAC) and EGAGP.INNER5 (GGGATGTCATTCCATTGCCA CCATGGGAAGTATTTATCACTA). These two products will anneal to each other hence can be used in the second PCR reaction to obtain a DNA which 'encodes' the EIAV gagpol.

The two PCR products are purified pooled and re-amplified using primers EGAGP.5'OUTER and EGAGP.3'OUTER. This product is inserted into the XhoI and SalI sites of pSP72 to make pSP72EIAVgagpolO'lap. pONY3 is cut with PvuII and NcoI and the 4.3 kb fragment is inserted into pSP72EIAVgagpolO'lap cut with PvuII and NcoI to make pSP-EGP. This is cut with XhoI and SalI (4.7 kb) and inserted into pCI-Neo at the XhoI and Sal I sites. This construct is called pCI-EGP. The RRE is cut out from pEGASUS-4(+) with SalI (0.7 kb) and inserted into pCI-EGP construct at the *Sal* I site to make pHORSE.

pHORSE3.1 is made by replacing the 1.5 kb XhoI/XbaI of pHORSE with the 1.6 kb XhoI/Xba I of pONY3.1.

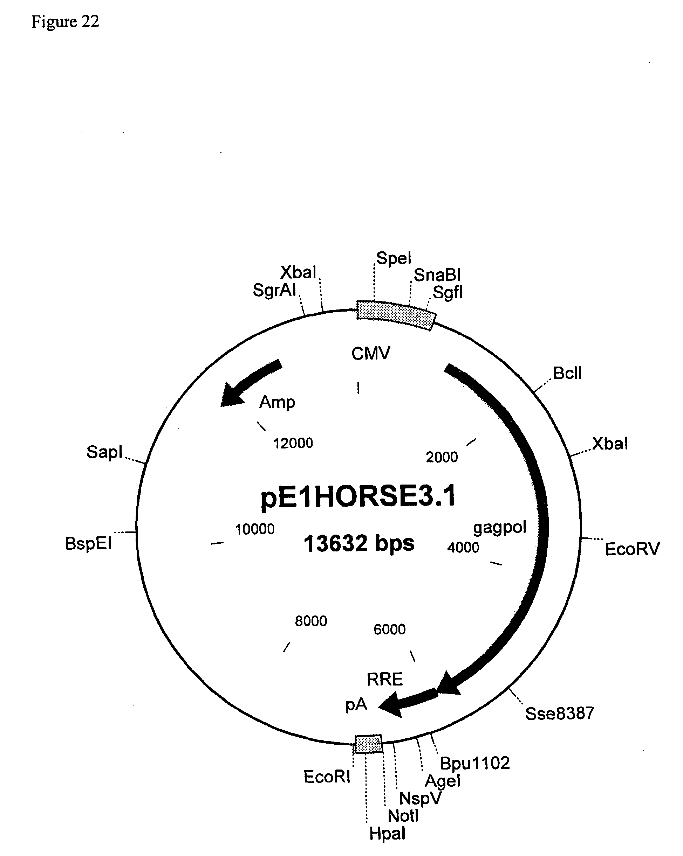

pHORSE3.1 is then cut with SnaBI and NotI. The 6.1 kb band encoding EIAV gagpol is inserted into pE1RevE cut with SnaBI and NotI (7.5 kb band is purified). This gives plasmid pE1HORSE3.1 (Figure 22).

### C. pE1PEGASUS - Genome Construct

pEGASUS-4(+) is cut with BglII and NotI. The 6.8 kb band containing the EIAV vector genome is inserted into pE1RevE cut with BglII and NotI (6.7 kb band is purified). This gives plasmid pE1PEGASUS (Figure 22).

### pCI-Rab

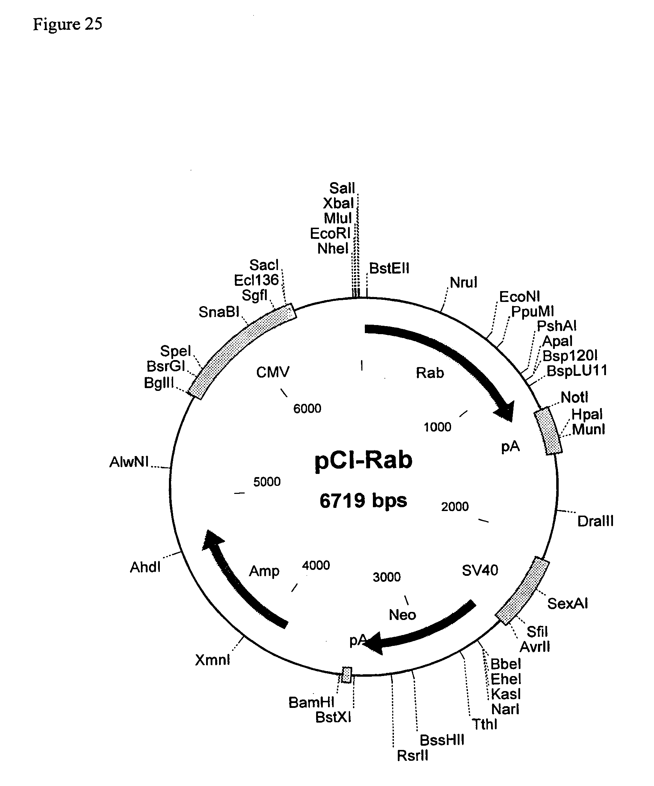

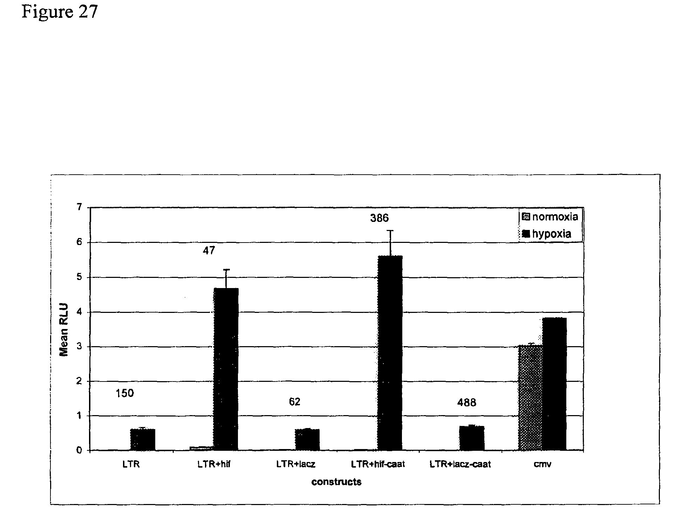

The rabies virus G-protein open reading frame is inserted into pCI-Neo (Figure 27) by cutting plasmid pSA91RbG with NsiI and AhdI. The 1.25 kb band is bluntended with T4 DNA polymerase and inserted into pCI-Neo cut with SmaI. This gives plasmid pCI-Rab (Figure 25).

pSA91RbG, as described in U.K. patent application no. 9811152.9, was constructed by cloning a 1.7 kb BglII rabies G fragment (strain ERA) from pSG5rabgp (Burger *et al*., 1991) into SA91, a derivative of pGW1HG (Soneoka *et al*., 1995) from which the *gpt* gene has been removed by digestion with BamHI and re-ligation.

### D. pE1Rab - Rabies Construct

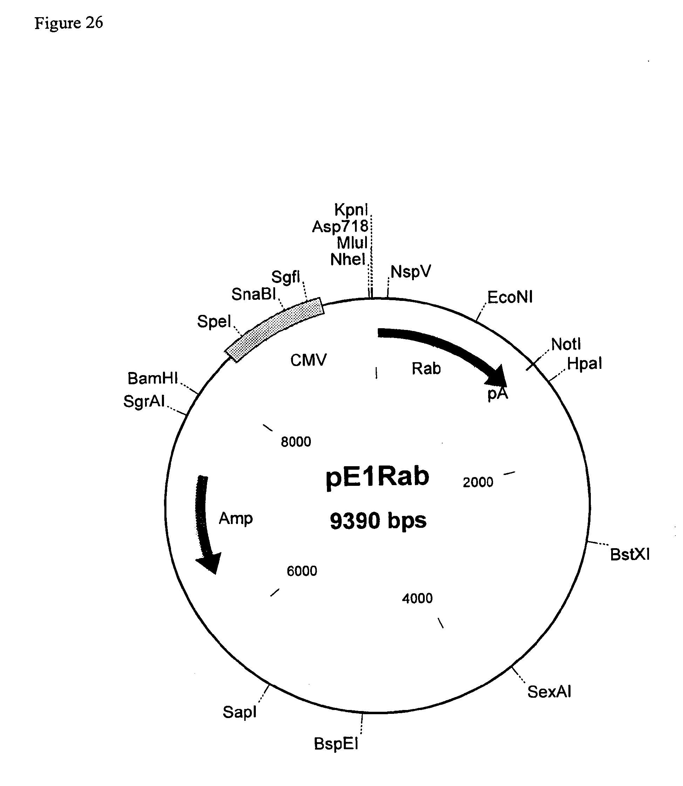

pCI-Rab is cut with SnaBI and NotI. The 1.9 kb band encoding Rabies envelope is inserted into pE1RevE cut with SnaBI and NotI (7.5 kb band is purified). This gives plasmid pE1Rab (Figure 26).

### Example 15 - Engineering stem cells to express a prodrug activating enzyme in response to hypoxia.

The retroviral vector, XiaGen-P450 or a lentiviral vector containing the human thrombospodin gene under the control of the OBhre11 construct may be used to transduce any cell type. As an example, we use human haematopoietic stem cells (HSCs), human peripheral blood buffy coat and human cord blood. Procedures for isolation of CD34⁺ HSCs and retroviral mediated gene transfer into these cells have been described previously (such as Charbord *et al*., 1996; Dunbar *et al*., 1996; Cassel *et al*., 1993; Emmons *et al*., 1997; WO-A-96/33281; WO-A-96/09400). An example of a suitable method is as follows.

HSCs are harvested from peripheral blood after mobilisation with G-CSF and/or cyclophosphamide (Cassel *et al*., 1993). G-CSF (Amgen) is given at a dose of 10 µg/kg/day sub-cutaneously for 7 days. Apheresis and enrichment of HSCs is carried out using the CellPro Stem Cell Separator system (Cassel *et al*., 1993). The HSC-enriched population is cultured at 10⁵ cells/ml in spent medium from RRV producer cells (Example 1) in the presence of 4 µg/ml protamine sulphate and 20 ng/ml IL-3 (Sandoz), 50 ng/ml IL-6 (Sandoz), 100 ng/ml SCF (Amgen) (Santiago-Schwartz *et al*., 1992; Charbord *et al*., 1996; Dao *et al*., 1997; Piacibello *et al*., 1997; Cassel *et al*., 1993). Other cytokines and/or autologous stromal cells prepared as described (Dunbar *et al*., 1996, 231) may also be added. After 24 hours the cells are centrifuged and resuspended in fresh RRV-containing medium with growth factors and protamine sulphate as above. This is repeated after a further 24 hours and the cells cultured for up to a further 48 hours. After this time the cells are trypsinised, washed several times in fresh medium by centrifugation and resuspended in Plasma-Lyte A for re-infusion. The total volume for re-infusion is approximately 25 - 50 ml. Patients are infused over a period of up to two hours. The number of cells infused is at least 10⁵ cells and may be up to the order of 10¹² cells.

Cells may also be matured along the myeloid differentiation pathway prior to re-infusion according to published methods (Haylock *et al*., 1992).

Transduction is as follows as described for retroviral vectors but the same methods are used for lentiviral vectors: Gene transfer with retroviral or lentiviral vectors is enhanced by optimising culture conditions of isolated CD34 cells such that they are cycling at the time of transduction. This is particularly necessary with MLV based vectors that require cell division to enable nuclear access and integration. Pseudotyping MLV vectors with different envelopes has also had a major impact on gene transfer. As reported in the literature GALV is markedly better than VSVG.

We have used a defined medium containing to ensure that cells are cycling at the time of transduction. Use of a defined media ensures that proliferative factors are present in the absence of anti-proliferative factors that may be found in serum or more complex media.

Upon isolation CD34 cells are transferred to the following media (based principally on Becker *et al*., 1998) for at least 24 hours prior to transduction. Serum-free medium X-VIVO 10, 1% BSA, 2mM L-glutamine, 1% pen/strep, 20 ng/ml IL 3, 100 U/ml IL 6, 50 ng/ml SCF, 100 ng/ml anti-TGFβ, 100 ng/ml Flt 3-L. The transduction protocol outlined (Becker *et al*., 1998) is used. This involves three cycles over 3 days and optimises the likelihood of the majority of cells undergoing mitosis in the presence of viral vector. The method is briefly as follows:-
Coat non-tissue culture grade plates with fibronectin fragment CH-269 at 10 µg/cm² (Takahara). Add virus to empty wells and allow to bind for 30mins. Wash with PBS. Add 10⁵ cells in 0.5ml media per well. Add 0.5ml virus of supernatant. Centrifuge at 1020 xg for 90 min. After 4 hours replace with fresh media as above. This is repeated for 3 consecutive days.

Cells isolated from peripheral blood or from sources derived from peripheral blood preferentially form a higher proportion of erythroid progenitors whereas CD34 cells from cord blood results in the formation of a broader spread of progenitor type. Cord blood derived cells have a higher inherent proliferative capacity. Increased stem cell numbers can be derived from peripheral blood that has been 'mobilised' prior to isolation with GM CSF. This approach is widely used in transplant patients.

Cells are then plated out in methocel medium containing growth factors (Methocel containing 10% fetal bovine serum, transferrin, glutamine, 2-mercaptoethanol, bovine serum albumin, IL3, IL6, IL11, SCF, Epo, GCSF, GMCSF) in a dark Class II safety cabinet according to the following protocol. Using a blunt needle attached to a 2.5 ml syringe, dispense 2.5 ml methocel into a 10 ml U tube avoiding air bubbles. Dispense 0.5 ml of the cell suspension (containing 3x the cell number required for each well so that 1 ml of the methocel will contain the desired cell number of 500/2000 cells per well) in Iscove's medium (Gibco) into the tube containing the methocel and vortex gently. Gently dispense 1 ml of the methocel and cell mix into a non-tissue culture petri dish (35x10mm) or a single well coverglass chamber using a blunt needle and syringe. Triplicate dishes/wells can be set up from each sample. Place the dishes/chambers into a Falcon 3025 dish (150x25mm) housing small petri dishes of sterile distilled water to ensure correct humidity. Incubate dishes at 37°C, 5% CO₂ and 5% O₂ and analyse colonies after 14 days.

To enhance production of CD34 derived monocytes, culture conditions are phased to optimise formation of GM progenitors (Haylock *et al*., 1992) and subsequently monocyte progenitors. This is enabled using the CFU assay to monitor aliquots from the primary culture and ensure conditions are optimal for monocyte generation. Supplementation of culture media with relatively high doses of GM CSF to optimise generation of GM progenitors, followed by phased administration of MCSF as the dominant growth factor skews differentiation toward the myeloid lineage. Following transduction of the HSC they are differentiated to form GM precursors

The GM precursor colonies are analysed for the expression of the retroviral vector by observation of the fluorescence of GFP. This is assessed under normoxia and hypoxia.

Alternatively the stem cell population can be transduced using the same procedures as described above but immediately after isolation. They can then be frozen or used immediately for transfer into patients. In this case totipotent stem cells are preserved.

### Example 16 - Interaction of engineered HSCs with ovarian cancer xenografts

As an example of the invention we have chosen to study HSC infiltration into tumours using ovarian cancer as the test system. This cancer is largely confined to the peritoneal cavity. A number of animal models are available to analyse HSC infiltration into ovarian tumours. Three human ovarian cancer xenografts OS, HU and LA have been derived from primary human tumours and established intraperitoneally and they are maintained by serial passage by that route (Ward *et al*., 1987). None of these lines grow well in long term culture. All grow primarily as free floating ascites surrounded by mucin. This is not reflective of the human disease that exists as solid implanted tumour deposits with accompanying ascites. Intraperitoneal administration of TNF converts the HU and LA models from this ascitic state to implanted solid tumour deposits on the surface of the peritoneum and associated organs (Malik *et al*., 1989). This closely represented the pathology of the human disease and more specifically resembles the original pathology of the tumour from which they are derived. This solid tumour model has been used as a more relevant starting point for study of therapeutic agents in ovarian cancer. (eg. interferon gamma, Malik *et al*., 1991; Burke *et al*., 1997). Human stem cells engineered with the adenoviral or retroviral or lentiviral vectors described above are introduced at a concentration of 10⁶ /0.1 ml into the peritoneal cavity of mice bearing the tumours described above. The HSCs express the therapeutic genes. The action of these genes extends beyond the HSC i.e. there is a bystander effect so that the surrounding tumour tissue is killed. In a particular example of this invention the vector XiaGen-P450 is used. The mice are treated with the engineered HSC and then treated with cyclophosphamide at 100 mg/kg administered intraperitoneally. The tumours of mice treated with engineered HSC are killed by the cyclophosphamide.

Similar procedures are used with AdHRE vectors following the published stem cell transduction procedure as follows. Adenoviral vector is used to transduce HSC at a high multiplicity of infection (100 to 500) in a small volume of cullture medium containing 200 units/ml IL-3, 200 units/ml GM-CSF, 200 units/ml G-CSF for 24 hours. After transduction with the adenoviral vector the HSC can either be differentiated or used directly. If differentiated cells are to be used then colony forming units-granulocye/macrophage (CFU-GM) are quantified in soft agar containing the above cytokines after incubation for 14 days. The CD34+ cells or differentiated cells are introduced into the animal and migrate to the target tissue and express a therapeutic gene.

### Example 17 - Treatment of ovarian cancer with modified human stem cells

Peripheral blood lymphocytes are collected from ovarian cancer patients as follows. The cells are transduced with the XiaGen-P450 retroviral vector as described above. The engineered stem cells are returned to the patient via an intraperitoneal injection broadly as described for monocytes by Stevenson *et al*., 1987. Basically 10⁸ to 10⁹ cells in 50 ml of isotonic saline are introduced directly into the abdominal cavity via a needle catheter monitored by ultrasound. The cells are distributed widely in the peritoneal cavity and remain associated with serosal surfaces and do not traffic to other organs. After 1 to 7 days the patients are treated with cyclophosphamide and tumours are analysed after a further 2 weeks. Reduction in tumour mass is a consequence of local activation of the cyclophosphamide by the engineered stem cells.

Similar procedures are used with AdHRE vectors following the stem cell transduction procedure outline above.

### Example 18 - Transduction of tumour cells with lenti-viral vectors

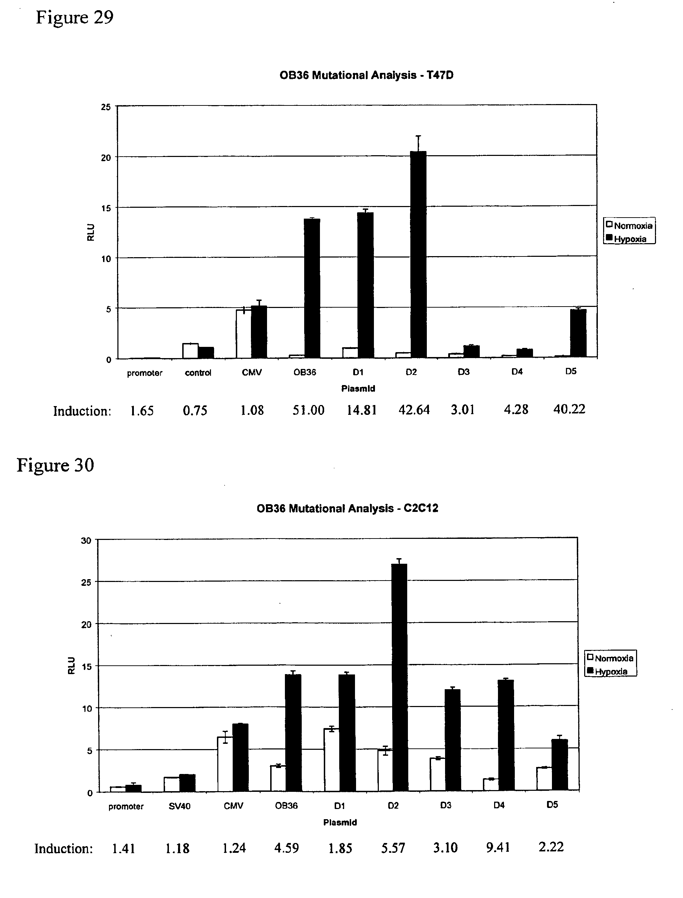

As a further example of the present invention we have chosen to study gene transfer to glioma. Gliomas are characterised by hypervascularity and invasiveness yet they are one of the most hypoxic tumours that have been studied [J. Folkman pp3075- 3085 In Cancer: Principles and Paractice of Oncology, Fifth Edition ed DeVita, Hellman, Rosenberg. Pub. Lippincott-Raven 1997]. A lentiviral vector is configured to deliver a combination therapy of a prodrug activating enzyme and an anti angiogenic factor, in this example this is the human cytochrome P450 gene and the anti-angiogenic factor TSP-1, both under the control of an HRE construct (as for Figure 16 but with lacZ and E-PAS replaced with P450 and TSP-1 sequences). The expression is activated in hypoxia thus ensuring that the therapy is administered in the local environment of the tumour. A suitable lentiviral vector is described in the Figure 30. The model systems use either the human cell line U87MG or the rat RT-2 line. Both give typical vascularised tumours that can be analysed either in normal rats or in nude or SCID mice (e.g. Wei *et al*., 1994). In both cases the tumour model is created by the intra-cerebral implantation of tumour cell lines. Lentiviral vector preparations are injected directly into the tumour mass at multiple sites. Vector is delivered in 1 to 3 µl amounts at a titre of at least 10⁴ transducing units/µl. The same procedure is used in treating human patients. In this case the tumour is located by PET or MRI scanning and injected with vector in 0.1 ml aliquots or alternatively at the time of surgical debulking the site can be treated with vector. Patients are treated with cyclophosphamide and the reduction in tumour growth is monitored by MRI scanning.

### Example 19 - Induction of lentiviral vector production and expression by desferrioxamine

Desferrioxamine is obtained from Sigma or as a clinical formulation from Novartis Pharmaceuticals as the licensed product Deferal. The level of induction achieved with Desferal is equal to or greater than that achieved by hypoxia.

For *in vitro* use producer cells containing the hypoxia regulated lentiviral vectors are cultured in flasks for 10 days in the presence of 50 micromolar to 1 millimolar desferrioxamine. Cultures release vector particles during this period to give total yields in excess of 10⁷/ml. For scale up cells are cultured in roller bottles and desferrioxamine is used at 50 to 200 micromolar for seven days. This system therefore exploits the presence of the HRE to alow induction of viral vectors. The system is described whereby the genome is regulated by HRE in response to desferrioxamine. It follows that the other components i.e. the gagpol and the envelope can be similarly regulated. It follows that this system can be used to regulate the production of the components from any retroviral or lentiviral vector.

For *in vivo* use patients are treated with the hypoxia regulated lentiviral vector or with cells that contain the hypoxia regulated lentiviral vector. Patients are then give a standard course of treatment with desferrioxamine. This activates the therapy in addition to any effects of hypoxia and in some cases may replace the requirement for local hypoxia. For example if the cells are implanted to provide a therapeutic protein such as Epo or a blood clotting factor, such as factor IX then the delivery can be regulated by adjusting the dose of Desferal.

### Example 20 - HIF-1 autoregulated HRE constructs in an MLV retroviral vector

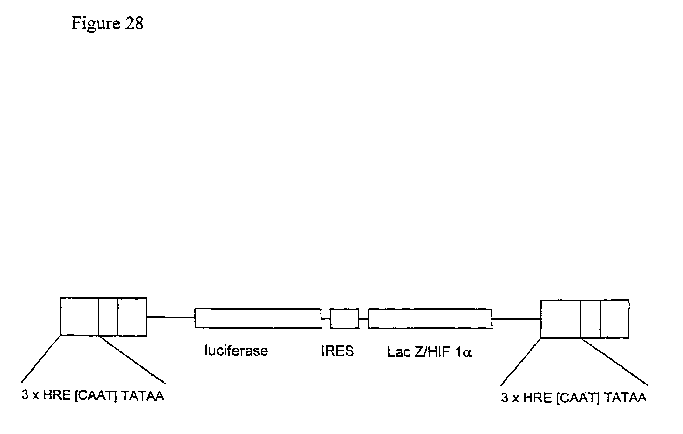

HRE HIF 1 autoregulated cassettes using 3 copies of the PGK derived HRE in place of the endogenous MLV enhancer and upstream of the core MLV promoter were constructed to further examine hypoxic induction using an HRE of the invention. Bicistronic casettes have been derived using the EMCV IRES to drive HIF-1α or control (lac Z) after a luciferase reporter (see Figure 28).

The results obtained show that co-expression of HIF-1α significantly increases expression under hypoxia however this is concomitant with a reduced induction ratio due to elevated normoxic expression (see Figure 27).

The core 'minimal' promoter of MLV largely consists of a CAAT box upstream of the TATAA box. We looked to see if removal of the CAAT box would reduce basal activity of the core promoter and thus reduce activity in normoxia. This is confirmed by the results shown in Figure 27 - i.e. removal of the CAAT box reduces the activity of the luc-IRES-HIF cassette in normoxia yet appears to maintain levels of activity under induction thereby improving the induction ration signiifcantly.

Thus, improved levels of induction can be gained by the co-expression of HIF-1. However this can be seen to elevate levels in normoxia. Reducing the activity of the core promoter has been shown to result in gains in the induction ratio. Indeed manipulation of the core promoter alone may improve induction profiles as well as in combination with HIF-1 expression. This thereby points to a route of improving the hypoxic switch and could be applied to other core promoters e.g. CMV and SV40 minimal promoters. Furthermore, this technique would be equally useful in cell targets where the normoxic level of the factors is unusually high as a way of reducing normoxic activity e.g. in C2C12 mouse skeletal muscle cells.

### Example 21 - Optimisation of OBHRE1 and OBHRE11 HRE

Although there is a consensus sequence for the HRE element, there are subtle single base variations found in naturally occurring HRE elements depending on the particular gene promoter. Furthermore, it has been suggested that particular members of the HIF family (bHLH PAS family) bind preferentially to certain half site sequences. By making single base substitutions in these half sites it may be possible to enhance the hypoxic response in particular cell types depending on the HIF/bHLH PAS family member expression profile in the cell target. Thus for example if a specific cell type expresses a specific HIF/bHLH PAS family member at high levels, then the HRE may be tailored accordingly to include half sites that are bound by that specific family member.

This was tested experimentally as explained below:
Five oligonucleotides were synthesised incorporating changes to the HRE binding sites in OBHRE1 (plasmid OB36). 
1 2
Mouse PGK1 GTCGTGCAGGACGTGACA (x3)
CAGCACGTCCTGCACTGT
3
 
D1 TACGTGCAGGACGTGACA (x3)
ATGCACGTCCTGCACTGT
The upstream GT flanking the HRE site 1 was changed to TA (found in the majority of other HREs). 
D2 GTCGTGCAGTACGTGACA (x3)
CAGCACGTCATGCACTGT
Site 2 was changed from GAC to TAC to make the site more like a HIF/MOP3 binding site. 
D3 GTCGTGCAGCACGTGACA (x3)
CAGCACGTCGTGCACTGT
Site 3 has been mutated to replace the final C with a G which is found in most other identified HREs. This also causes site 2 to mutate to CA*CGTGA* which is a MOP4/MOP3 binding site. 
D4 GTCGTGCAGGACGTGGCA (x3)
CAGCACGTCCTGCACCGT
Mutating this to G is predicted to make site 2 bind ARNT rather than MOP3.

D5 GTCGTGCAGTACGTGGCA (x3)
CAGCACGTCATGCACCGT

This construct includes the same mutation as D4. However the D2 mutation is also added. This results in a putative HIF/ARNT site.

### Results (see Figures 29 and 30)

D1 - This construct has increased normoxic expression levels, but has made little impact on hypoxic expression levels and therefore decreased the induction ratio when compared with OBHRE1.
D2 - This construct has clearly increased both the normoxic and hypoxic levels of expression.
D3 - Mutating the CGTC to CGTG in the mouse PGK HRE element has reduced, both normoxic and hypoxic levels of expression in T47D cells and caused a significant drop in induction ratio. However the effect is far less pronounced in C2C12 cells, indicating that the activity of this construct is highly dependent on the cell background tested.
D4 - Expression levels under hypoxia are low and induction ratio is decreased in T47Ds. Normoxic levels are decreased in C2C12s which significantly increases the induction ratio when compared with OBHRE1.
D5 Adding the D2 mutation to D4 has decreased both normoxic and hypoxic expression levels in T47Ds, preserving the induction ratio. In C2CI2 hypoxic expression levels are decreased which has severely affected induction ratio.

### Summary

The single base change in D2 shows an improvement to OBHRE1. The single base changes described above may be useful as a means of tailoring HRE function to a particular cell target according to the HIF transcription factor evident in that cell type.

### References

Ace et al., 1989, J Virol 63: 2260-2269
Becker et al., 1998, Hum Gene Ther 9, 1561
Beerrepoot *et al*., 1996, 56, 3747
Bender et al., 1987, J Virol 61: 1639-1646
Blatt 1994, Anticancer Res., 14, 2109
Blomer et al., 1997, J. Virol. 71: 6641-6649
Bodine et al., 1991, Exp. Hematol. 19: 206-212
Bregni et al., 1998, Gene Ther 5, 465
Burger et al., 1991 J.Gen. Virol. 72. 359-367
Burke et al., 1997, Eur. J. Cancer, 33
Cassel et al., 1993, Exp Haematol 21, 585
Charbord et al 1996 Br J Haematol 94: 449-454
Chou and Roizman, 1992, PNAS 89: 3266-3270
Chou et al., 1994, J. Virol 68: 8304-8311
Clarke and Gordon 1998, J Leukoc Biol 63, 153
Cornelisse et al.,1996, Pathol Res Pract 192: 684-693
Cosset et al., 1995, J. Virol. 69: 7430-7436
Cotton et al., 1993 J Virol 67:3777-3785
Crapper et al., 1991, The Lancet, 337, 1304
Dao et al 1997 Blood 89: 446-456
Dunbar et al., 1996, Hum Gene Ther 7:231-253.
Duphar and Emmons, 1994, Stem Cells 12: 563-576
Ema et al., 1997, Proc Natl Acad Sci USA 94, 4273
Emmons et al., 1997, Blood 89, 4040;
Firth et al., 1994, Proc Natl Acad Sci USA 91, 6496
Firth et al., 1995, J Biol Chem 270, 21021
Fisher et al 1996 Virolology 217: 11-22
Flamme et al., 1997, Mech Dev 63, 51
Fraser et al., 1990, Blood 76: 1071-1076
Frey et al., 1998, Blood 91, 2781).
Gorziglia et al., 1996 J. Virol. 70: 4173-4178;
Gosh-Choudhury et al., 1986, Gene 50: 161
Gossen and Bujard, 1992, PNAS 89: 5547-5551
Giardina and Grady 1995, Semin. Hematol. 32, 304
Gossen et al, 1995, Science 268: 1766-1769)
Graham et al., 1977, J Gen Virol 36, 59
Haylock et al., 1992, Blood 80, 1405
Hearing and Shenk 1983, Cell 33, 695
Inoue et al., 1989, J Biol Chem 264: 14954-14959
Jaggar et al., 1997 (Hum Gene Ther 8 2239
Kim et al., 1998, J. Virol., 72, 811-816.
Koong et al., 1994, Cancer Res 54, 1425
Krougliak et al., 1995, Hum. Gen. Ther. 6: 1575
Kuhl et al., 1987, Cell 50, 1057
Levrero et al., 1991, Gene 101: 195
Lieber et al., 1996 J. Virol. 70: 8944-8960
Lonergan et al., 1998, Mol. Cell Biol. 18: 732
MacLean et al., 1991, J Gen Virol 72: 632-639
MacGregor et al., 1991 Methods in Molecular Biology Vol7 ed E. J. Murray p217-235
Madan et al., 1993, Proc. Natl. Acad Sci. 90: 3928
Malik et al., 1989, Int. J. Cancer 44, 918
Malik et al., 1991, Cancer Res 51, 6643
Maxwell et al., 1999, Nature 399: 271
Meliillo et al 1996 J. Biol. Chem 272, 12236-12243
Miller, A.D. and G.J. Rosman, 1989, Biotechniques 7(9): p. 980-2, 984-6, 989-90.
Myoshi et al., 1998 J. Virol 72, 8150
Neering et al., 1996 Blood 88, 1147
Payne et al., 1998, J Virol 72, 483.
Pear et al., 1993, Proc Natl Acad Sci 90: 8392-8396
Peshavaria and Day, 1991, Biochem. J. 275: 427-433
Piacibello et al., 1997 Blood 89: 2644-2653
Rice and Knipe, 1990, J. Virol 64: 1704-1715
Santiago-Schwartz et al., 1992, J Leuk Biol 52: 274-281
Semenza and Wang, 1992, Mol. Cell. Biol. 12: 5447-5454
Semenza et al., 1996, J. Biol. Chem 51 32529
Smith et al., 1992, Virology 186: 74-86
Soneoka et al., 1995 Nucl. Acids Res. 23: 628-633
Stevenson et al., 1987, Cancer Res 47, 6100
Stratford-Perricaudet et al., 1992, J. Clin. Invest. 90:626-630
Takenaka et al., 1989, J. Biol. Chem. 264: 2363-2367
Taniguchi et al., 1995, J Cancer Res Clin Oncol 121, 516
Trian et al., 1997, Gene Dev. 11, 72
Voest et al., 1993, Cancer Chemother. Pharmacol. 31, 357
Wang and Semenza, 1993a, PNAS 90: 4304
Wang and Semenza 1993b, Blood, 82, 3610
Wang et al., 1995, J Biol Chem 270, 1230
Ward et al., 1987, Cancer Res 47, 2662
Watanabe et al., 1996, Blood 87, 5032
Watanabe et al., 1998, Leukaemia and Lymphoma 29, 439
Wei et al., 1994, Human Gene Therapy 5, 969
Weisener et al., 1998 Blood 92 2262-2268
Yeh et al., 1996, J. Virol. 70: 559
Zacharova et al., 1997, AIDS Res Hum Retroviruses 13, 719
Zhang et al., 1994, Mol Cell Biol 14, 8085

## Claims

1. A polynucleotide comprising at least three repeats of a phosphoglycerate kinase (PGK) hypoxia response element (HRE) operably linked to an SV40 promoter or an MLV promoter.

2. The polynucleotide according to claim 1, wherein the middle repeat comprises a deletion in the HIF-1 consensus binding site.

3. The polynucleotide according to claim 1 or claim 2 comprising at least three repeats of the HRE operably linked to the promoter and upstream (5' to) the promoter and at least three repeats of the HRE operably linked to the promoter and downstream (3' to) the promoter.

4. The polynucleotide according to any of claims 1 to 3 wherein the HRE repeats are direct repeats.

5. The polynucleotide according to any of claims 1 to 4 wherein the HRE comprises a nucleotide sequence as shown in SEQ I.D. No. 1 or SEQ I.D. No. 2.

6. The polynucleotide according to any of claims 1 to 5 comprising a nucleotide sequence as shown in SEQ I.D. No. 9.

7. The polynucleotide according to any of claims 2 to 6 comprising a nucleotide sequence as shown in SEQ ID. No. 3, SEQ ID. No. 4 or SEQ ID. No. 5.

8. The polynucleotide according to any of claims 2 to 7 operably linked to a nucleic acid of interest (NOI) such that the polynucleotide directs expression of the NOI in a host cell.

9. The polynucleotide according to claim 8 wherein the NOI encodes HIF-1.

10. The polynucleotide according to any of claims 1 to 9 wherein the NOI encodes IFN-γ.

11. The polynucleotide according to any of claims 1 to 10 wherein the promoter lacks a CAAT box sequence.

12. The polynucleotide according to any of claims 8 to 11 wherein the host cell is a tumour cell.

13. The polynucleotide according to of any of claims 8 to 12 wherein the NOI encodes a polypeptide of therapeutic use.

14. The polynucleotide according to any of claims 8 to 13 wherein the NOI encodes a polypeptide which is cytotoxic.

15. The polynucleotide according to any of claims 8 to 14 wherein the NOI encodes a polypeptide capable of converting a precursor prodrug into a cytotoxic compound.

16. The polynucleotide according to any of claims 12 to 15 wherein the NOI is selected from polynucleotide sequences encoding proteins involved in the regulation of cell division, enzymes involved in cellular metabolic pathways, transcription factors and heat shock proteins.

17. The polynucleotide according to any one of claims 12 to 16 for use in delivering the NOI to a mammalian cell.

18. A nucleic acid vector comprising a polynucleotide as defined in any of the preceding claims.

19. A viral vector comprising a polynucleotide as defined in any one of claims 1 to 17.

20. The viral vector according to claim 19 which further comprises a nucleotide sequence selected from (i) a nucleotide sequence encoding an inhibitory RNA molecule capable of effecting the cleavage, directly or indirectly, of VHL RNA; (ii) one or more inhibitory RNA molecules that bind to and prevent VHL RNA processing and/or expression; and (iii) a nucleotide sequence encoding a polypeptide capable of inhibiting the binding of VHL to Elongin B and/or Elongin C.

21. The viral vector according to claim 20 wherein said polypeptide is a non-functional derivative of wild type VHL.

22. The viral vector according to any one of claims 19 to 21 wherein the viral vector is a retroviral vector.

23. The viral vector according to any one of claims 19 to 21 wherein the viral vector is an adenoviral vector.

24. The viral vector according to claim 22 wherein the vector is a lentiviral vector.

25. A polynucleotide according to any one of claims 1 to 17, a nucleic acid vector according to claim 18 or a viral vector according to any of claims 19 to 24 for use in the treatment or prevention of a human or animal patient suffering from a disease in which hypoxia is a cause or a symptom or is otherwise present.

26. Use of a polynucleotide according to any one of claims 1 to 17, a nucleic acid vector according to claim 18 or a viral vector according to any one of claims 19 to 24 in the manufacture of a composition for the treatment or prevention of a human or animal patient suffering from cancer.

27. A pharmaceutical composition comprising a polynucleotide according to any one of claims 1 to 17, a nucleic acid vector according to claim 19 or a viral vector according to any one of claims 19 to 24 together with a pharmaceutically acceptable carrier or diluent.

28. The pharmaceutical composition according to claim 27 for use in the treatment or prevention of a human or animal patient suffering from a disease in which hypoxia is a cause or a symptom or is otherwise present, wherein an effective amount of the pharmaceutical composition is administered to the patient in need of such treatment.

29. Use of a pharmaceutical composition according to claim 27 in the manufacture of a medicament for the treatment or prevention of a human or animal patient suffering from cancer, wherein an effective amount of the pharmaceutical composition is administered to the patient in need of such treatment.

30. A method of producing a viral strain which method comprises introducing a polynucleotide as defined in any one of claims 1 to 17 into the genome of a virus.

31. A host cell comprising the polynucleotide according to any of claims 1 to 17.

## Patentansprüche

1. Polynukleotid, welches wenigstens drei Wiederholungen eines Phosphoglyceratkinase-(PGK-) Hypoxie-Response-Elements (HRE) umfaßt, die funktionsfähig mit einem SV40-Promotor oder einem MLV-Promotor verknüpft sind.

2. Polynukleotid nach Anspruch 1, wobei die mittlere Wiederholung eine Deletion in der HIF-1-Consensus-Bindungsstelle umfaßt.

3. Polynukleotid nach Anspruch 1 oder Anspruch 2, welches wenigstens drei Wiederholungen des HRE, die funktionsfähig mit dem Promotor verknüpft sind und aufstromig (5') zu dem Promotor liegen, und wenigstens drei Wiederholungen des HRE, die funktionsfähig mit dem Promotor verknüpft sind und abstromig (3') zu dem Promotor liegen, umfaßt.

4. Polynukleotid nach einem der Ansprüche 1 bis 3, wobei die HRE-Wiederholungen direkte Wiederholungen sind.

5. Polynukleotid nach einem der Ansprüche 1 bis 4, wobei das HRE eine Nukleotidsequenz umfaßt, wie sie in SEQ ID NO: 1 oder SEQ ID NO: 2 gezeigt ist.

6. Polynukleotid nach einem der Ansprüche 1 bis 5, welches eine Nukleotidsequenz umfaßt, wie sie in SEQ ID NO: 9 gezeigt ist.

7. Polynukleotid nach einem der Ansprüche 2 bis 6, welches eine Nukleotidsequenz umfaßt, wie sie in SEQ ID NO: 3, SEQ ID NO: 4 oder SEQ ID NO: 5 gezeigt ist.

8. Polynukleotid nach einem der Ansprüche 2 bis 7, welches funktionsfähig mit einer interessierenden Nukleinsäure (NOI) verknüpft ist, so daß das Polynukleotid die Expression der NOI in einer Wirtszelle steuert.

9. Polynukleotid nach Anspruch 8, wobei die NOI HIF-1 codiert.

10. Polynukleotid nach einem der Ansprüche 1 bis 9, wobei die NOI IFN-γ codiert.

11. Polynukleotid nach einem der Ansprüche 1 bis 10, wobei dem Promotor eine CAAT-Box-Sequenz fehlt.

12. Polynukleotid nach einem der Ansprüche 8 bis 11, wobei die Wirtszelle eine Tumorzelle ist.

13. Polynukleotid nach einem der Ansprüche 8 bis 12, wobei die NOI ein Polypeptid mit therapeutischem Nutzen codiert.

14. Polynukleotid nach einem der Ansprüche 8 bis 13, wobei die NOI ein Polypeptid codiert, welches zytotoxisch ist.

15. Polynukleotid nach einem der Ansprüche 8 bis 14, wobei die NOI ein Polypeptid codiert, welches in der Lage ist, einen Arzneimittelpräkursor in eine zytotoxische Verbindung umzuwandeln.

16. Polynukleotid nach einem der Ansprüche 12 bis 15, wobei die NOI unter Polynukleotidsequenzen ausgewählt ist, die Proteine, welche an der Regulation der Zellteilung beteiligt sind, Enzyme, welche an zellulären Stoffwechselwegen beteiligt sind, Transkriptionsfaktoren und Hitzeschockproteine codieren.

17. Polynukleotid nach einem der Ansprüche 12 bis 16 zur Verwendung bei der Zuführung der NOI an eine Säugerzelle.

18. Nukleinsäurevektor, welcher ein Polynukleotid umfaßt, wie es in einem der vorangegangenen Ansprüche definiert ist.

19. Viraler Vektor, welcher ein Polynukleotid umfaßt, wie es in einem der Ansprüche 1 bis 17 definiert ist.

20. Viraler Vektor nach Anspruch 19, welcher weiterhin eine Nukleotidsequenz umfaßt, die ausgewählt ist unter (i) einer Nukleotidsequenz, die ein inhibitorisches RNA-Molekül codiert, welches die Spaltung von VHL-RNA direkt oder indirekt bewirken kann, (ii) einem oder mehreren inhibitorischen RNA-Molekülen, die an VHL-RNA binden und deren Prozessierung und/oder Expression verhindern, und (iii) einer Nukleotidsequenz, die ein Polypeptid codiert, welches in der Lage ist, die Bindung von VHL an Elongin B und/oder Elongin C zu hemmen.

21. Viraler Vektor nach Anspruch 20, wobei das Polypeptid ein nicht-funktionales Derivat von Wildtyp-VHL ist.

22. Viraler Vektor nach einem der Ansprüche 19 bis 21, wobei der virale Vektor ein retroviraler Vektor ist.

23. Viraler Vektor nach einem der Ansprüche 19 bis 21, wobei der virale Vektor ein adenoviraler Vektor ist.

24. Viraler Vektor nach Anspruch 22, wobei der Vektor ein lentiviraler Vektor ist.

25. Polynukleotid nach einem der Ansprüche 1 bis 17, Nukleinsäurevektor nach Anspruch 18 oder viraler Vektor nach einem der Ansprüche 19 bis 24 zur Verwendung bei der Behandlung eines menschlichen oder tierischen Patienten, der an einer Erkrankung leidet, bei welcher Hypoxie eine Ursache oder ein Symptom ist oder auf andere Weise vorliegt, oder bei der Vorbeugung dieser Erkrankung.

26. Verwendung eines Polynukleotids nach einem der Ansprüche 1 bis 17, eines Nukleinsäurevektors nach Anspruch 18 oder eines viralen Vektors nach einem der Ansprüche 19 bis 24 bei der Herstellung einer Zusammensetzung für die Behandlung eines menschlichen oder tierischen Patienten, der an Krebs leidet, oder bei der Vorbeugung von Krebs.

27. Pharmazeutische Zusammensetzung, welche ein Polynukleotid nach einem der Ansprüche 1 bis 17, einen Nukleinsäurevektor nach Anspruch 18 oder einen viralen Vektor nach einem der Ansprüche 19 bis 24 zusammen mit einem pharmazeutisch verträglichen Träger oder Verdünnungsmittel umfaßt.

28. Pharmazeutische Zusammensetzung nach Anspruch 27 zur Verwendung bei der Behandlung eines menschlichen oder tierischen Patienten, der an einer Erkrankung leidet, bei welcher Hypoxie eine Ursache oder ein Symptom ist oder auf andere Weise vorliegt, oder bei der Vorbeugung dieser Erkrankung, wobei eine wirksame Menge der pharmazeutischen Zusammensetzung an den Patienten verabreicht wird, der einer solchen Behandlung bedarf.

29. Verwendung einer pharmazeutischen Zusammensetzung nach Anspruch 27 bei der Herstellung eines Medikaments zur Behandlung eines menschlichen oder tierischen Patienten, der an Krebs leidet, oder bei der Vorbeugung von Krebs, wobei eine wirksame Menge der pharmazeutischen Zusammensetzung an den Patienten verabreicht wird, der einer solchen Behandlung bedarf.

30. Verfahren zum Erzeugen eines Virusstamms, wobei das Verfahren das Einbringen eines Polynukleotids, wie es in einem der Ansprüche 1 bis 17 definiert ist, in das Genom eines Virus umfaßt.

31. Wirtszelle, welche das Polynukleotid nach einem der Ansprüche 1 bis 17 umfaßt.

## Revendications

1. Polynucléotide comprenant au moins trois segments répétés d'un élément de réponse à l'hypoxie (HRE) de phosphoglycérate-kinase (PGK) liés de manière fonctionnelle à un promoteur de SV40 ou à un promoteur de MLV.

2. Polynucléotide suivant la revendication 1, dans lequel le segment répété médian comprend une délétion dans le site de liaison consensus HIF-1.

3. Polynucléotide suivant la revendication 1 ou la revendication 2, comprenant au moins trois segments répétés du HRE liés de manière fonctionnelle au promoteur et en amont du (en 5' par rapport au) promoteur et au moins trois segments répétés du HRE liés de manière fonctionnelle au promoteur et en aval du (en 3' par rapport au) promoteur.

4. Polynucléotide suivant l'une quelconque des revendications 1 à 3, dans lequel les segments répétés de HRE sont des segments répétés directs.

5. Polynucléotide suivant l'une quelconque des revendications 1 à 4, dans lequel le HRE comprend une séquence de nucléotides telle que représentée que la SEQ ID N° 1 ou la SEQ ID N° 2.

6. Polynucléotide suivant l'une quelconque des revendications 1 à 5, comprenant une séquence de nucléotides telle que représentée dans la SEQ ID N° 9.

7. Polynucléotide suivant l'une quelconque des revendications 2 à 6, comprenant une séquence de nucléotides telle que représentée dans la SEQ ID N° 3, la SEQ ID N° 4 ou la SEQ ID N° 5.

8. Polynucléotide suivant l'une quelconque des revendications 2 à 7, lié de manière fonctionnelle à un acide nucléique intéressant (NOI) de telle sorte que le polynucléotide dirige l'expression du NOI dans une cellule hôte.

9. Polynucléotide suivant la revendication 8, dans lequel le NOI code pour le HIF-1.

10. Polynucléotide suivant l'une quelconque des revendications 1 à 9, dans lequel le NOI code pour l'IFNγ-γ.

11. Polynucléotide suivant l'une quelconque des revendications 1 à 10, dans lequel le promoteur est dépourvu de séquence de boîte CAAT.

12. Polynucléotide suivant l'une quelconque des revendications 8 à 11, dans lequel la cellule hôte est une cellule tumorale.

13. Polynucléotide suivant l'une quelconque des revendications 8 à 12, dans lequel le NOI code pour un polypeptide à usage thérapeutique.

14. Polynucléotide suivant l'une quelconque des revendications 8 à 13, dans lequel le NOI code pour un polypeptide qui est cytotoxique.

15. Polynucléotide suivant l'une quelconque des revendications 8 à 14, dans lequel le NOI code pour un polypeptide capable de convertir un promédicament précurseur en un composé cytotoxique.

16. Polynucléotide suivant l'une quelconque des revendications 12 à 15, dans lequel le NOI est choisi parmi des séquences polynucléotidiques codant pour des protéines impliquées dans la régulation de la division cellulaire, des enzymes impliquées dans des voies métaboliques cellulaires, des facteurs de transcription et des protéines de choc thermique.

17. Polynucléotide suivant l'une quelconque des revendications 12 à 16, destiné à être utilisé dans l'administration du NOI à une cellule de mammifère.

18. Vecteur d'acide nucléique comprenant un polynucléotide tel que défini dans l'une quelconque des revendications précédentes.

19. Vecteur viral comprenant un polynucléotide tel que défini dans l'une quelconque des revendications 1 à 17.

20. Vecteur viral suivant la revendication 19, qui comprend en outre une séquence de nucléotides choisie entre (i) une séquence de nucléotides codant pour une molécule d'ARN inhibiteur capable d'effectuer le clivage, directement ou indirectement, de l'ARN VHL ; (ii) une ou plusieurs molécules d'ARN inhibiteur qui se lient à, et empêchent la maturation et/ou l'expression de, l'ARN VHL ; et (iii) une séquence de nucléotides codant pour un polypeptide capable d'inhiber la liaison de VHL à l'élongine B et/ou l'élongine C.

21. Vecteur viral suivant la revendication 20, dans lequel ledit polypeptide est un dérivé non fonctionnel du VHL de type sauvage.

22. Vecteur viral suivant l'une quelconque des revendications 19 à 21, ledit vecteur viral étant un vecteur rétroviral.

23. Vecteur viral suivant l'une quelconque des revendications 19 à 21, ledit vecteur viral étant un vecteur adénoviral.

24. Vecteur viral suivant la revendication 22, ledit vecteur étant un vecteur lentiviral.

25. Polynucléotide suivant l'une quelconque des revendications 1 à 17, vecteur d'acide nucléique suivant la revendication 18 ou vecteur viral suivant l'une quelconque des revendications 19 à 24, destiné à être utilisé dans le traitement ou la prévention d'un patient humain ou animal souffrant d'une maladie dans laquelle l'hypoxie est une cause ou un symptôme ou est par ailleurs présente.

26. Utilisation d'un polynucléotide suivant l'une quelconque des revendications 1 à 17, d'un vecteur d'acide nucléique suivant la revendication 18 ou d'un vecteur viral suivant l'une quelconque des revendications 19 à 24 dans la production d'une composition destinée au traitement ou à la prévention d'un patient humain ou animal souffrant d'un cancer.

27. Composition pharmaceutique comprenant un polynucléotide suivant l'une quelconque des revendications 1 à 17, un vecteur d'acide nucléique suivant la revendication 19 ou un vecteur viral suivant l'une quelconque des revendications 19 à 24 conjointement avec un support ou diluant pharmaceutiquement acceptable.

28. Composition pharmaceutique suivant la revendication 27, destinée à être utilisée dans le traitement ou la prévention d'un patient humain ou animal souffrant d'une maladie dans laquelle l'hypoxie est une cause ou un symptôme ou est par ailleurs présente, dans laquelle une quantité efficace de la composition pharmaceutique est administrée au patient ayant besoin d'un tel traitement.

29. Utilisation d'une composition pharmaceutique suivant la revendication 27 dans la production d'un médicament destiné au traitement ou à la prévention d'un patient humain ou animal souffrant d'un cancer, dans laquelle une quantité efficace de la composition pharmaceutique est administrée au patient ayant besoin d'un tel traitement.

30. Procédé pour la production d'une souche virale, procédé qui comprend l'introduction d'un polynucléotide tel que défini dans l'une quelconque des revendications 1 à 17 dans le génome d'un virus.

31. Cellule hôte comprenant le polynucléotide suivant l'une quelconque des revendications 1 à 17.
